# EUROPEAN PATENT SPECIFICATION

(11) **EP 3 704 146 B1**
(45) Date of publication and mention of the grant of the patent: **15.12.2021**
(21) Application number: 18800534.2
(22) Date of filing: 30.10.2018
(51) Int. Cl.: C07K 16/00, A61P 35/00

(54) **TRIFAB-CONTORSBODY**
TRIFAB-CONTORSBODY
TRIFAB-CONTORSBODY

(30) Priority: 01.11.2017 EP 17199608
(43) Date of publication of application: 09.09.2020
(73) Proprietor: F. Hoffmann-La Roche AG, 4070 Basel (CH)
(72) Inventor: BRINKMANN, Ulrich, 82377 Penzberg (DE); DICKOPF, Steffen, 82377 Penzberg (DE); GEORGES, Guy, 82377 Penzberg (DE); THOREY, Irmgard, 82377 Penzberg (DE)
(74) Representative: Skolaut, Alexander
(86) International application number: PCT/EP2018/079612
(87) International publication number: WO 2019/086395

(56) References cited:
- EP-A1- 1 378 520
- WO-A1-2017/191101
- KLAUS MAYER ET AL: "TriFabs-Trivalent IgG-Shaped Bispecific Antibody Derivatives: Design, Generation, Characterization and Application for Targeted Payload Delivery", INTERNATIONAL JOURNAL OF MOLECULAR SCIENCES, vol. 16, no. 12, 17 November 2015 (2015-11-17), pages 27497-27507, XP055441768, DOI: 10.3390/ijms161126037
- ULRICH BRINKMANN ET AL: "The making of bispecific antibodies", MABS, vol. 9, no. 2, 10 January 2017 (2017-01-10), pages 182-212, XP055374463, US ISSN: 1942-0862, DOI: 10.1080/19420862.2016.1268307
- SONG LI-PING ET AL: "A NEW MODEL OF TRISPECIFIC ANTIBODY WITH CYTOTOXICITY AGAINST TUMOR CELLS", SHENGWU HUAXUE YU SHENGWU WULI XUEBAO - ACTA BIOCHIMICA ETBIOPHYSICA SINICA, SHANGHAI KEXUE JISHU CHUBANSHE, SHANGHAI, CN, vol. 35, no. 6, 1 June 2003 (2003-06-01), pages 503-510, XP002388106, ISSN: 0582-9879

## Description

The current invention is in the field of target binding molecules. Herein is reported a multispecific antibody comprising at least three antigen binding sites whereby the molecule is highly compact and of relatively low molecular weight.

### Background of the Invention

Since the development of the first monoclonal antibodies by Koehler and Milstein in 1974 a lot of efforts have been dedicated to the development of antibodies which are appropriate for therapy in humans. The first monoclonal antibodies which became available had been developed in mice and rats. These antibodies when used for therapy of a human being caused unwanted side effects due to anti-rodent antibodies. A lot of efforts have been dedicated to the reduction or even elimination of such unwanted side effects.

In the past years an ever growing number of human monoclonal antibodies or humanized monoclonal antibodies have reached the market. Well-known examples include for example Herceptin^{®} and MabThera^{®} from Hoffmann-La Roche, Basel.

Furthermore, new antibody formats derived from the wild-type four chain Y-shaped antibody format have been developed. These formats are mainly bi- and multispecific formats. For a review see e.g. Kontermann, R., mAbs 4 (2012) 182-197.

In US 2009/0175867 a single-chain multivalent binding proteins with effector function is reported.

In WO 2014/131711 are reported bispecific antibodies wherein the second and the third antigen binding moiety may be fused to the Fc domain directly or through an immunoglobulin hinge region.

In EP 15176083 a novel antibody format having reduced molecular weight in comparison to a full-length antibody and use thereof is reported.

WO 2007/048022 discloses antibody-polypeptide fusion proteins and methods for producing and using same.

WO 2007/146968 discloses single-chain multivalent binding proteins with effector function.

EP 1 378 520 discloses a cyclic single strand trispecific antibody.

WO 2016/087416 discloses a multispecific antibody comprising at least three antigen binding sites, wherein two antigen binding sites are formed by a first antigen binding moiety and a second antigen binding moiety and the third antigen binding site is formed by a variable heavy chain domain (VH3) and a variable light chain domain (VL3).

Klaus Meyer et al., International Jounal of Molecular Sciences, vol. 16, no.12, (2015-11-17), pp. 27497-27507; discloses TriFabs (Trivalent IgG-shaped bispecific antibody derivatives).

### Summary of the Invention

Herein is reported a new target binder. This new target binder is a trivalent, bi- or tri-specific, bi-circular polypeptide, wherein each of the circular polypeptides comprises three variable domain-constant domain (V-C) dimers. In more detail, in the first polypeptide the N-terminal dimer, V₁ₐ-C₁ₐ, comprises a first part of a first binding site, the central dimer, V₂ₐ-C₂ₐ, comprises a first part of a second binding site, and the C-terminal dimer, V_{1b}-C_{1b}, comprises the second part of the first binding site, V₁ₐ-C₁ₐ and V_{1b}-C_{1b}. The second polypeptide comprises as central dimer the second part of the second binding site, V_{2b}-C_{2b}, and as N- and C-terminal dimers the first and the second part of a third binding site. The respective first and second parts of the first, second and third binding sites associate with each other to form complete or functional first to third binding sites. By the association of the first and second part of the first and third binding site each of the first and second polypeptide is circularized. Each of the associations can be independently of each other either non-covalently or covalently. In case the association is covalently it is not by a peptide bond, but, e.g. by a disulfide bond

The invention is is a multispecific antibody comprising three antigen binding sites, wherein
a) the first antigen binding site is formed by a first circular fusion polypeptide, comprising a first part of a first binding domain, a second part of a first binding domain and a first part of a third binding domain, wherein
   - the first part of the first binding domain is fused either directly or via a first peptidic linker to the N-terminus of the first part of the third binding domain, and
   - the second part of the first binding domain is fused either directly or via a second peptidic linker to the C-terminus of the first part of the third binding domain, and
   - the first part of the first binding domain is a heavy chain Fab fragment (VH₁-CH1) or a light chain Fab fragment (VL₁-CL), whereby the second part of the first binding domain is a light chain Fab fragment if the first part of the first binding domain is a heavy chain Fab fragment or vice versa, and
   - the first part of the first binding domain and the second part of the first binding domain are associated with each other and form the first antigen binding site,
b) the second antigen binding site is formed by a second circular fusion polypeptide, comprising a first part of a second binding domain, a second part of a second binding domain and the second part of the third binding domain, wherein
   - the first part of the second binding domain is fused either directly or via a third peptidic linker to the N-terminus of the second part of the third binding domain, and
   - the second part of the second binding domain is fused either directly or via a fourth peptidic linker to the C-terminus of the second part of the third binding domain, and
   - the first part of the second binding domain is a heavy chain Fab fragment (VH₂-CH1) or a light chain Fab fragment (VL₂-CL), whereby the second part of the second binding domain is a light chain Fab fragment if the first part of the second binding domain is a heavy chain Fab fragment or vice versa, (whereby the first part of the second binding site is selected independently of the first part of the first binding site), and
   - the first part of the second binding domain and the second part of the second binding domain are associated with each other and form the second antigen binding site,
c) the third antigen binding site is formed by the first part of the third binding domain and the second part of the third binding domain, wherein
   - the first part of the third binding domain is either a variable heavy chain-linker-CH3 domain fusion polypeptide (VH₃-L-CH3) or a variable light chain-linker-CH3 domain fusion polypeptide (VL₃-L-CH3), and
   - the second part of the third binding domain is a variable heavy chain-CH3 domain fusion polypeptide if the first part of the third binding domain in the first circular fusion polypeptide is a variable light chain-CH3 domain fusion polypeptide, or vice versa, and
   - the first part of the third binding domain and the second part of the third binding domain are associated with each other and form the third antigen binding site,
   wherein the two constant heavy chain domains 3 (CH3) are altered to promote heterodimerization by
   i) generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains, or substituting at least one original amino acid residue in one of the CH3 domains by a positively charged amino acid, and substituting at least one original amino acid residue in the other one of the CH3 domains by a negatively charged amino acid, or
   ii) introduction of at least one cysteine residue in each CH3 domain such that a disulfide bond is formed between the CH3 domains, or
   iii) both modifications of i) and ii), wherein
the multispecific antibody is devoid of constant heavy chain domains 2 (CH2) and a hinge region.

In one embodiment the first and the second circular fusion polypeptide each comprises exactly one part of a binding domain N-terminal to the part of the third antigen binding domain and exactly one, but different, part of the binding domain C-terminal to the part of the third binding domain.

In one embodiment the first part of the first binding domain and the second part of the first binding domain in the first circular fusion polypeptide are covalently associated with each other and/or the first part of the second binding domain and the second part of the second binding domain in the second circular fusion polypeptide are covalently associated with each other. In one embodiment the covalent association is by a bond other than a peptide bond. In one preferred embodiment the covalent association is by a disulfide bond.

### In one embodiment

i) the C-terminus of the CH1 domain of the first part of the first or second binding site is conjugated to the N-terminus of the part of the third binding domain, and the N-terminus of the VL domain of the second part of the first or second binding site is conjugated to the C-terminus of the third binding domain, or
ii) the C-terminus of the VH domain of the first part of the first or second binding site is conjugated to the N-terminus of the part of the third binding domain, and the N-terminus of the CL domain of the second part of the first or second binding site is conjugated to the C-terminus of the third binding domain.

In one embodiment the first and/or the second and/or the third antigen binding site is disulfide stabilized by introduction of cysteine residues independently of each other at the following positions to form a disulfide bond between the VH and VL domains (numbering according to Kabat):
- VH at position 44, and VL at position 100,
- VH at position 105, and VL at position 43, or
- VH at position 101, and VL at position 100.

In one embodiment the first part of the first or the second binding domain is an antibody heavy chain Fab fragment (VH+CH1) and the second part of the first or the second binding domain is an antibody light chain Fab fragment (VL+CL) or vice versa.

In one embodiment the multispecific antibody fusion polypeptide exerts effector function. In one embodiment the effector function is ADCC or/and CDC.

In one embodiment the first part of the first binding domain is fused via a first peptidic linker of SEQ ID NO: 16 or SEQ ID NO: 17 to the N-terminus of the part of the third binding domain and the second part of the first binding domain is fused via a second peptidic linker of SEQ ID NO: 16 or SEQ ID NO: 17 to the C-terminus of the part of the third binding domain, whereby the first and the second peptidic linker are selected independently of each other.

In one embodiment the first part of the second binding domain is fused via a first peptidic linker of SEQ ID NO: 16 or SEQ ID NO: 17 to the N-terminus of the part of the third binding domain and the second part of the second binding domain is fused via a second peptidic linker of SEQ ID NO: 16 or SEQ ID NO: 17 to the C-terminus of the part of the third binding domain, whereby the first and the second peptidic linker are selected independently of each other.

In one preferred embodiment the first part of the first binding domain is fused via a first peptidic linker of SEQ ID NO: 38 to the N-terminus of the part of the third binding domain and the second part of the first binding domain is fused via a second peptidic linker of SEQ ID NO: 16 to the C-terminus of the part of the third binding domain, and the first part of the second binding domain is fused via a first peptidic linker of SEQ ID NO: 38 to the N-terminus of the part of the third binding domain and the second part of the second binding domain is fused via a second peptidic linker of SEQ ID NO: 16 to the C-terminus of the part of the third binding domain.

In one embodiment the third binding site specifically binds to human CD3 or human CD4 or human CD28.

In one embodiment the multispecific antibody comprising three antigen binding sites comprises
a first circular fusion polypeptide, comprising in N- to C-terminal direction a heavy chain Fab fragment (VH-CH1) as first part of the first binding domain, a linker of SEQ ID NO: 38, a variable heavy chain-CH3 domain fusion polypeptide as first part of the third binding domain, wherein the CH3 domain comprises the mutation T366W (optionally further mutation S354C or Y349C), a linker of SEQ ID NO: 16, and a light chain Fab fragment (VL-CL) as second part of the first binding domain,
and
a second circular fusion polypeptide, comprising in N- to C-terminal direction a light chain Fab fragment (VL-CL) as first part of the second binding domain, a linker of SEQ ID NO: 38, a variable light chain-CH3 domain fusion polypeptide as second part of the third binding domain, wherein the CH3 domain comprises the mutations T366S, L368A and Y407V (optionally further the mutation Y349C or S354C), a linker of SEQ ID NO: 16, and a heavy chain Fab fragment (VH-CH1) as second part of the second binding domain,
(numbering according to Kabat EU index)
and
the third binding site specifically binds to human CD3 or CD4 or CD28.

In one embodiment each (circular) (single chain) fusion polypeptide comprises in N- to C-terminal direction before the part of the third binding domain (i.e. N-terminal to the part of the third binding domain) exactly one antibody variable domain and after the part of the third binding domain (i.e. C-terminal to the part of the third domain) exactly one antibody variable domain.

In one embodiment the target is a cell surface antigen or the soluble ligand of a cell surface receptor.

In one embodiment the binding domain is a Fab, a DAF or a bispecific Fab.

In one embodiment the first and/or the second binding domain is a (conventional) Fab, wherein the first part of the binding domain comprises an antibody heavy chain variable domain (VH) and at least an N-terminal fragment of a (or a complete) first antibody heavy chain constant domain (CH1) and the respective other part of the binding domain comprises an antibody light chain variable domain (VL) and at least an N-terminal fragment of a (or a complete) antibody light chain constant domain (CL), or vice versa. In one embodiment the first part of the binding domain comprises in N- to C-terminal direction VH-CH1 and the second part of the binding domain comprises in N- to C-terminal direction VL-CL, or vice versa.

In one embodiment the amino acid residue having a larger side chain volume than the original amino acid residue is selected from R, F, Y and W.

In one embodiment the amino acid residue having a smaller side chain volume than the original amino acid residue is selected from A, S, T and V.

In one embodiment the amino acid residue having a larger side chain volume than the original amino acid residue is selected from R, F, Y and W, and the amino acid residue having a smaller side chain volume than the original amino acid residue is selected from A, S, T and V.

In one embodiment one CH3 domain of the third binding site comprises a T366W mutation (knob mutation; knob side), and the other CH3 domain of third binding site comprises the mutations T366S, L368A and Y407V (hole mutations; hole side) (numberings according to EU index of Kabat).

In one embodiment one CH3 domain of the third binding site comprises a T366W mutation (knob mutation; knob side), and the other CH3 domain of third binding site comprises the mutations T366S, L368A and Y407V (hole mutations; hole side) (numberings according to EU index of Kabat).

In one embodiment one CH3 domain of the third binding site comprises the S354C and T366W mutations (knob-cys mutation; knob-cys side), and the other CH3 domain of third binding site comprises the mutations Y349C, T366S, L368A and Y407V (hole-cys mutations; hole-cys side) (numberings according to EU index of Kabat).

In one embodiment the multispecific antibody comprises three antigen binding sites, wherein
a) the first antigen binding site is formed by a first antibody heavy chain variable domain (VH₁) and a first antibody light chain variable domain (VL₁) pair (VH₁/VL₁-pair) of a first circular fusion polypeptide, wherein
   - the VH₁ is part of a first heavy chain Fab fragment (VH₁-CH1) and the VL₁ is part of a first light chain Fab fragment (VL₁-CL),
   - the VH₁-CH1 is conjugated via a first peptidic linker to either the N-terminus or the C-terminus of a first variable domain-linker-antibody constant domain 3 fusion polypeptide (V₃-L-CH3) and the VL₁-CL is conjugated via a second peptidic linker to the respective other terminus of the first V₃-L-CH3,
   - the VH₁-CH1 and the VL₁-CL are associated with each other and form the VH₁VL₁-pair,
b) the second antigen binding site is formed by a second antibody heavy chain variable domain (VH₂) and a second antibody light chain variable domain (VL₂) pair (VH₂/VL₂-pair) of a second circular fusion polypeptide, wherein
   - the VH₂ is part of a second heavy chain Fab fragment (VH₂-CH1) and the VL₂ is part of a second light chain Fab fragment (VL₂-CL),
   - the VH₂-CH1 is conjugated via a third peptidic linker to either the N-terminus or the C-terminus of a second variable domain-linker-antibody constant domain 3 fusion polypeptide (V₃-L-CH3) and the VL₂-CL is conjugated via a fourth peptidic linker to the respective other terminus of the second V₃-L-CH3,
   - the VH₂-CH1 and the VL₂-CL are associated with each other form the VH₂/VL₂-pair,
c) the third antigen binding site is formed by the first V₃-L-CH3 and the second V₃-L-CH3, wherein
   - the first V₃-L-CH3 is either a variable heavy chain domain-linker-antibody constant domain 3 fusion polypeptide (VH₃-L-CH3) or a variable light chain domain-linker-antibody constant domain 3 fusion polypeptide (VL₃-L-CH3),
   - the second V₃-CH3 is a variable heavy chain domain-linker-antibody constant domain 3 fusion polypeptide if the first V₃-L-CH3 is a variable light chain domain-linker-antibody constant domain 3 fusion polypeptide or vice versa,
   - the VH₃-L-CH3 and the VL₃-L-CH3 are associated with each other form the VH₃/VL₃-pair,
wherein the two antibody constant domains 3 (CH3) are altered to promote heterodimerization by introducing the mutation T366W and optionally the mutation S354C in one of the CH3s and the mutations T366S, L368A and Y407V and optionally the mutation Y349C in the respective other CH3 (numberings according to EU index of Kabat),
wherein the multispecific antibody is devoid of antibody constant domains 2 (CH2) and a hinge region.

In one embodiment the linker in the first and/or second part of the third binding domain is absent (i.e. the first part of the third binding domain is a variable heavy chain domain-antibody constant domain 3 fusion polypeptide and the second part of the third binding domain is a variable light chain domain-antibody constant domain 3 fusion polypeptide, or vice versa).

In one embodiment the first and the second binding site specifically bind to the same or a different target and the third binding site specifically binds to a target different from the target of the first and the second binding site.

One aspect as reported herein is an immunoconjugate comprising the multispecific antibody as reported herein and a cytotoxic agent.

One aspect as reported herein is a pharmaceutical formulation comprising the multispecific antibody as reported herein and a pharmaceutically acceptable carrier.

One aspect as reported herein is the multispecific antibody as reported herein for use as a medicament.

One aspect as reported herein is the use of the multispecific antibody as reported herein in the manufacture of a medicament.

### Detailed Description of the Invention

### I. Definitions

The knobs into holes dimerization modules and their use in antibody engineering are described in Carter P.; Ridgway J.B.B.; Presta L.G.: Immunotechnology, Volume 2, Number 1, February 1996, pp. 73-73(1).

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991).

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) is used for the constant heavy chain domains (CHI, Hinge, CH2 and CH3, which is herein further clarified by referring to "numbering according to Kabat EU index" in this case).

Useful methods and techniques for carrying out the current invention are described in e.g. Ausubel, F.M. (ed.), Current Protocols in Molecular Biology, Volumes I to III (1997); Glover, N.D., and Hames, B.D., ed., DNA Cloning: A Practical Approach, Volumes I and II (1985), Oxford University Press; Freshney, R.I. (ed.), Animal Cell Culture ― a practical approach, IRL Press Limited (1986); Watson, J.D., et al., Recombinant DNA, Second Edition, CHSL Press (1992); Winnacker, E.L., From Genes to Clones; N.Y., VCH Publishers (1987); Celis, J., ed., Cell Biology, Second Edition, Academic Press (1998); Freshney, R.I., Culture of Animal Cells: A Manual of Basic Technique, second edition, Alan R. Liss, Inc., N.Y. (1987).

The use of recombinant DNA technology enables the generation of derivatives of a nucleic acid. Such derivatives can, for example, be modified in individual or several nucleotide positions by substitution, alteration, exchange, deletion or insertion. The modification or derivatization can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA; Hames, B.D., and Higgins, S.G., Nucleic acid hybridization ― a practical approach (1985) IRL Press, Oxford, England).

It must be noted that as used herein and in the appended claims, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein. It is also to be noted that the terms "comprising", "including", and "having" can be used interchangeably.

The term "about" denotes a range of +/- 20 % of the thereafter following numerical value. In one embodiment the term about denotes a range of +/- 10 % of the thereafter following numerical value. In one embodiment the term about denotes a range of +/- 5 % of the thereafter following numerical value.

"Affinity" refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (kd). Affinity can be measured by common methods known in the art, including those described herein.

The term "antibody-dependent cellular cytotoxicity (ADCC)" is a function mediated by Fc receptor binding and refers to lysis of target cells mediated by an antibody Fc-region in the presence of effector cells. ADCC is measured in one embodiment by the treatment of a preparation of target expressing erythroid cells (e.g. K562 cells expressing recombinant target) with an Fc-region comprising multicircular fusion polypeptide as reported herein in the presence of effector cells such as freshly isolated PBMC (peripheral blood mononuclear cells) or purified effector cells from buffy coats, like monocytes or NK (natural killer) cells. Target cells are labeled with Cr-51 and subsequently incubated with the multicircular fusion polypeptide. The labeled cells are incubated with effector cells and the supernatant is analyzed for released Cr-51. Controls include the incubation of the target endothelial cells with effector cells but without the multicircular fusion polypeptide. The capacity of the multicircular fusion polypeptide to induce the initial steps mediating ADCC is investigated by measuring the binding to Fcγ receptors expressing cells, such as cells, recombinantly expressing FcyRI and/or FcyRIIA or NK cells (expressing essentially FcyRIIIA). In one preferred embodiment binding to FcyR on NK cells is measured.

The term "binding to" denotes the binding of a binding site to its target, such as e.g. of an antibody binding site comprising an antibody heavy chain variable domain and an antibody light chain variable domain (VH/VL-pair) to the respective antigen. This binding can be determined using, for example, a BIAcore^{®} assay (GE Healthcare, Uppsala, Sweden).

For example, in one possible embodiment of the BIAcore^{®} assay the antigen is bound to a surface and binding of the antibody binding site is measured by surface plasmon resonance (SPR). The affinity of the binding is defined by the terms ka (association constant: rate constant for the association to form a complex), kd (dissociation constant; rate constant for the dissociation of the complex), and KD (kd/ka). Alternatively, the binding signal of a SPR sensorgram can be compared directly to the response signal of a reference, with respect to the resonance signal height and the dissociation behaviors.

The term "BiFab" denotes a molecule comprising two pairs of V₁-C₁/V₂-C₂ wherein V denotes an antibody variable domain and C denotes an antibody constant domain, which are associated with each other. For example, the pairs can be VH₁-CH1/VL₁-CL and VH₂-CH3₁/VL₂-CH3₂. Likewise, the term "TriFab" denotes a molecule comprising three pairs of V₁-C₁/V₂-C₂ wherein V denotes an antibody variable domain and C denotes an antibody constant domain, which are associated with each other. For example, the pairs can be VH₁-CH1/VL₁-CL, VH₂-CH1/VL₂-CL, and VH₃-CH3₁/VL₃-CH3₂.

The term "CH1 domain" denotes the part of an antibody heavy chain polypeptide that extends approximately from EU position 118 to EU position 215 (EU numbering system). In one embodiment a CH1 domain comprises the amino acid sequence of ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKKV (SEQ ID NO: 01).

The term "CH2 domain" denotes the part of an antibody heavy chain polypeptide that extends approximately from EU position 231 to EU position 340 (EU numbering system according to Kabat). In one embodiment a CH2 domain comprises the amino acid sequence of APELLGGPSV FLFPPKPKDT LMISRTPEVT CVWDVSHEDP EVKFNWYVDG VEVHNAKTKP REEQESTYRW SVLTVLHQDW LNGKEYKCKV SNKALPAPIE KTISKAK (SEQ ID NO: 02). The CH2 domain is unique in that it is not closely paired with another domain. Rather, two N-linked branched carbohydrate chains are interposed between the two CH2 domains of an intact native Fc-region. It has been speculated that the carbohydrate may provide a substitute for the domain-domain pairing and help stabilize the CH2 domain. Burton, Mol. Immunol. 22 (1985) 161-206.

The term "CH3 domain" denotes the part of an antibody heavy chain polypeptide that extends approximately from EU position 341 to EU position 446. In one embodiment the CH3 domain comprises the amino acid sequence of

The term "cytotoxic agent" as used herein refers to a substance that inhibits or prevents a cellular function and/or causes cell death or destruction. Cytotoxic agents include, but are not limited to, radioactive isotopes (e.g., At-211, 1-131, 1-125, Y-90, Re-186, Re-188, Sm-153, Bi-212, P-32, Pb-212 and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "complement-dependent cytotoxicity (CDC)" refers to lysis of cells induced by the Fc-region of an antibody as reported herein in the presence of complement. CDC is measured in one embodiment by the treatment of target expressing human endothelial cells with a multicircular fusion polypeptide as reported herein in the presence of complement. The cells are in one embodiment labeled with calcein. CDC is found if the multicircular fusion polypeptide induces lysis of 20 % or more of the target cells at a concentration of 30 µg/ml. Binding to the complement factor C1q can be measured in an ELISA. In such an assay in principle an ELISA plate is coated with concentration ranges of the multicircular fusion polypeptide, to which purified human C1q or human serum is added. C1q binding is detected by an antibody directed against C1q followed by a peroxidase-labeled conjugate. Detection of binding (maximal binding Bmax) is measured as optical density at 405 nm (OD405) for peroxidase substrate ABTS^{®} (2,2'-azino-di-[3-ethylbenzthiazoline-6-sulfonate]).

"Effector functions" refer to those biological activities attributable to the Fc-region of an antibody, which vary with the antibody class from which it is derived. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B-cell receptor); and B-cell activation.

Fc receptor binding dependent effector functions can be mediated by the interaction of the Fc-region of an antibody with Fc receptors (FcRs), which are specialized cell surface receptors on hematopoietic cells. Fc receptors belong to the immunoglobulin superfamily, and have been shown to mediate both the removal of antibody-coated pathogens by phagocytosis of immune complexes, and the lysis of erythrocytes and various other cellular targets (e.g. tumor cells) presenting the Fc-region, via antibody dependent cell mediated cytotoxicity (ADCC) (see e.g. Van de Winkel, J.G. and Anderson, C.L., J. Leukoc. Biol. 49 (1991) 511-524). FcRs are defined by their specificity for immunoglobulin isotypes: Fc receptors for IgG type Fc-regions are referred to as FcyR. Fc receptor binding is described e.g. in Ravetch, J.V. and Kinet, J.P., Annu. Rev. Immunol. 9 (1991) 457-492; Capel, P.J., et al., Immunomethods 4 (1994) 25-34; de Haas, M., et al., J. Lab. Clin. Med. 126 (1995) 330-341; Gessner, J.E., et al., Ann. Hematol. 76 (1998) 231-248.

Cross-linking of receptors for the Fc-region of IgG type antibodies (FcyR) triggers a wide variety of effector functions including phagocytosis, antibody-dependent cellular cytotoxicity, and release of inflammatory mediators, as well as immune complex clearance and regulation of antibody production. In humans, three classes of FcyR have been characterized, which are:
- FcyRI (CD64) binds monomeric IgG with high affinity and is expressed on macrophages, monocytes, neutrophils and eosinophils. Modification in the Fc-region IgG at least at one of the amino acid residues E233-G236, P238, D265, N297, A327 and P329 (numbering according to EU index of Kabat) reduce binding to FcyRI. IgG2 residues at positions 233-236, substituted into IgG1 and IgG4, reduced binding to FcyRI by 10³-fold and eliminated the human monocyte response to antibody-sensitized red blood cells (Armour, K.L., et al., Eur. J. Immunol. 29 (1999) 2613-2624).
- FcyRII (CD32) binds complexed IgG with medium to low affinity and is widely expressed. This receptor can be divided into two sub-types, FcyRIIA and FcyRIIB. FcyRIIA is found on many cells involved in killing (e.g. macrophages, monocytes, neutrophils) and seems able to activate the killing process. FcyRIIB seems to play a role in inhibitory processes and is found on B cells, macrophages and on mast cells and eosinophils. On B-cells it seems to function to suppress further immunoglobulin production and isotype switching to, for example, the IgE class. On macrophages, FcyRIIB acts to inhibit phagocytosis as mediated through FcyRIIA. On eosinophils and mast cells the B-form may help to suppress activation of these cells through IgE binding to its separate receptor. Reduced binding for FcyRIIA is found e.g. for antibodies comprising an IgG Fc-region with mutations at least at one of the amino acid residues E233-G236, P238, D265, N297, A327, P329, D270, Q295, A327, R292, and K414 (numbering according to EU index of Kabat).
- FcyRIII (CD16) binds IgG with medium to low affinity and exists as two types. FcyRIIIA is found on NK cells, macrophages, eosinophils and some monocytes and T cells and mediates ADCC. FcyRIIIB is highly expressed on neutrophils. Reduced binding to FcyRIIIA is found e.g. for antibodies comprising an IgG Fc-region with mutation at least at one of the amino acid residues E233-G236, P238, D265, N297, A327, P329, D270, Q295, A327, S239, E269, E293, Y296, V303, A327, K338 and D376 (numbering according to EU index of Kabat).

Mapping of the binding sites on human IgG1 for Fc receptors, the above mentioned mutation sites and methods for measuring binding to FcyRI and FcyRIIA are described in Shields, R.L., et al. J. Biol. Chem. 276 (2001) 6591-6604.

An "effective amount" of an agent, e.g., a pharmaceutical formulation, refers to an amount effective, at dosages and for periods of time necessary, to achieve the desired therapeutic or prophylactic result.

The term "epitope" refers to that part of a given target that is required for specific binding between the target and a binding site. An epitope may be continuous, i.e. formed by adjacent structural elements present in the target, or discontinuous, i.e. formed by structural elements that are at different positions in the primary sequence of the target, such as in the amino acid sequence of a protein as target, but in close proximity in the three-dimensional structure, which the target adopts in a native environment, such as in a bodily fluid.

The term "Fc receptor" as used herein refers to activation receptors characterized by the presence of a cytoplasmatic ITAM sequence associated with the receptor (see e.g. Ravetch, J.V. and Bolland, S., Annu. Rev. Immunol. 19 (2001) 275-290). Such receptors are FcyRI, FcyRIIA and FcyRIIIA. The term "no binding of FcyR" denotes that at an antibody concentration of 10 µg/ml the binding of an antibody as reported herein to NK cells is 10 % or less of the binding found for anti-OX40L antibody LC.001 as reported in WO 2006/029879.

While IgG4 shows reduced FcR binding, antibodies of other IgG subclasses show strong binding. However, Pro238, Asp265, Asp270, Asn297 (loss of Fc carbohydrate), Pro329, Leu234, Leu235, Gly236, Gly237, Ile253, Ser254, Lys288, Thr307, Gln311, Asn434, and His435 are residues which provide if altered also reduce FcR binding (Shields, R.L., et al. J. Biol. Chem. 276 (2001) 6591-6604; Lund, J., et al., FASEB J. 9 (1995) 115-119; Morgan, A., et al., Immunology 86 (1995) 319-324; and EP 0 307 434).

The term "hinge region" denotes the part of an antibody heavy chain polypeptide that joins in a wild-type antibody heavy chain the CH1 domain and the CH2 domain, e. g. from about position 216 to about position 230 according to the EU number system of Kabat, or from about position 226 to about position 230 according to the EU number system of Kabat. The hinge regions of other IgG subclasses can be determined by aligning with the hinge-region cysteine residues of the IgG1 subclass sequence.

The hinge region is normally a dimeric molecule consisting of two polypeptides with identical amino acid sequence. The hinge region generally comprises about 25 amino acid residues and is flexible allowing the associated target binding sites to move independently. The hinge region can be subdivided into three domains: the upper, the middle, and the lower hinge domain (see e.g. Roux, et al., J. Immunol. 161 (1998) 4083).

The terms "full length antibody", "intact antibody", and "whole antibody" are used herein interchangeably to refer to an antibody having a structure substantially similar to a native antibody structure.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

An "immunoconjugate" is a multispecific antibody as reported herein conjugated to one or more molecule(s), including but not limited to a cytotoxic agent.

An "individual" or "subject" is a mammal. Mammals include, but are not limited to, domesticated animals (e.g. cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95 % or 99 % purity as determined by, for example, electrophoretic (e.g., SDS-PAGE, isoelectric focusing (IEF), capillary electrophoresis) or chromatographic (e.g., size exclusion chromatography or ion exchange or reverse phase HPLC). For review of methods for assessment of antibody purity, see, e.g., Flatman, S. et al., J. Chrom. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "heavy chain" denotes the longer polypeptide chains of native IgG antibodies comprising (in N- to C-terminal direction) a heavy chain variable domain (VH), antibody constant domain 1 (CHI), a hinge region, antibody constant domain 2 (CH2) and antibody constant domain 3 (CH3). The "class" of an antibody or an Fc-region refers to the type of constant domain or constant region possessed by the heavy chains or fragments thereof. There are five major classes: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "light chain" denotes the shorter polypeptide chains of native IgG antibodies comprising (in N- to C-terminal direction) a light chain variable domain (VL) and a light chain constant domain (CL). The light chain of an antibody may be assigned to one of two types, called kappa (κ) and lambda (λ), based on the amino acid sequence of its constant domain, see SEQ ID NO: 04 for a human kappa light chain constant domain and SEQ ID NO: 05 for a human lambda light chain constant domain.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked multispecific antibody" refers to a multispecific antibody that is not conjugated to a moiety (e.g., a cytotoxic moiety) or radiolabel. The naked multispecific antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3), whereby between the first and the second constant domain a hinge region is located. Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

The term "paratope" refers to that part of a given antibody molecule that is required for specific binding between a target and a binding site. A paratope may be continuous, i.e. formed by adjacent amino acid residues present in the binding site, or discontinuous, i.e. formed by amino acid residues that are at different positions in the primary sequence of the amino acid residues, such as in the amino acid sequence of the CDRs of the amino acid residues, but in close proximity in the three-dimensional structure, which the binding site adopts.

The term "peptidic linker" denotes a linker of natural and/or synthetic origin. A peptidic linker consists of a linear chain of amino acids wherein the 20 naturally occurring amino acids are the monomeric building blocks which are connected by peptide bonds. The chain has a length of from 1 to 50 amino acid residues, preferred between 1 and 28 amino acid residues, especially preferred between 3 and 25 amino acid residues. The peptidic linker may contain repetitive amino acid sequences or sequences of naturally occurring polypeptides. The peptidic linker has the function to ensure that the domains of a circular fusion polypeptide can perform their biological activity by allowing the domains to fold correctly and to be presented properly. Preferably the peptidic linker is a "synthetic peptidic linker" that is designated to be rich in glycine, glutamine, and/or serine residues. These residues are arranged e.g. in small repetitive units of up to five amino acids, such as GGGS (SEQ ID NO: 06), GGGGS (SEQ ID NO: 07), QQQG (SEQ ID NO: 08), QQQQG (SEQ ID NO: 09), SSSG (SEQ ID NO: 10) or SSSSG (SEQ ID NO: 11). This small repetitive unit may be repeated for two to five times to form a multimeric unit, such as e.g. (GGGS)2 (SEQ ID NO: 12), (GGGS)3 (SEQ ID NO: 13), (GGGS)4 (SEQ ID NO: 14), (GGGS)5 (SEQ ID NO: 15), (GGGGS)2 (SEQ ID NO: 16), (GGGGS)3 (SEQ ID NO: 17), (GGGGS)4 (SEQ ID NO: 18), (GGGGS)4GG (SEQ ID NO: 19), GG(GGGGS)3 (SEQ ID NO: 20) and (GGGGS)6 (SEQ ID NO: 21). At the amino- and/or carboxy-terminal ends of the multimeric unit up to six additional arbitrary, naturally occurring amino acids may be added. Other synthetic peptidic linkers are composed of a single amino acid, that is repeated between 10 to 20 times and may comprise at the amino- and/or carboxy-terminal end up to six additional arbitrary, naturally occurring amino acids, such as e.g. serine in the linker GSSSSSSSSSSSSSSSG (SEQ ID NO: 22). All peptidic linkers can be encoded by a nucleic acid molecule and therefore can be recombinantly expressed. As the linkers are themselves peptides, the antifusogenic peptide is connected to the linker via a peptide bond that is formed between two amino acids.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs) (see, e.g., Kindt, T.J. et al. Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively (see, e.g., Portolano, S., et al., J. Immunol. 150 (1993) 880-887; Clackson, T., et al., Nature 352 (1991) 624-628).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

The invention is exemplified in the following using polypeptides of different structure and specificity. These are presented only in order to exemplify the invention. This has not to be construed as a limitation. The true scope is set forth in the claims.

### II. The multispecific antibody as reported herein

Herein is reported a multispecific antibody comprising two circular fusion polypeptides each of them comprising a VH/VL-pair and thereby a first and a second binding site, whereby a third VH/VL-pair and thereby a third binding site is formed by the associated of the two circular fusion polypeptides.

The invention is based at least in part on the finding that the fusion of the light chain variable domain to the C-terminus of a (full length) heavy chain, or of the heavy chain variable domain to a full length light chain, results in the formation of a functional binding site comprising the respective VH and VL domains (VH/VL-pair) of the same polypeptide, i.e. the pair of the variable light chain domain and the variable heavy chain domain within a single polypeptide chain form a functional VH/VL-pair and thereby a functional binding site by intrachain circularization. In this circular polypeptide the N-terminal portion comprises a first part of a binding domain and the C-terminal portion comprises a second part of a binding domain. The first part of the binding domain and the second part of the binding domain (associate with each other to) form a complete or functional binding site. Thereby the polypeptide is circularized.

The invention is based at least in part on the finding that a multispecific antibody can be provided comprising two circular fusion polypeptides, wherein each of the circular fusion polypeptides comprises a functional binding site formed by a VH/VL-pair, a further binding site is formed by the association of the two circular fusion polypeptides.

The invention is based at least in part on the finding that it is possible, by modifying the length of the peptidic linkers connecting the individual antibody variable domains to the respective N- and C-termini of a fusion polypeptide of a part of the third binding domain and a heterodimerization domain, to adjust the geometry/distance of the two binding sites formed by the circular fusion polypeptides in the resulting multispecific antibody.

The invention is based at least in part on the finding that the binding geometry of a dimeric, i.e. dicircular, fusion polypeptide can be changed depending on the lengths of the first/third peptidic linker and the second/fourth peptidic linker (i.e. the linker length ratio). Thereby it is possible to fix the geometry of the two Fab-like binding arms with respect to each other.

Herein is disclosed a multispecific antibody comprising three antigen binding sites, wherein
a) the first antigen binding site is formed by a first circular fusion polypeptide, comprising a first part of a first binding domain, a second part of a first binding domain and a first part of a third binding domain, wherein
   - the first part of the first binding domain is fused either directly or via a first peptidic linker to the N-terminus of the first part of the third binding domain,
   - the second part of the first binding domain is fused either directly or via a second peptidic linker to the C-terminus of the first part of the third binding domain,
   - the first part of the first binding domain is a heavy chain Fab fragment (VH₁-CH1) or a light chain Fab fragment (VL₁-CL), whereby the second part of the first binding domain is a light chain Fab fragment if the first part of the first binding domain is a heavy chain Fab fragment or vice versa,
   - the first part of the first binding domain and the second part of the first binding domain are associated with each other and form the first antigen binding site,
b) the second antigen binding site is formed by a second circular fusion polypeptide, comprising a first part of a second binding domain, a second part of a second binding domain and the second part of the third binding domain, wherein
   - the first part of the second binding domain is fused either directly or via a third peptidic linker to the N-terminus of the second part of the third binding domain,
   - the second part of the second binding domain is fused either directly or via a fourth peptidic linker to the C-terminus of the second part of the third binding domain,
   - the first part of the second binding domain is a heavy chain Fab fragment (VH₂-CH1) or a light chain Fab fragment (VL₂-CL), whereby the second part of the second binding domain is a light chain Fab fragment if the first part of the second binding domain is a heavy chain Fab fragment or vice versa,
   - the first part of the second binding domain and the second part of the second binding domain are associated with each other and form the second antigen binding site,
c) the third antigen binding site is formed by the first part of the third binding domain and the second part of the third binding domain, wherein
   - the first part of the third binding domain is either a variable heavy chain-CH3 domain fusion polypeptide (VH₃-CH3) or a variable light chain-CH3 domain fusion polypeptide (VL₃-CH3),
   - the second part of the third binding domain is a variable heavy chain-CH3 domain fusion polypeptide if the first part of the second binding domain in the first circular fusion polypeptide is a variable light chain-CH3 domain fusion polypeptide or vice versa,
   - the first part of the third binding domain and the second part of the third binding domain are associated with each other and form the third antigen binding site,
wherein the two constant heavy chain domains 3 (CH3) are altered to promote heterodimerization by
i) generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains, or
   substituting at least one original amino acid residue in one of the CH3 domains by a positively charged amino acid, and substituting at least one original amino acid residue in the other one of the CH3 domains by a negatively charged amino acid, or
ii) introduction of at least one cysteine residue in each CH3 domain such that a disulfide bond is formed between the CH3 domains, or
iii) both modifications of i) and ii),
wherein the multispecific antibody is devoid of constant heavy chain domains 2 (CH2) and a hinge region.

In one embodiment the multispecific antibody comprises three antigen binding sites, wherein
a) the first antigen binding site is formed by a first antibody heavy chain variable domain (VH₁) and a first antibody light chain variable domain (VL₁) pair (VH₁/VL₁-pair) of a first circular fusion polypeptide, wherein
   - the VH₁ is part of a first heavy chain Fab fragment (VH₁-CH1) and the VL₁ is part of a first light chain Fab fragment (VL₁-CL),
   - the VH₁-CH1 is conjugated via a first peptidic linker to either the N-terminus or the C-terminus of a first variable domain-linker-antibody constant domain 3 fusion polypeptide (V₃-L-CH3) and the VL₁-CL is conjugated via a second peptidic linker to the respective other terminus of the first V₃-CH3,
   - the VH₁-CH1 and the VL₁-CL are associated with each other and form the VH₁/VL₁-pair,
b) the second antigen binding site is formed by a second antibody heavy chain variable domain (VH₂) and a second antibody light chain variable domain (VL₂) pair (VH₂/VL₂-pair) of a second circular fusion polypeptide, wherein
   - the VH₂ is part of a second heavy chain Fab fragment (VH₂-CH1) and the VL₂ is part of a second light chain Fab fragment (VL₂-CL),
   - the VH₂-CH1 is conjugated via a third peptidic linker to either the N-terminus or the C-terminus of a second variable domain-linker-antibody constant domain 3 fusion polypeptide (V₃-L-CH3) and the VL₂-CL is conjugated via a fourth peptidic linker to the respective other terminus of the second V₃-CH3,
   - the VH₂-CH1 and the VL₂-CL are associated with each other form the VH₂/VL₂-pair,
c) the third antigen binding site is formed by the first V₃-L-CH3 and the second V₃-L-CH3, wherein
   - the first V₃-L-CH3 is either a variable heavy chain domain-linker-antibody constant domain 3 fusion polypeptide (VH₃-L-CH3) or a variable light chain domain-linker-antibody constant domain 3 fusion polypeptide (VL₃-L- CH3),
   - the second V₃-L-CH3 is a variable heavy chain domain-linker-antibody constant domain 3 fusion polypeptide if the first V₃-L-CH3 is a variable light chain domain-linker-antibody constant domain 3 fusion polypeptide or vice versa,
   - the VH₃-L-CH3 and the VL₃-L-CH3 are associated with each other form the VH₃/VL₃-pair,
wherein the two antibody constant domains 3 (CH3) are altered to promote heterodimerization by introducing the mutation T366W and optionally the mutation S354C in one of the CH3s and the mutations T366S, L368A and Y407V and optionally the mutation Y349C in the respective other CH3 (numberings according to EU index of Kabat),
wherein the multispecific antibody is devoid of antibody constant domains 2 (CH2) and a hinge region.

The first part of the first and/or second binding domain and the respective second part thereof can be non-covalently or covalently associated with each other. If the association is covalently it is by a bond other than a peptide bond, such as e.g. by a disulfide bond.

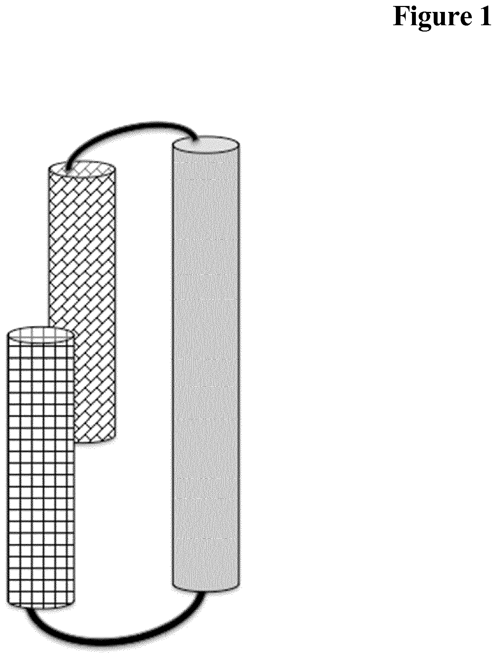

The circular fusion polypeptides contained in the multispecific antibody as reported herein are each single chain polypeptides. The general structure of an isolated circular fusion polypeptide is shown in Figure 1.

The multispecific antibody as reported herein is based on a dimeric circular fusion polypeptide comprising a first circular fusion polypeptide and a second circular fusion polypeptide, i.e. it is dicircular, wherein the first and the second circular fusion polypeptide are identical or different.

The circular fusion polypeptides of the multispecific antibody as reported herein comprise beside the N- and C-terminally fused parts of the respective binding domains a core region comprising a part of the third binding site as well as a heterodimerization domain. In this case the structural property of the core region is the provision of a dimerization functionality. The heterodimerization domain of the first circular fusion polypeptide can be conjugated to the heterodimerization domain of the second circular fusion polypeptide by at least one non-peptide bond, in one embodiment by at least one disulfide bond.

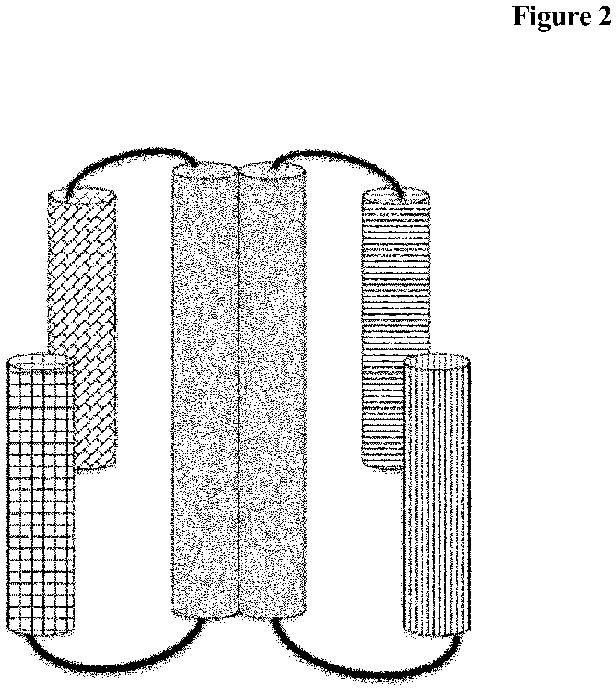

The basic dicircular fusion polypeptides herein are also termed Contorsbody. The general structure of the dicircular fusion polypeptide/Contorsbody is shown in Figure 2.

Compared to normal IgG antibodies, the Contorsbody is using only one chain and not a heavy and a light chain for providing a binding site (VH/VL-pair). The particularity of the Contorsbody based multispecific antibody as reported herein is that a third binding site (VH/VL-pair) is formed by the dimerization of two circular fusion polypeptides and this third binding site/part of the binding domain is located in between the heavy chain Fab fragment and the light chain Fab fragment.

The dimeric circular fusion polypeptide without third binding site is used in the following to show the specific properties of the dicircular fusion polypeptide forming the basis of the multispecific antibody as reported herein. Beside the Fab fragments also isolated variable domains could be used as parts of a binding site.

In this example single chain fusion polypeptides with a "hinge-CH2-CH3" domain as dimerization domain in between the heavy chain Fab fragment and the light chain Fab fragment dimerize via their Fc-regions, wherein the two Fabs are fixed in their orientation to each other. In contrast, in a normal IgG type antibody the two Fabs are more flexible and much differently oriented.

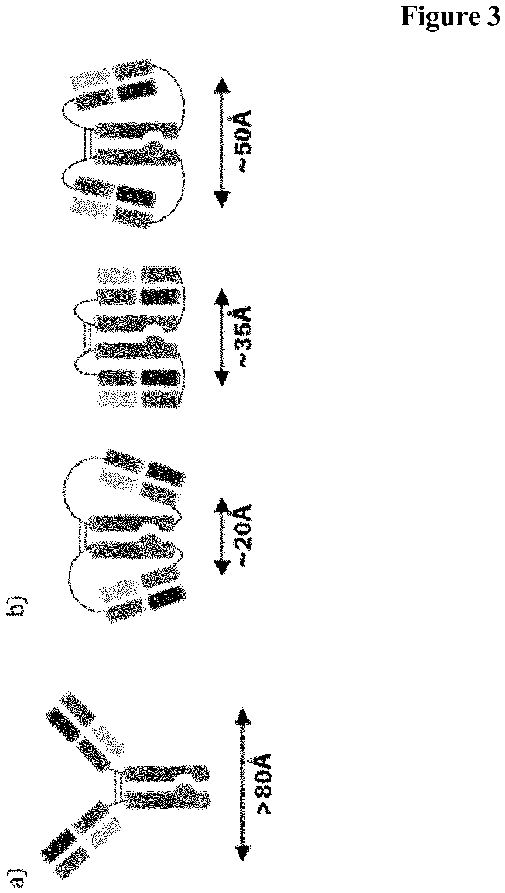

The geometry of the two binding sites relative to each other can be modulated in a Contorsbody by the length of the employed linkers. The orientation and spatial distance between the binding sites of a normal antibody of the IgG type and a dicircular fusion polypeptide forming the basis of the multispecific antibody as reported herein are shown in Figure 3.

The spatial distance between the binding sites of a conventional antibody of the IgG type is about 80 Å (angstrom) or more. The spatial distance between the binding sites of a dicircular fusion polypeptide as reported herein can be between about 20 Å to about 50 Å depending on the length and length ratio of the peptidic linker in the individual circular fusion polypeptides forming the dicircular fusion polypeptide. Depending on the intended geometry the peptidic linker is selected. In one embodiment the first to fourth peptidic linker is independently of each other selected from the group of peptidic linker consisting of SEQ ID NO: 06 to SEQ ID NO: 22.

### II.1. Comparative monospecific, bivalent antibodies formed from dicircular fusion polypeptides

A monospecific dicircular fusion polypeptide comprises two circular fusion polypeptides wherein each of the circular fusion polypeptides specifically binds to the same epitope on the same target, i.e. comprises the same binding site.

An exemplary monospecific dicircular fusion polypeptide is an anti-Her2 Contorsbody (comprising the circular fusion polypeptide of SEQ ID NO: 23). It was produced by transfecting HEK293 cells with a vector containing a nucleotide sequence encoding the circular fusion polypeptide.

It has been found that a conventional protein A affinity chromatography is suitable to extract the Fc-region containing part of the cultivation supernatant. Alternatively, a hexahistidine C-terminal tag (SEQ ID NO: 24) connected via a GSG peptidic linker for purification purpose can be used.

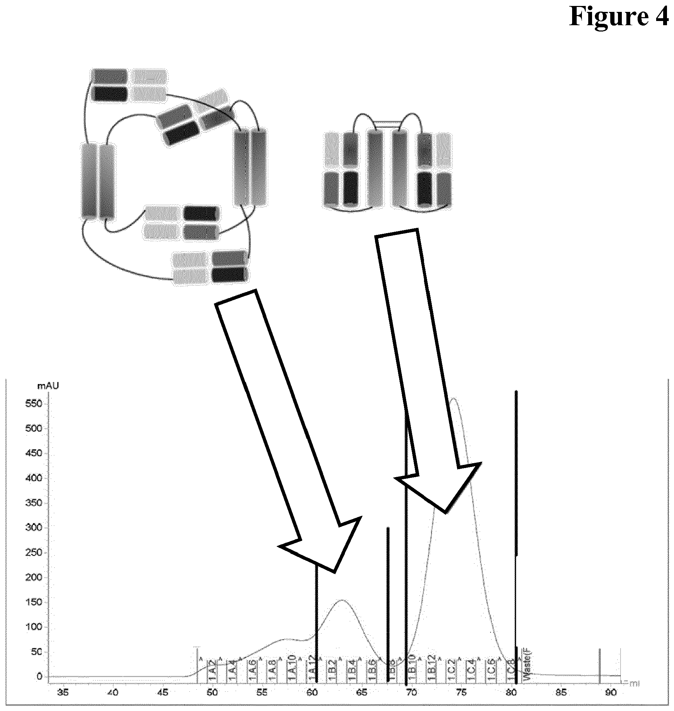

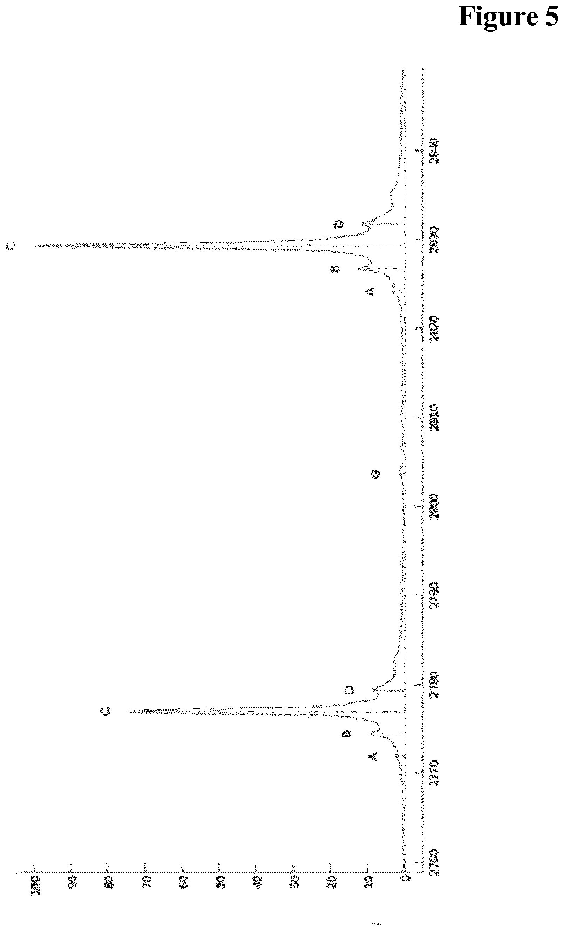

Preparative size exclusion chromatography was used in the second purification step to separate the circular fusion polypeptide from product related impurities, mostly higher order structures of the circular fusion polypeptide (a small portion of aggregates is also seen in the chromatogram as it is also for an antibody of the IgG type) (see e.g. Figure 4). Typical yield for such constructs is averaging 10 mg/liter. The anti-Her2 Contorsbody has been expressed in several batches (from 0.5 liter to 2 liter shake flask scale). Product quality has been analyzed by mass spectrometry (see Figure 5). The identity of the product was confirmed with a purity grade above 95%.

The binding of the anti-Her2 Contorsbody has been determined using surface plasmon resonance (SPR, e.g. BIAcore) in two different settings in order to assess the affinity and the avidity of the molecules compared to a conventional anti-Her2 antibody of the IgG type.

In the first setting (1 in the following Table; for affinity) the respective anti-Her2 Contorsbody was captured by an anti-human Fc-region antibody conjugated to the SPR chip surface. As analyte the Her2 extracellular domain (ECD) was used. In the second setting (2 in the following Table; for avidity) the anti-Her2 antibody pertuzumab (marketed as Perjeta^{®}) was immobilized on the chip surface and the ECD of Her2 was captured thereby. As analyte the respective Contorsbody was used. The avidity of the IgG type reference antibody trastuzumab, the bivalent anti-Her2 Contorsbody was measured using concentration series of the analyte. As reference in both setting the anti-Her2 antibody trastuzumab (marketed as Herceptin^{®}) has been used.

| **setting** | **molecule** | **kₐ [M⁻¹s⁻¹]** | **k_{d} [s⁻¹]** | **t(1/2) [min]** | **K_{D} [M]** | **ratio Rₘₐₓ exp./theor.** |
|---|---|---|---|---|---|---|
| 1 | trastuzumab | 6.8E+05 | 5.2E-04 | 22.2 | 7.7E-10 | 114 |
| 1 | Contorsbody | 2.1E+05 | 1.9E-03 | 6.1 | 9.1E-09 | 41 |
| 2 | trastuzumab | 1.03E+06 | 6.36E-05 | 181.7 | 6.2E-11 | 101.5 |
| 2 | Contorsbody | 1.11E+06 | 6.59E-05 | 175.4 | 5.9E-11 | 102.3 |

The Contorsbody is much more compact compared to a conventional antibody of the IgG type.

The anti-Her2 Contorsbodies have been tested in a proliferation assay. Similarly, to Scheer et al. (Scheer et al., PLoS One 7 (2012) e51817) who used chemically cross-linked trastuzumab Fabs, the anti-Her2 Contorsbodies recruited receptors on the cell surface and promoted an activation signal. Trastuzumab, a binder for an Her2 epitope located on the receptor stalk domain, is keeping the receptors away from each other and, and consequently is antagonizing the proliferation. Trastuzumab and the dicircular anti-Her2 Contorsbody, respectively, are antiproliferative and pro-proliferative.

The ability of the anti-Her2 Contorsbodies to bind FcRn receptor has been determined. Compared to trastuzumab, an antibody of the IgG type, IgG1 subclass, the binding of the anti-Her2 Contorsbodies to human FcRn receptor is surprisingly higher, i.e. around 10x for the bicircular Contorsbody. Against cynomolgus FcRn trastuzumab and the Contorsbodies behave similarly, the dimeric Contorsbody being 4.5 times more affine than trastuzumab.

Using tryptophan autofluorescence, the first thermal denaturation temperature Tₘ1 is approx. 63 °C for the Contorsbody. Using static light scattering, an aggregation onset temp. of approx. 66 °C was determined for the Contorsbody. Using dynamic light scattering, an aggregation onset temp. of approx. 66°C was determined for the Contorsbody. In all experiments the formulation was 1 mg/mL Contorsbody in 20 mM His/His^{∗}HCl, 140 mM NaCl.

It could be confirmed by mass spectrometry that no mixed Fabs are formed.

Other examples of a dicircular mono-specific Contorsbodies are anti-cMET Contorsbody (circular fusion polypeptide of SEQ ID NO: 25), and anti-CD20 Contorsbodies with different variable domains ((1) circular fusion polypeptide of SEQ ID NO: 26; (2) circular fusion polypeptide of SEQ ID NO: 27). In the following Table the expression rate, the yield and the quality of these Contorsbodies are given.

| | anti-cMET Contorsbody | anti-CD20 Contorsbody (1) | anti-CD20 Contorsbody (2) |
|---|---|---|---|
| expression volume [ml] | 250 | 500 | 500 |
| concentration [mg/ml] | 0.60 | 1.06 | 1.0 |
| amount (mg) | 0.78 | 3.1 | 5.7 |
| % monomer (analytical SEC) | 100.00 | 97.9 | 98.4 |
| % main peak (CE-SDS) | 96.30 | 99.3 | 99.6 |
| yield [mg/1] | 3.12 | 6.2 | 11.4 |

All Contorsbodies have been expressed transiently in HEK-293 cells with yields ranging from 2 to 15 mg/L. The product after protein A column is above 85 % and is purified from side-products by SEC column chromatography up to a purity above 96 % in general.

### II.2. Comparative bispecific, bivalent antibodies formed from dicircular fusion polypeptides

A bispecific multicircular fusion polypeptide comprises two circular fusion polypeptides wherein each of the circular fusion polypeptides specifically binds to a different target and/or to a different epitope on the same target, i.e. comprises at least two binding sites of different specificity.

Without being bound by this theory it is postulated that the self-assembly of the corresponding domains of the binding sites in the single chain polypeptide is prevalent to inter-chain association, i.e. in other words, after expression of a single circular fusion polypeptide the individual, non-functional domains of the binding site associate and form a functional binding site. For example, if the functional binding site is a Fab the Fab portions, i.e. the heavy chain fragment (VH-CH1) and the light chain fragment (VL-CL), form a constitutive, binding competent Fab moiety and the orphan half antibody of this first assembled circular fusion polypeptide associates consecutively with another circular fusion polypeptide to form a bicircular fusion polypeptide (Contorsbody comprising a dimer of two single circular fusion polypeptides). In order to obtain a multispecific multicircular fusion polypeptide the heterodimerization of the isolated circular fusion polypeptides has to be promoted. One exemplary heterodimerization promoting element are the mutations according to the knob-into-hole technology.

It is possible to modify the length of the peptidic linker in order to vary the geometry/distance of the two binding sites of the resulting bispecific Contorsbody; the same is also true for monospecific Contorsbodies.

It is possible to modify the geometry/distance of the binding sites by changing the relative position(s) of the binding site(s) to each other.

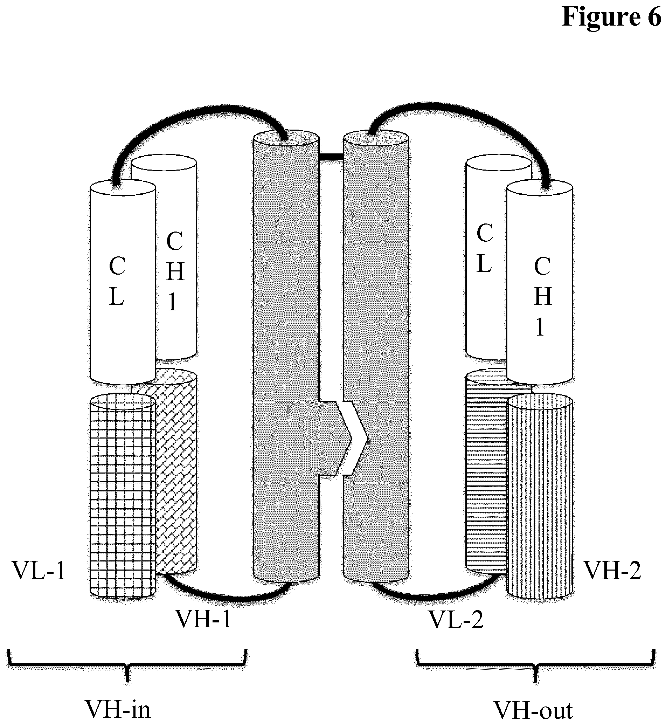

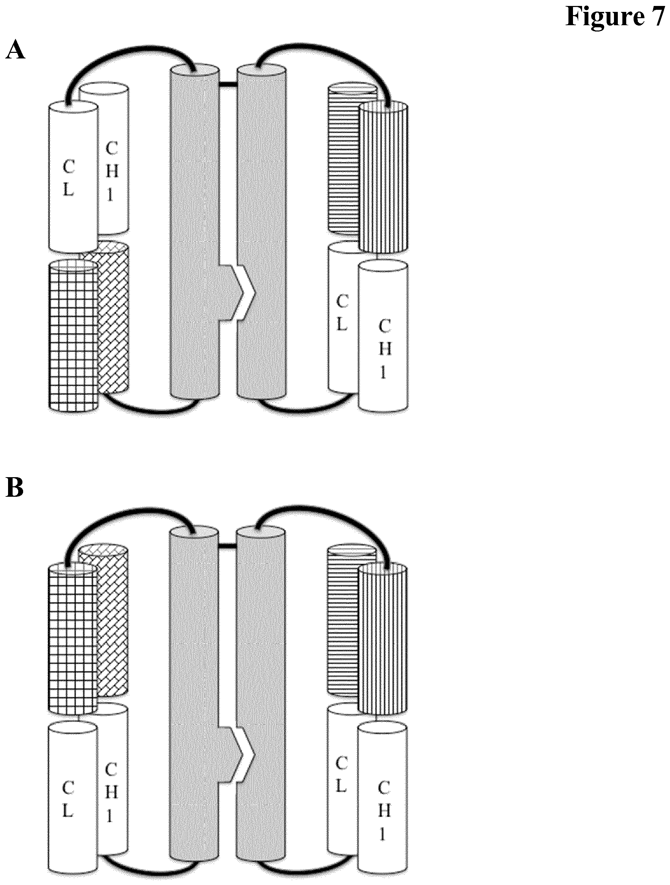

For example, in case of a Fab as binding site, the sequence of the domains of the heavy chain Fab fragment and the light chain Fab fragment can be inverted, i.e., e.g., the heavy chain Fab fragment can have the domain sequence VH-CH1 or CH1-VH (from N- to C-terminus), respectively, and likewise the light chain Fab fragment can have the domain sequence VL-CL or CL-VL (from the N- to C-terminus) or mixed. Assuming that a linker is present before and after the central fusion polypeptide of the part of the third binding site and the core domain, i.e. before and after the fusion polypeptide of the part of the third binding site and the antibody constant domain 3, the Fab fragment is limited in its orientations with regard to the core domain. Consequently, the relative position of the VH domain is either close to the Fc-region, called here "VH-in", or more apart from the Fc-region, called here "VH-out" (see Figures 6 and 7).

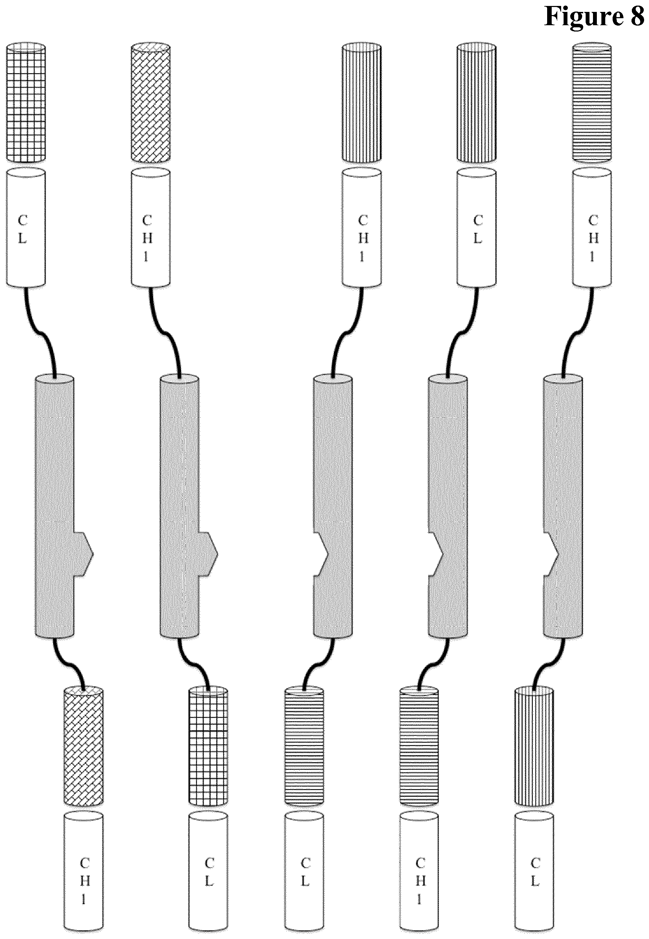

It is possible to also modify the assembly behavior in using the CrossMab technology, i.e. a domain exchange in one arm. This can further be combined with charge variants in the exchanged or non-exchanged arm. Exemplary chains of anti-cMET circular fusion polypeptides with the respective orientation used for the production of bispecific bicircular fusion polypeptides are shown in Figure 8 (VH-out, VH-in, and the respective expression rate, yield and quality of these different chain combinations are given in the following Table.

| | VH-out-knob/VH -out-hole | VH-in-knob/VH -out-hole | VH-out-knob/VH -out-hole-CH-CL-crossed | VH-in-knob/VH -out-hole-CH-CL-crossed | VH-out-knob/VH -in-hole-VH-VL-crossed | VH-in-knob/VH -in-hole-VH-VL-crossed |
|---|---|---|---|---|---|---|
| expression volume [ml] | 250 | 250 | 250 | 250 | 250 | 250 |
| concentratio n [mg/ml] | 1.80 | 0.60 | 0.48 | 1.17 | 0.36 | 0.65 |
| amount [mg] | 2.88 | 0.60 | 0.81 | 1.17 | 0.43 | 0.65 |
| monomer (analytical SEC) [%] | 98.10 | 93.80 | 97.34 | 97.90 | 98.74 | 98.88 |
| main peak (CE-SDS) [%] | 96.80 | 98.80 | 95.73 | 100.00 | 100.00 | 100.00 |
| yield [mg/l] | 11.52 | 2.40 | 3.24 | 4.68 | 1.72 | 2.60 |

| | | | | | | |
|---|---|---|---|---|---|---|
| VH-out-knob = SEQ ID NO: 28, VH-in-knob = SEQ ID NO: 29, VH-out-hole = SEQ ID NO: 30, VH-out-hole-CH-CL-crossed = SEQ ID NO: 31, VH-in-hole-VH-VL-crossed = SEQ ID NO: 32. | | | | | | |

The generally applicable techniques for making conventional bispecific antibodies can also be used and adopted to make bispecific dicircular fusion polypeptides.

For example, techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A. et al., EMBO J. 10 (1991) 3655-3659), and "knob-in-hole" engineering (see, e.g., US 5,731,168). Multispecific antibodies may also be made by engineering electrostatic steering effects for making antibody Fc-heterodimeric molecules (WO 2009/089004); cross-linking two or more antibodies or fragments (see, e.g., US 4,676,980, and Brennan, M. et al., Science 229 (1985) 81-83); using leucine zippers to produce bi-specific antibodies (see, e.g., Kostelny, S.A. et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (sFv) dimers (see, e.g. Gruber, M et al., J. Immunol. 152 (1994) 5368-5374); and preparing trispecific antibodies as described, e.g., in Tutt, A. et al., J. Immunol. 147 (1991) 60-69).

Generally, each time a geometrical constraint is required to fix the orientation of two binding sites, a Contorsbody can be used.

### II.3 Bi- and trispecific, trivalent antibodies formed from dicircular fusion polypeptides according to the current invention

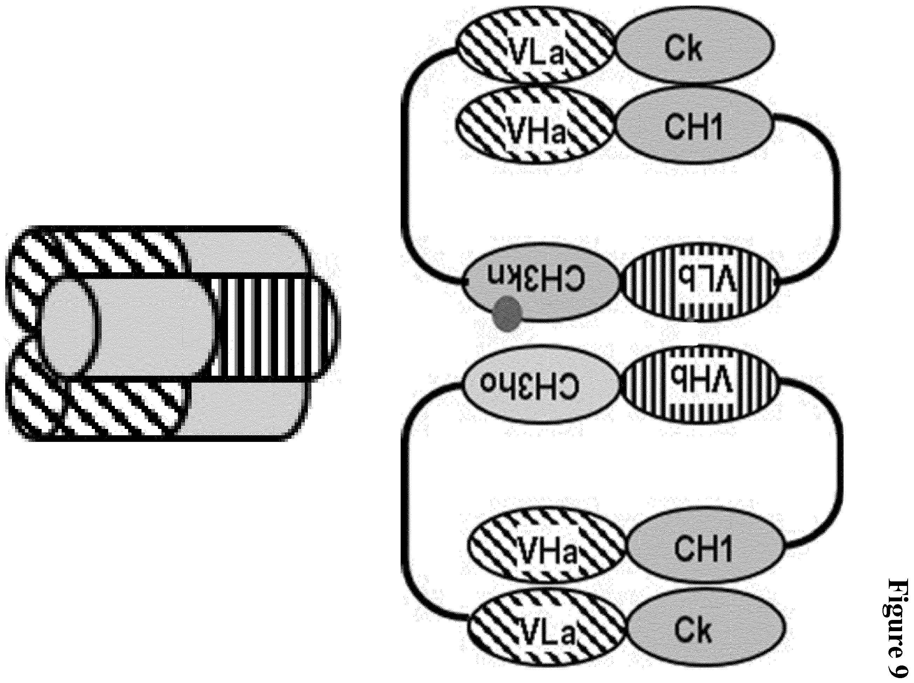

The design and composition of an exemplary multispecific antibody according to the current invention is shown in Figure 9. Such an antibody comprising three binding sites can be designated as TriFab-Contorsbody.

From Figure 9 lower part it can be seen that the binding sites are arranged in opposite position in a TriFab-Contorsbody. Thus, a TriFab-Contorsbody is tight molecule with defined and limited flexibility. It has a very special and defined geometry. Upon binding to its antigens it connects two antigens (cells presenting said antigens) like a magnet with poles.

An exemplary TriFab-Contorsbody is an anti-LeY/biotin antibody. It comprises two circular fusion polypeptides, whereof the first has the sequence of domains as follows:
anti-LeY antibody VH - CH1 - peptidic linker 1 - anti-biotin antibody VL ― linker - CH3 with knob mutation ― peptidic linker 2 ― anti-LeY antibody VL ― CL(kappa)

The respective amino acid sequence is listed in SEQ ID NO: 34.

The second circular fusion polypeptide has the sequence of domains as follows:
anti-LeY antibody VH - CH1 - peptidic linker 1 - anti-biotin antibody VL ― linker - CH3 with hole mutation ― peptidic linker 2 ― anti-LeY antibody VL ― CL(kappa)

The respective amino acid sequence is listed in SEQ ID NO: 35.

The molecule was expressed and purified alike the method described in the Examples.

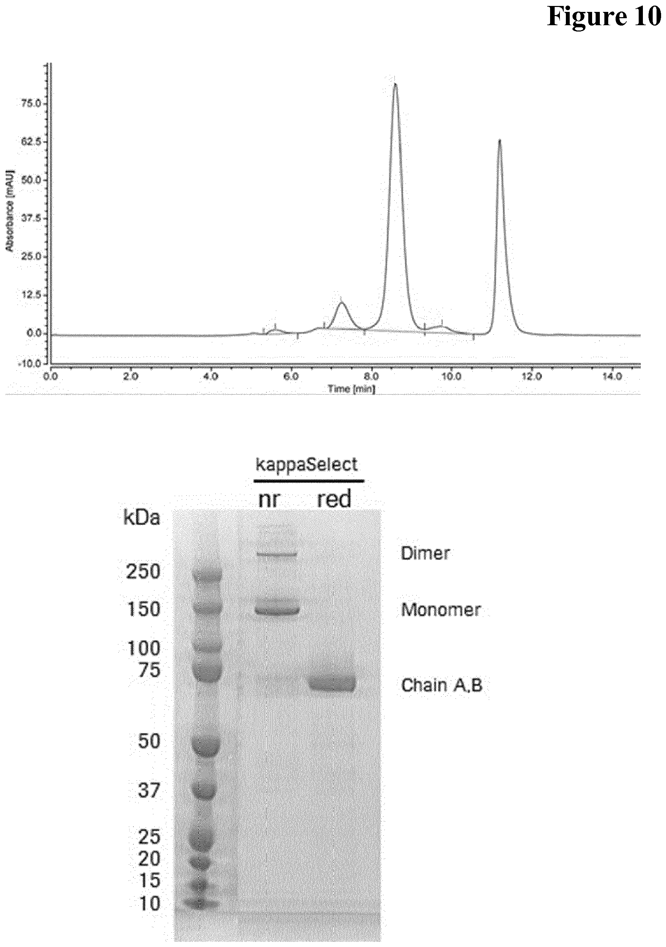

Figure 10 shows the SEC and the SDS-page of the KappaSelect purified cultivation supernatant.

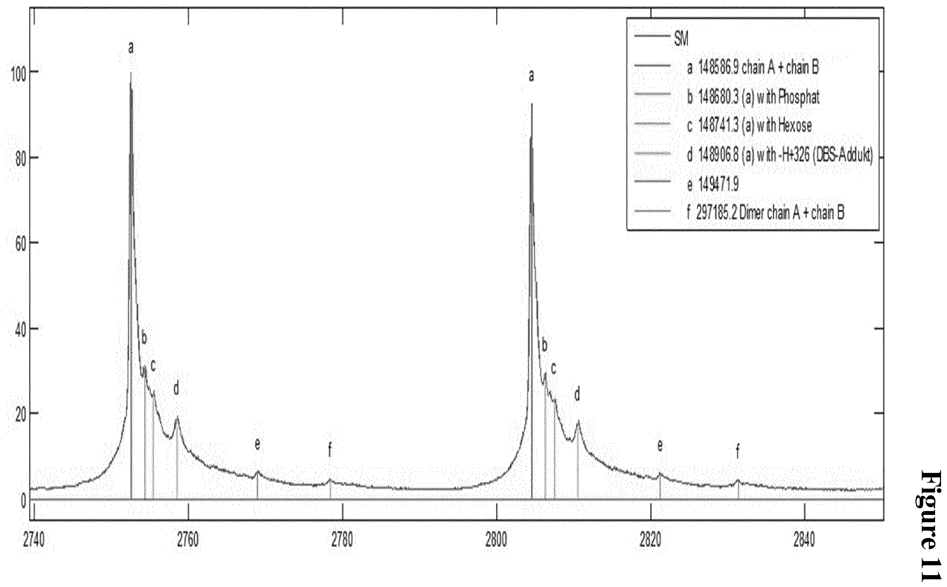

The identity of the molecule was confirmed by MS as shown in Figure 11 in the data in the following Table:

| | **mass expected [Da]** | **mass determined [Da]** |
|---|---|---|
| whole molecule | 148582 | 148586 |
| chain A | 73635 | 73635 |
| chain B | 74980 | 74980 |

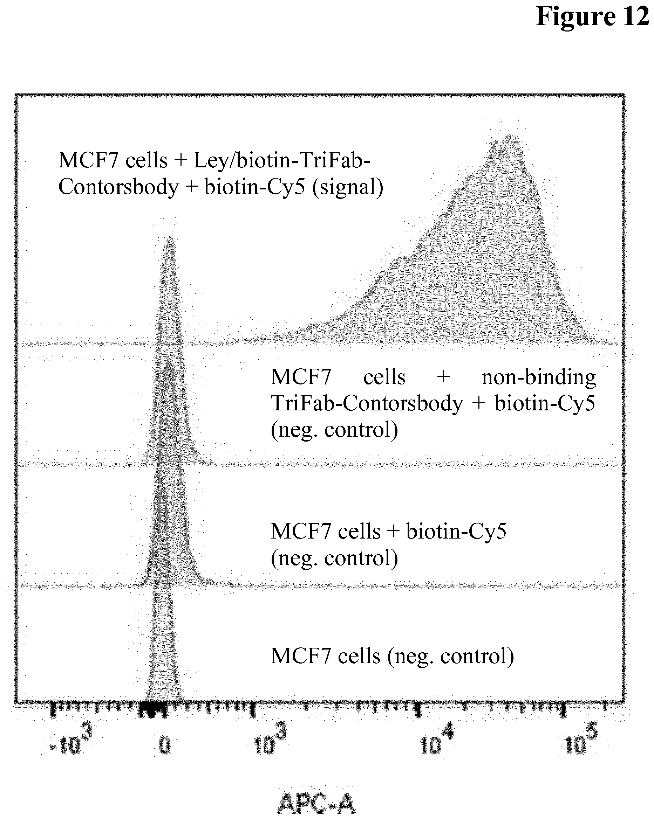

The functionality of the molecule was confirmed by FACS analyses demonstrating bispecificity of the LeY-Bio TriFab-Contorsbody. The TriFab-Contorsbody binds to the cell surface via its anti-LeY binding specificity. By capture of the fluorescent biotin-Cy5 conjugate via its anti-biotin binding site the cell-bound TriFab-Contorsbody becomes detectable in the FACS analysis, i.e. cell-associated Cy5 signals indicate simultaneous functionality of both binding sites, i.e. of the binding sites located in the individual circular fusion polypeptides as well as the binding site formed by the dimerization of the two circular fusion polypeptides. The FACS data is shown in Figure 12.

The invention is a multispecific antibody comprising three antigen binding sites and consisting of two circular fusion polypeptides, wherein
a) the first circular fusion polypeptide comprises a first part of a first binding domain, a second part of a first binding domain, and a first part of a third binding domain, wherein
   - the first part of the first binding domain is fused either directly or via a first peptidic linker to the N-terminus of the first part of the third binding domain,
   - the second part of the first binding domain is fused either directly or via a second peptidic linker to the C-terminus of the first part of the third binding domain,
   - the first part of the first binding domain is a heavy chain Fab fragment (VH₁-CH1) or a light chain Fab fragment (VL₁-CL), whereby the second part of the first binding domain is a light chain Fab fragment if the first part of the first binding domain is a heavy chain Fab fragment or vice versa,
   - the first part of the first binding domain and the second part of the first binding domain are associated with each other and are together the first antigen binding site,
b) the second circular fusion polypeptide comprises a first part of a second binding domain, a second part of a second binding domain, and the second part of the third binding domain, wherein
   - the first part of the second binding domain is fused either directly or via a third peptidic linker to the N-terminus of the second part of the third binding domain,
   - the second part of the second binding domain is fused either directly or via a fourth peptidic linker to the C-terminus of the second part of the third binding domain,
   - the first part of the second binding domain is a heavy chain Fab fragment (VH₂-CH1) or a light chain Fab fragment (VL₂-CL), whereby the second part of the second binding domain is a light chain Fab fragment if the first part of the second binding domain is a heavy chain Fab fragment or vice versa,
   - the first part of the second binding domain and the second part of the second binding domain are associated with each other and are together the second antigen binding site,
c) the first part of the third binding domain and the second part of the third binding domain are together the third antigen binding site, wherein
   - the first part of the third binding domain is either a variable heavy chain-CH3 domain fusion polypeptide (VH₃-CH3) or a variable light chain-CH3 domain fusion polypeptide (VL₃-CH3),
   - the second part of the third binding domain is a variable heavy chain-CH3 domain fusion polypeptide if the first part of the second binding domain in the first circular fusion polypeptide is a variable light chain-CH3 domain fusion polypeptide or vice versa,
   - the first part of the third binding domain and the second part of the third binding domain are associated with each other and form the third antigen binding site,
wherein the two constant heavy chain domains 3 (CH3) are altered to promote heterodimerization by
i) generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains, or substituting at least one original amino acid residue in one of the CH3 domains by a positively charged amino acid, and substituting at least one original amino acid residue in the other one of the CH3 domains by a negatively charged amino acid, or
ii) introduction of at least one cysteine residue in each CH3 domain such that a disulfide bond is formed between the CH3 domains, or
iii) both modifications of i) and ii),
wherein the multispecific antibody is devoid of constant heavy chain domains 2 (CH2) and a hinge region.

In one embodiment of all aspects the first and the second circular fusion polypeptide each comprises exactly one part of a binding domain N-terminal to the part of the third antigen binding domain and exactly one, but different, part of the binding domain C-terminal to the part of the third binding domain.

In one embodiment of all aspects the first part of the first binding domain and the second part of the first binding domain in the first circular fusion polypeptide are covalently associated with each other and/or the first part of the second binding domain and the second part of the second binding domain in the second circular fusion polypeptide are covalently associated with each other. In one embodiment the covalent association is by a bond other than a peptide bond. In one preferred embodiment the covalent association is by a disulfide bond.

In one embodiment of all aspects
i) the C-terminus of the CH1 domain of the first part of the first or second binding site is conjugated to the N-terminus of the part of the third binding domain, and the N-terminus of the VL domain of the second part of the first or second binding site is conjugated to the C-terminus of the third binding domain, or
ii) the C-terminus of the VH domain of the first part of the first or second binding site is conjugated to the N-terminus of the part of the third binding domain, and the N-terminus of the CL domain of the second part of the first or second binding site is conjugated to the C-terminus of the third binding domain.

In one embodiment of all aspects the third antigen binding site is disulfide stabilized by introduction of cysteine residues independently of each other at the following positions to form a disulfide bond between the VH and VL domains (numbering according to Kabat):
- VH at position 44, and VL at position 100,
- VH at position 105, and VL at position 43, or
- VH at position 101, and VL at position 100.

In one embodiment of all aspects the first part of the first or the second binding domain is an antibody heavy chain Fab fragment (VH+CH1) and the second part of the first or the second binding domain is an antibody light chain Fab fragment (VL+CL) or vice versa.

In one embodiment of all aspects the multispecific antibody fusion polypeptide exerts effector function. In one embodiment the effector function is ADCC or/and CDC.

In one embodiment of all aspects the first part of the first binding domain is fused via a first peptidic linker of SEQ ID NO: 16 or SEQ ID NO: 17 to the N-terminus of the part of the third binding domain and the second part of the first binding domain is fused via a second peptidic linker of SEQ ID NO: 16 or SEQ ID NO: 17 to the C-terminus of the part of the third binding domain, whereby the first and the second peptidic linker are selected independently of each other.

In one embodiment of all aspects the first part of the second binding domain is fused via a first peptidic linker of SEQ ID NO: 16 or SEQ ID NO: 17 to the N-terminus of the part of the third binding domain and the second part of the second binding domain is fused via a second peptidic linker of SEQ ID NO: 16 or SEQ ID NO: 17 to the C-terminus of the part of the third binding domain, whereby the first and the second peptidic linker are selected independently of each other.

In one embodiment of all aspects each (circular) (single chain) fusion polypeptide comprises in N- to C-terminal direction before the part of the third binding domain (i.e. N-terminal to the part of the third binding domain) exactly one antibody variable domain and after the part of the third binding domain (i.e. C-terminal to the part of the third domain) exactly one antibody variable domain.

In one embodiment of all aspects the target is a cell surface antigen or the soluble ligand of a cell surface receptor.

In one embodiment of all aspects the binding domain is a Fab, a DAF or a bispecific Fab.

In one embodiment of all aspects the first and/or the second binding domain is a (conventional) Fab, wherein the first part of the binding domain comprises an antibody heavy chain variable domain (VH) and at least an N-terminal fragment of a (or a complete) first antibody heavy chain constant domain (CH1) and the respective other part of the binding domain comprises an antibody light chain variable domain (VL) and at least an N-terminal fragment of a (or a complete) antibody light chain constant domain (CL), or vice versa. In one embodiment the first part of the binding domain comprises in N- to C-terminal direction VH-CH1 and the second part of the binding domain comprises in N- to C-terminal direction VL-CL, or vice versa.

In one embodiment of all aspects the amino acid residue having a larger side chain volume than the original amino acid residue is selected from R, F, Y and W.

In one embodiment of all aspects the amino acid residue having a smaller side chain volume than the original amino acid residue is selected from A, S, T and V.

In one embodiment of all aspects the amino acid residue having a larger side chain volume than the original amino acid residue is selected from R, F, Y and W, and the amino acid residue having a smaller side chain volume than the original amino acid residue is selected from A, S, T and V.

In one embodiment of all aspects one CH3 domain of the third binding site comprises a T366W mutation (knob mutation; knob side), and the other CH3 domain of third binding site comprises the mutations T366S, L368A and Y407V (hole mutations; hole side) (numberings according to EU index of Kabat).

In one embodiment of all aspects one CH3 domain of the third binding site comprises a T366W mutation (knob mutation; knob side), and the other CH3 domain of third binding site comprises the mutations T366S, L368A and Y407V (hole mutations; hole side) (numberings according to EU index of Kabat).

In one embodiment of all aspects one CH3 domain of the third binding site comprises the S354C and T366W mutations (knob-cys mutation; knob-cys side), and the other CH3 domain of third binding site comprises the mutations Y349C, T366S, L368A and Y407V (hole-cys mutations; hole-cys side) (numberings according to EU index of Kabat).

One aspect as reported herein is a multispecific antibody comprising three antigen binding sites, wherein
a) the first antigen binding site is formed by a first antibody heavy chain variable domain (VH₁) and a first antibody light chain variable domain (VL₁) pair (VH₁/VL₁-pair) of a first circular fusion polypeptide, wherein
   - the VH₁ is part of a first heavy chain Fab fragment (VH₁-CH1) and the VL₁ is part of a first light chain Fab fragment (VL₁-CL),
   - the VH₁-CH1 is conjugated via a first peptidic linker to either the N-terminus or the C-terminus of a first variable domain-antibody constant domain 3 fusion polypeptide (V₃-CH3) and the VL₁-CL is conjugated via a second peptidic linker to the respective other terminus of the first V₃-CH3,
   - the VH₁-CH1 and the VL₁-CL are associated with each other and form the VH₁/VL₁-pair,
b) the second antigen binding site is formed by a second antibody heavy chain variable domain (VH₂) and a second antibody light chain variable domain (VL₂) pair (VH₂/VL₂-pair) of a second circular fusion polypeptide, wherein
   - the VH₂ is part of a second heavy chain Fab fragment (VH₂-CH1) and the VL₂ is part of a second light chain Fab fragment (VL₂-CL),
   - the VH₂-CH1 is conjugated via a third peptidic linker to either the N-terminus or the C-terminus of a second variable domain-antibody constant domain 3 fusion polypeptide (V₃-CH3) and the VL₂-CL is conjugated via a fourth peptidic linker to the respective other terminus of the second V₃-CH3,
   - the VH₂-CH1 and the VL₂-CL are associated with each other form the VH₂/VL₂-pair,
c) the third antigen binding site is formed by the first V₃-CH3 and the second V₃-CH3, wherein
   - the first V₃-CH3 is either a variable heavy chain-antibody constant domain 3 fusion polypeptide (VH₃-CH3) or a variable light chain-antibody constant domain 3 fusion polypeptide (VL₃-CH3),
   - the second V₃-CH3 is a variable heavy chain-antibody constant domain 3 fusion polypeptide if the first V₃-CH3 is a variable light chain-antibody constant domain 3 fusion polypeptide or vice versa,
   - the VH₃-CH3 and the VL₃-CH3 are associated with each other form the VH₃/VL₃-pair,
wherein the two antibody constant domains 3 (CH3) are altered to promote heterodimerization by introducing the mutation T366W and optionally the mutation S354C in one of the CH3s and the mutations T366S, L368A and Y407V and optionally the mutation Y349C in the respective other CH3 (numberings according to EU index of Kabat),
wherein the multispecific antibody is devoid of antibody constant domains 2 (CH2) and a hinge region.

In one embodiment the linker in the first and/or second part of the third binding domain is absent (i.e. the first part of the third binding domain is a variable heavy chain domain-antibody constant domain 3 fusion polypeptide and the second part of the third binding domain is a variable light chain domain-antibody constant domain 3 fusion polypeptide, or vice versa).

In one embodiment the linker L is a peptidic linker. In one embodiment the linker L has the amino acid sequence of SEQ ID NO: 33.

All the other not mentioned above listed embodiments also pertain to this aspect.

One aspect as reported herein is a multispecific antibody comprising (at least) three antigen binding VH/VL-pairs,
whereby the multispecific antibody comprises/consists of
   a) a first circular fusion polypeptide comprising in N- to C-terminal direction
      i) a first variable heavy chain domain (VH₁),
      ii) an antibody heavy chain constant domain 1 (CHI),
      iii) a first peptidic linker,
      iv) a second variable heavy chain domain (VH₂),
      v) a first CH3 domain (CH3₁),
      vi) a second peptidic linker,
      vii) a first variable light chain domain (VL₁), and
      viii) an antibody light chain constant domain (CL),
      wherein the domains identified under i) and vii) are associated with each other and are a first VH/VL-pair specifically binding to a first antigen,
      and
   b) a second circular fusion polypeptide comprising in N- to C-terminal direction
      i) a third variable heavy chain domain (VH₃),
      ii) an antibody heavy chain constant domain 1 (CHI),
      iii) a third peptidic linker,
      iv) a second variable light chain domain (VL₂),
      v) a second CH3 domain (CH3₂),
      vi) a fourth peptidic linker,
      vii) a third variable light chain domain (VL₃), and
      viii) an antibody light chain constant domain (CL),
      wherein the domains identified under i) and vii) are associated with each other and are a second VH/VL-pair specifically binding to a second antigen,
wherein the CH3 domains are altered to promote heterodimerization by generation of a protuberance in the first CH3 domain by substituting at least one original amino acid residue by an amino acid residue having a larger side chain volume than the original amino acid residue, and generation of a cavity in the second CH3 domain by substituting at least one original amino acid residue by an amino acid residue having a smaller side chain volume than the original amino acid residue, such that the protuberance generated in one of the CH3 domains is positionable in the cavity generated in the other one of the CH3 domains,
wherein the domains identified under a) v) and b) v) are conjugated to each other by a disulfide bonds (to form a dimer of the first circular fusion polypeptide and the second fusion polypeptide),
   and
wherein the domains identified under a) iv) and b) iv) are associated with
each other and are a third VH/VL-pair specifically binding to a third antigen. In one embodiment the first antigen and the second antigen are the same antigen and the third antigen is an antigen different therefrom.

In one embodiment of all aspects the first and the second binding site specifically bind to the same (first) antigen and the third binding site specifically binds to a different (second) antigen.

In one embodiment of all aspects the first binding site specifically binds to a first antigen, the second binding site specifically binds to a second antigen, and the third binding site specifically binds to a third antigen, whereby the first the second and the third antigen are different antigens.

### III. Binding sites

### III.1. Antibody fragment derived binding sites

In certain embodiments, the binding sites in the multispecific antibody as reported herein are each composed of an antibody heavy chain variable domain (VH) and an antibody light chain variable domain (VL).

In certain embodiments, one or two of the binding sites of the multispecific antibody is/are an antibody fragment. Antibody fragments include, but are not limited to, Fab, Fab', Fab'-SH, and Fv-fragments. For a review of certain antibody fragments, see Hudson, P.J. et al., Nat. Med. 9 (2003) 129-134. For a review of scFv fragments, see, e.g., Plueckthun, A., In; The Pharmacology of Monoclonal Antibodies, Vol. 113, Rosenburg and Moore (eds.), Springer-Verlag, New York (1994), pp. 269-315; see also WO 93/16185; US 5,571,894 and US 5,587,458. For discussion of Fab fragments comprising salvage receptor binding epitope residues and having increased in vivo half-life, see US 5,869,046.

The antibody fragment can be also a "Dual Acting Fab" or "DAF" (see, US 2008/0069820, for example).

Antibody fragments can be made by various techniques, including but not limited to proteolytic digestion of an intact antibody as well as production by recombinant host cells (e.g. E. coli or phage), as described herein.

If the binding site is a Fab then the Fab can be a conventional Fab, a DAF or a bispecific Fab.

In case of a conventional Fab one part of a binding domain comprises an antibody heavy chain variable domain (VH) and at least an N-terminal fragment of a (or a complete) first antibody heavy chain constant domain (CH1) and the respective other part of the binding domain comprises an antibody light chain variable domain (VL) and at least an N-terminal fragment of a (or a complete) antibody light chain constant domain (CL). The order of these domains may be any as long as association thereof and forming of a (functional) binding site is possible (i.e. not prevented).

In one embodiment one part of the binding domain comprises in N- to C-terminal direction VH-CH1 and the other part of the binding domain comprises in N- to C-terminal direction VL-CL.

In case of a bispecific Fab one part of the binding domain comprises an antibody heavy chain variable domain (VH) and at least an N-terminal fragment of a (or a complete) first antibody heavy chain constant domain (CH1) and the respective other binding domain comprises an antibody light chain variable domain (VL) and at least an N-terminal fragment of a (or a complete) antibody light chain constant domain (CL), wherein herein said binding domain comprises two non-overlapping paratopes in the complementary pair of a heavy chain variable domain (VH) and a light chain variable domain (VL), wherein the first paratope comprises residues from CDR1 and CDR3 of the VL domain and CDR2 of the VH domain, and the second paratope comprises residues from CDR1 and CDR3 of the VH domain and CDR2 of the VL domain.

In one embodiment the first paratope comprises residues from CDR1 and CDR3 of the VL domain and CDR2 of the VH domain, and the second paratope comprises residues from CDR1 and CDR3 of the VH domain and CDR2 of the VL domain.

In one embodiment the heavy chain variable domain of the binding site is based on a human VH3 family heavy chain sequence and the light chain variable domain of the binding site is based on a human Vkappa1 family light chain sequence.

In one embodiment the heavy chain variable domain of the binding site is based on a human VH3 family heavy chain sequence and the light chain variable domain of the binding site is based on a human Vlambdal family light chain sequence.

### III.2. Chimeric and humanized antibody derived binding sites

In certain embodiments, one or two or three of the binding sites in the multispecific antibody are chimeric domains derived from a chimeric antibody, e.g. a humanized antibody.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "hypervariable region" or "HVR", as used herein, refers to each of the regions of an antibody variable domain comprising the amino acid residue stretches which are hypervariable in sequence ("complementarity determining regions" or "CDRs") and/or form structurally defined loops ("hypervariable loops"), and/or contain the antigen-contacting residues ("antigen contacts"). Generally, antibodies comprise six HVRs; three in the VH (HI, H2, H3), and three in the VL (LI, L2, L3).

### HVRs include

(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (HI), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. In some embodiments, some FR residues in a humanized antibody are substituted with corresponding residues from a non-human antibody (e.g., the antibody from which the HVR residues are derived), e.g., to restore or improve antibody specificity or affinity.

A "humanized" antibody refers to an antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

Humanized antibodies and methods of making them are reviewed, e.g., in Almagro, J.C. and Fransson, J., Front. Biosci. 13 (2008) 1619-1633, and are further described, e.g., in Riechmann, I. et al., Nature 332 (1988) 323-329; Queen, C. et al., Proc. Natl. Acad. Sci. USA 86 (1989) 10029-10033; US 5, 821,337, US 7,527,791, US 6,982,321, and US 7,087,409; Kashmiri, S.V. et al., Methods 36 (2005) 25-34 (describing specificity determining region (SDR) grafting); Padlan, E.A., Mol. Immunol. 28 (1991) 489-498 (describing "resurfacing"); Dall'Acqua, W.F. et al., Methods 36 (2005) 43-60 (describing "FR shuffling"); and Osbourn, J. et al., Methods 36 (2005) 61-68 and Klimka, A. et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J. et al., J. Immunol. 151 (1993) 2296-2308; framework regions derived from the consensus sequence of human antibodies of a particular subgroup of light or heavy chain variable regions (see, e.g., Carter, P. et al., Proc. Natl. Acad. Sci. USA 89 (1992) 4285-4289; and Presta, L.G. et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M. et al., J. Biol. Chem. 272 (1997) 10678-10684 and Rosok, M.J. et al., J. Biol. Chem. 271 (19969 22611-22618).

### III.3. Human antibody derived binding sites

In certain embodiments, the one or two or three binding site in the multispecific antibody as reported herein is/are composed of an antibody heavy chain variable domain (VH) and an antibody light chain variable domain (VL). In certain embodiments, the variable domains are from a human antibody.

Human antibodies can be produced using various techniques known in the art. Human antibodies are described generally in van Dijk, M.A. and van de Winkel, J.G., Curr. Opin. Pharmacol. 5 (2001) 368-374 and in Lonberg, N., Curr. Opin. Immunol. 20 (2008) 450-459.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125. See also, e.g., US 6,075,181 and US 6,150,584 describing XENOMOUSE^{™} technology; US 5,770,429 describing HUMAB^{®} technology; US 7,041,870 describing K-M MOUSE^{®} technology; US 2007/0061900, describing VELOCIMOUSE^{®} technology; WO 2007/131676 describing an immunoreconstituted mouse). Human variable regions from intact antibodies generated by such animals may be further modified.

Human antibodies can also be made by hybridoma-based methods. Human myeloma and mouse-human heteromyeloma cell lines for the production of human monoclonal antibodies have been described (see, e.g., Kozbor, D., J. Immunol. 133 (1984) 3001-3005; Brodeur, B.R. et al., Monoclonal Antibody Production Techniques and Applications, Marcel Dekker, Inc., New York (1987), pp. 51-63; and Boerner, P. et al., J. Immunol. 147 (1991) 86-95). Human antibodies generated via human B-cell hybridoma technology are also described in Li, J. et al., Proc. Natl. Acad. Sci. USA 103 (2006) 3557-3562. Additional methods include those described, for example, in US 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191.

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### III.4. Library-derived antibody binding sites

In certain embodiments, one or two or three of the binding site in the multispecific antibody as reported herein is/are composed of an antibody heavy chain variable domain (VH) and an antibody light chain variable domain (VL). In certain embodiments, the variable domains are isolated by screening combinatorial libraries for antibodies with the desired activity or activities.

For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature 348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G. et al., Ann. Rev. Immunol. 12 (1994) 433-455. Phage typically display antibody fragments, either as single-chain Fv (scFv) fragments or as Fab fragments. Libraries from immunized sources provide high-affinity binding sites to the immunogen without the requirement of constructing hybridomas. Alternatively, the naive repertoire can be cloned (e.g., from human) to provide a single source of antibodies to a wide range of non-self- and also self-antigens without any immunization as described by Griffiths, A.D. et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement *in vitro,* as described by Hoogenboom, H.R. and Winter, G., J. Mol. Biol. 227 (1992) 381-388. Patent publications describing human antibody phage libraries include, for example: US 5,750,373, and US 2005/0079574, US 2005/0119455, US 2005/0266000, US 2007/0117126, US 2007/0160598, US 2007/0237764, US 2007/0292936, and US 2009/0002360.

Antibodies or antibody fragments isolated from human antibody libraries are considered human antibodies or human antibody fragments herein.

### IV. Hetero-multi(di)merization domains

For assuring the correct association of the two circular fusion polypeptides to form the multispecific antibody as reported herein different technologies can be used. One of them is the so called "knob-in-hole" engineering (see, e.g., US 5,731,168). Multicircular fusion polypeptides may also be made by engineering electrostatic steering effects for making Fc-heterodimeric molecules (WO 2009/089004); cross-linking two or more circular fusion polypeptides (see, e.g., US 4,676,980, and Brennan, M. et al., Science 229 (1985) 81-83); using leucine zippers to produce bicircular fusion polypeptides (see, e.g., Kostelny, S.A. et al., J. Immunol. 148 (1992) 1547-1553).

The "CH3 domain" comprises the stretch of residues C-terminal to a CH2 domain in an Fc region (i.e. from an amino acid residue at about position 341 to an amino acid residue at about position 447 of an IgG). Within an antibody according to the invention, one respective CH3 domain is arranged at the C-terminus of the VH₃ and VL₃ domain of the third binding site. The "CH3 domains" herein are variant CH3 domains, wherein the amino acid sequence of the natural CH3 domain was subjected to at least one distinct amino acid substitution (i.e. modification of the amino acid sequence of the CH3 domain) in order to promote dimerization of the two CH3 domains facing each other within the multispecific antibody.

Several approaches for CH3-modifications in order to support heterodimerization have been described, for example in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291.

Typically, in the heterodimerization approaches known in the art, the CH3 domain of one heavy chain and the CH3 domain of the other heavy chain are both engineered in a complementary manner so that the heavy chain comprising one engineered CH3 domain can no longer homodimerize with another heavy chain of the same structure. Thereby the heavy chain comprising one engineered CH3 domain is forced to heterodimerize with the other heavy chain comprising the CH3 domain, which is engineered in a complementary manner.

One heterodimerization approach known in the art is the so-called "knobs-into-holes" technology, which is described in detail providing several examples in e.g. WO 96/027011, Ridgway, J.B., et al., Protein Eng. 9 (1996) 617-621; Merchant, A.M., et al., Nat. Biotechnol. 16 (1998) 677-681; and WO 98/050431. In the "knobs-into-holes" technology, within the interface formed between two CH3 domains in the tertiary structure of the antibody, particular amino acids on each CH3 domain are engineered to produce a protuberance ("knob") in one of the CH3 domains and a cavity ("hole") in the other one of the CH3 domains, respectively. In the tertiary structure of the multispecific antibody the introduced protuberance in the one CH3 domain is positionable in the introduced cavity in the other CH3 domain.

In one embodiment of the multispecific antibody, the CH3 domains are altered according to the knobs-into-holes technology. The multispecific antibody according to this embodiment is herein also referred to as "CH3(KiH)-engineered multispecific antibody" (wherein the abbreviation "KiH" stands for the "knob-into-hole technology"). Hence, according to this embodiment within a CH3(KiH)-engineered multispecific antibody the CH3 domains are altered to promote heterodimerization by generation of a protuberance in one of the CH3 domains by substituting at least one original amino acid residue by an amino acid residue having a larger side chain volume than the original amino acid residue; and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains.

In other words, this embodiment relates to a CH3(KiH)-engineered multispecific antibody according to the invention comprising a first polypeptide comprising an antibody constant domain 3 and a second polypeptide comprising an antibody constant domain 3, wherein in the tertiary structure of the antibody the CH3 domain of the first polypeptide and the CH3 domain of the second polypeptide form an interface that is located between the respective CH3 domains, wherein the respective amino acid sequences of the CH3 domain of the first polypeptide and the CH3 domain of the second polypeptide each comprise a set of amino acids that is located within said interface in the tertiary structure of the antibody,
- wherein from the set of amino acids that is located in the interface in the CH3 domain of one polypeptide at least one amino acid residue is substituted by an amino acid residue having a larger side chain volume than the original amino acid residue, thereby generating a protuberance within the interface, wherein the protuberance is located in the CH3 domain of the one polypeptide, and wherein the protuberance is positionable in a cavity located in the CH3 domain of the other polypeptide within the interface; and
- wherein from the set of amino acids that is located in the interface in the CH3 domain of the other polypeptide at least one amino acid residue is substituted by an amino acid residue having a smaller side chain volume than the original amino acid residue, thereby generating a cavity within the interface, wherein the cavity is located in the CH3 domain of the other polypeptide, and wherein in the cavity the protuberance within the interface located in the CH3 domain of the one polypeptide is positionable.

In one embodiment of said CH3(KiH)-engineered multispecific antibody according to the invention said amino acid residue having a larger side chain volume than the original amino acid residue is selected from R, F, Y and W.

In one embodiment of said CH3(KiH)-engineered multispecific antibody according to the invention said amino acid residue having a smaller side chain volume than the original amino acid residue is selected from A, S, T and V.

In one embodiment of said CH3(KiH)-engineered multispecific antibody according to the invention said amino acid residue having a larger side chain volume than the original amino acid residue is selected from R, F, Y and W; and said amino acid residue having a smaller side chain volume than the original amino acid residue is selected from A, S, T and V.

In one embodiment of said CH3(KiH)-engineered multispecific antibody according to the invention, one of the CH3 domains comprises a T366W mutation, and the respective other CH3 domain comprises T366S, L368A and Y407V mutations (numberings according to EU index of Kabat).

In one embodiment of said CH3(KiH)-engineered multispecific antibody according to the invention, one of the CH3 domains comprises T366W and G407Y mutations, and the respective other CH3 domain comprises T366S, L368A and Y407V mutations (numberings according to EU index of Kabat).

In another embodiment of said CH3(KiH)-engineered multispecific antibody according to the invention, one of the CH3 domains comprises T366W, R409D and K370E mutations, and the respective other CH3 domain comprises T366S, L368A, Y407V, D399K and E357K mutations (numberings according to EU index of Kabat).

Alternatively to or in combination with the modifications according to the knobs-into-holes technology as defined above, the CH3 domains of the multispecific antibody according to the invention are altered to promote heterodimerization based on other heterodimerization approaches known in the art, preferably the ones described in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954 and WO 2013/096291.

In another embodiment of the multispecific antibody, alternatively to or in combination with the modifications according to the knobs-into-holes technology the CH3 domains are altered by the introduction of charged amino acids with opposite charges at specific amino acid positions in the CH3/CH3-domain-interface (e.g. as described in EP 1870459). The multispecific antibody according to this embodiment is herein also referred to as "CH3(+/-)-engineered multispecific antibody" (wherein the abbreviation "+/-" stands for the oppositely charged amino acids that were introduced in the respective CH3 domains). Hence, according to this embodiment within a CH3(+/-)-engineered multispecific antibody the CH3 domains are altered to promote heterodimerization by substituting at least one original amino acid residue in one of the CH3 domains by a positively charged amino acid; and substituting at least one original amino acid residue in the other one of the CH3 domains by a negatively charged amino acid.

In other words, this embodiment relates to a CH3(+/-)-engineered multispecific antibody according to the invention comprising a first polypeptide and a second polypeptide, wherein in the tertiary structure of the antibody the CH3 domain of the first polypeptide and the CH3 domain of the second polypeptide form an interface that is located between the respective antibody CH3 domains, wherein the respective amino acid sequences of the CH3 domain of the first polypeptide and the CH3 domain of the second polypeptide each comprise a set of amino acids that is located within said interface in the tertiary structure of the antibody, wherein from the set of amino acids that is located in the interface in the CH3 domain of one polypeptide a first amino acid is substituted by a positively charged amino acid and from the set of amino acids that is located in the interface in the CH3 domain of the other polypeptide a second amino acid is substituted by a negatively charged amino acid.

In one embodiment of said CH3(+/-)-engineered multispecific antibody according to the invention the positively charged amino acid is selected from K, R and H; and the negatively charged amino acid is selected from E or D.

In one embodiment of said CH3(+/-)-engineered multispecific antibody according to the invention the positively charged amino acid is selected from K and R; and the negatively charged amino acid is selected from E or D.

In one embodiment of said CH3(+/-)-engineered multispecific antibody according to the invention the positively charged amino acid is K; and the negatively charged amino acid is E.

In one embodiment of said CH3(+/-)-engineered multispecific antibody according to the invention in one of the CH3 domains the amino acid R at position 409 (numbering according to EU index of Kabat) is substituted by D and the amino acid K at position 370 (numbering according to EU index of Kabat) is substituted by E; and in the respective other CH3 domain the amino acid D at position 399 (numbering according to EU index of Kabat) is substituted by K and the amino acid E at position 357 (numbering according to EU index of Kabat) is substituted by K.

In another embodiment of the multispecific antibody, the CH3 domains are disulfide stabilized. The multispecific antibody according to this embodiment is herein also referred to as "CH3(S-S)-engineered multispecific antibody" (wherein the abbreviation "S-S" stands for the disulfide stabilization). Hence, according to this embodiment within a CH3(S-S)-engineered multispecific antibody the CH3 domains are altered to promote heterodimerization by introduction of at least one cysteine residue in each CH3 domain such that a disulfide bond is formed between the CH3 domains.

In other words, this embodiment relates to a CH3(S-S)-engineered multispecific antibody according to the invention comprising a first polypeptide and a second polypeptide, wherein in the tertiary structure of the antibody the CH3 domain of the first polypeptide and the CH3 domain of the second polypeptide form an interface that is located between the respective antibody CH3 domains, wherein the respective amino acid sequences of the CH3 domain of the first polypeptide and the CH3 domain of the second polypeptide each comprise a set of amino acids that is located within said interface in the tertiary structure of the antibody, from the set of amino acids that is located in the interface in the CH3 domain of the one polypeptide a first amino acid is substituted by cysteine; and from the set of amino acids that is located in the interface in the CH3 domain of the other polypeptide a second amino acid is substituted by cysteine, wherein the second amino acid is facing the first amino acid within the interface, such that a disulfide bridge between the CH3 domain of the one polypeptide and the CH3 domain of the other polypeptide can be formed via the introduced cysteine residues.

In one embodiment of the CH3(S-S)-engineered multispecific antibody the CH3 domains are disulfide stabilized by a E356C or a S354C mutation in one of the CH3 domains and a Y349C mutation in the other CH3 domain (numberings according to EU index of Kabat). In one embodiment of the CH3(S-S)-engineered multispecific antibody the CH3 domains are disulfide stabilized by a S354C mutation in one of the CH3 domains and a Y349C mutation in the other CH3 domain (numberings according to EU index of Kabat).

In yet another preferred embodiment of the multispecific antibody, the CH3 domains are disulfide stabilized and altered according to the knobs-into-holes technology. The multispecific antibody according to this embodiment is herein also referred to as "CH3(KSS)-engineered multispecific antibody" (wherein the abbreviation "K" stands for the knobs-into-holes technology and the "SS" stands for the disulfide stabilization). Hence, according to this embodiment, within a CH3(KSS)-engineered multispecific antibody the CH3 domains are altered to promote heterodimerization by generation of a protuberance in one of the CH3 domains by substituting at least one original amino acid residue by an amino acid residue having a larger side chain volume than the original amino acid residue; and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains; and additional introduction of at least one cysteine residue in each CH3 domain such that a disulfide bond is formed between the CH3 domains.

In other words, this embodiment relates to a CH3(KSS)-engineered multispecific antibody according to the invention comprising a first polypeptide and a second polypeptide, wherein in the tertiary structure of the antibody the CH3 domain of the first polypeptide and the CH3 domain of the second polypeptide form an interface that is located between the respective antibody CH3 domains, wherein the respective amino acid sequences of the CH3 domain of the first polypeptide and the CH3 domain of the second polypeptide each comprise a set of amino acids that is located within said interface in the tertiary structure of the antibody,
- wherein from the set of amino acids that is located in the interface in the CH3 domain of one polypeptide at least one amino acid residue is substituted by an amino acid residue having a larger side chain volume than the original amino acid residue, thereby generating a protuberance within the interface, wherein the protuberance is located in the CH3 domain of the one polypeptide, and wherein the protuberance is positionable in a cavity located in the CH3 domain of the other polypeptide within the interface; and
- wherein from the set of amino acids that is located in the interface in the CH3 domain of the other polypeptide at least one amino acid residue is substituted by an amino acid residue having a smaller side chain volume than the original amino acid residue, thereby generating a cavity within the interface, wherein the cavity is located in the CH3 domain of the other polypeptide, and wherein in the cavity the protuberance within the interface located in the CH3 domain of the one polypeptide is positionable; and wherein
- from the set of amino acids that is located in the interface in the CH3 domain of the one polypeptide a first amino acid is substituted by cysteine; and from the set of amino acids that is located in the interface in the CH3 domain of the other polypeptide a second amino acid is substituted by cysteine, wherein the second amino acid is facing the first amino acid within the interface; such that a disulfide bridge between the CH3 domain of the one polypeptide and the CH3 domain of the other polypeptide can be formed via the introduced cysteine residues.

In one embodiment of said CH3(KSS)-engineered multispecific antibody according to the invention the E356C or S354C mutation is introduced in the CH3 domain of the "knob" chain and the Y349C mutations are introduced in the CH3 domain of the "hole" chain.

In one embodiment of said CH3(KSS)-engineered multispecific antibody according to the invention said amino acid residue having a larger side chain volume than the original amino acid residue is selected from R, F, Y and W.

In one embodiment of said CH3(KSS)-engineered multispecific antibody according to the invention said amino acid residue having a smaller side chain volume than the original amino acid residue is selected from A, S, T and V.

In one embodiment of said CH3(KSS)-engineered multispecific antibody according to the invention said amino acid residue having a larger side chain volume than the original amino acid residue is selected from R, F, Y and W; and said amino acid residue having a smaller side chain volume than the original amino acid residue is selected from A, S, T and V.

In one embodiment of said CH3(KSS)-engineered multispecific antibody according to the invention said amino acid residue having a larger side chain volume than the original amino acid residue is selected from R, F, Y and W; and said amino acid residue having a smaller side chain volume than the original amino acid residue is selected from A, S, T and V, and the CH3 domains are disulfide stabilized by a E356C or a S354C mutation in one of the CH3 domains (in one embodiment a S354C mutation) and a Y349C mutation in the other CH3 domain (numberings according to EU index of Kabat).

In one embodiment of said CH3(KSS)-engineered multispecific antibody according to the invention, the CH3 domain of the one polypeptide (the polypeptide comprising the "knob") comprises a T366W mutation, and the CH3 domain of the other polypeptide (the polypeptide comprising the "hole") comprises T366S, L368A and Y407V mutations (numberings according to EU index of Kabat), and the CH3 domains are disulfide stabilized by a E356C or a S354C mutation in one of the CH3 domains (in one embodiment a S354C mutation) and a Y349C mutation in the other CH3 domain (numberings according to EU index of Kabat).

In one embodiment of said CH3(KSS)-engineered multispecific antibody according to the invention, the CH3 domain of the one polypeptide (the polypeptide comprising the "knob") comprises T366W and G407Y mutations, and the CH3 domain of the other polypeptide (the polypeptide comprising the "hole") comprises T366S, L368A and Y407V mutations (numberings according to EU index of Kabat), and the CH3 domains are disulfide stabilized by a E356C or a S354C mutation in one of the CH3 domains (in one embodiment a S354C mutation) and a Y349C mutation in the other CH3 domain (numberings according to EU index of Kabat).

In another embodiment of said CH3(KSS)-engineered multispecific antibody according to the invention, the CH3 domain of the one polypeptide (the polypeptide comprising the "knob") comprises T366W, R409D and K370E mutations, and the CH3 domain of the other polypeptide (the polypeptide comprising the "hole") comprises T366S, L368A, Y407V, D399K and E357K mutations (numberings according to EU index of Kabat), the CH3 domains are disulfide stabilized by a E356C or a S354C mutation in one of the CH3 domains (in one embodiment a S354C mutation) and a Y349C mutation in the other CH3 domain (numberings according to EU index of Kabat).

In yet another preferred embodiment of the multispecific antibody, the CH3 domains are disulfide stabilized and altered by the introduction of charged amino acids with opposite charges at specific amino acid positions in the CH3/CH3-domain-interface. The multispecific antibody according to this embodiment is herein also referred to as "CH3(+/-/SS)-engineered multispecific antibody" (wherein the abbreviation "+/-" stands for the amino acids of opposite charge and the "SS" stands for the disulfide stabilization). Hence, according to this embodiment, within a CH3((+/-/SS)-engineered multispecific antibody the CH3 domains are altered to promote heterodimerization by substituting at least one original amino acid residue in one of the CH3 domains by a positively charged amino acid; and substituting at least one original amino acid residue in the other one of the CH3 domains by a negatively charged amino acid; and additional introduction of at least one cysteine residue in each CH3 domain such that a disulfide bond is formed between the CH3 domains.

In other words, this embodiment relates to a CH3(+/-/SS)-engineered multispecific antibody according to the invention comprising a first polypeptide and a second polypeptide, wherein in the tertiary structure of the antibody the CH3 domain of the first polypeptide and the CH3 domain of the second polypeptide form an interface that is located between the respective antibody CH3 domains, wherein the respective amino acid sequences of the CH3 domain of the first polypeptide and the CH3 domain of the second polypeptide each comprise a set of amino acids that is located within said interface in the tertiary structure of the antibody,
- wherein from the set of amino acids that is located in the interface in the CH3 domain of one polypeptide a first amino acid is substituted by a positively charged amino acid; and
- wherein from the set of amino acids that is located in the interface in the CH3 domain of the other polypeptide a second amino acid is substituted by a negatively charged amino acid; and wherein
- from the set of amino acids that is located in the interface in the CH3 domain of the one polypeptide a first amino acid is substituted by cysteine; and from the set of amino acids that is located in the interface in the CH3 domain of the other polypeptide a second amino acid is substituted by cysteine, wherein the second amino acid is facing the first amino acid within the interface; such that a disulfide bridge between the CH3 domain of the one polypeptide and the CH3 domain of the other polypeptide can be formed via the introduced cysteine residues.

In one embodiment of the invention, the third binding site of the multispecific antibody is disulfide stabilized. Hence, the VH₃ and VL₃ domains are altered by introduction of at least one cysteine residue in the VH₃ domain and one cysteine residue in the VL₃ domain such that a disulfide bond is formed between the VH₃ and VL₃ domains. In one embodiment of the invention, the third binding site of the multispecific antibody is disulfide stabilized by introduction of cysteine residues at the following positions to form a disulfide bond between the VH₃ and VL₃ domains (numbering according to Kabat):
- VH₃ at position 44, and VL₃ at position 100;
- VH₃ at position 105, and VL₃ at position 43; or
- VH₃ at position 101, and VL₃ at position 100.

In one preferred embodiment the third binding site is disulfide stabilized by introduction of cysteine residues in the VH₃ domain at position 44, and in the VL₃ domain at position 100.

In one preferred embodiment of the invention, the third binding site of the multispecific antibody is disulfide stabilized and the CH3 domains are disulfide stabilized.

Without being bound to this theory, the at least two disulfide bonds formed by this modification in different domains of the altered polypeptides of the multispecific antibody according to the invention replace the wild-type IgG hinge disulfide interactions and thereby support heterodimerization while allowing antigen access to the third binding site.

In one embodiment of the invention, the first and/or second binding site of the multispecific antibody is also disulfide stabilized by introduction of cysteine residues independently of each other at the following positions to form a disulfide bond between the VH and VL domains (numbering according to Kabat):
- VH at position 44, and VL at position 100;
- VH at position 105, and VL at position 43; or
- VH at position 101, and VL at position 100.

In one embodiment, the third binding site of a CH3(S-S)-engineered multispecific antibody according to the invention is disulfide stabilized by introduction of cysteine residues at the following positions to form a disulfide bond between the VH₃ and VL₃ domains (numbering according to Kabat):
- VH₃ at position 44, and VL₃ at position 100;
- VH₃ at position 105, and VL₃ at position 43; or
- VH₃ at position 101, and VL₃ at position 100.

In one preferred embodiment of the invention, the third binding site of a CH3(S-S)-engineered multispecific antibody according to the invention is disulfide stabilized by introduction of cysteine residues in the VH₃ domain at position 44, and in the VL₃ domain at position 100.

In another preferred embodiment of the invention, the third binding site of a CH3(S-S)-engineered multispecific antibody according to the invention is disulfide stabilized by introduction of cysteine residues in the VH₃ domain at position 44, and in the VL₃ domain at position 100; and the CH3 domains are disulfide stabilized by a E356C or a S354C mutation in one of the CH3 domains and a Y349C mutation in the other CH3 domain (numberings according to EU index of Kabat).

In another preferred embodiment of the invention, the heterodimerization is supported by knobs-into-holes modifications within the CH3 domains and, in addition, the third binding site of the multispecific antibody and the CH3 domains are disulfide stabilized, respectively. In a multispecific antibody according to this embodiment, the heterodimerization of the knobs-into-holes modified heavy chains is further supported by an artificial interchain disulfide bond, which is ― in contrast to known knobs-into-holes approaches ― not located within the CH3 domain, but in a different domain (i.e. between the VH₃ and VL₃ domains). Within the antibody according to this embodiment heterodimerization of the third binding module (comprising the VH₃-CH3 and VL₃-CH3 polypeptides) is promoted by four distinct interactions: (i) the natural interaction between VH₃ and VL₃, (ii) the disulfide stabilization in the VH₃/VL₃ interface, (iii) the disulfide stabilization in the CH3/CH3 interface; and (iv) the knobs-into-holes modifications in the CH3/CH3 interface. By this, formation of heterodimers rather than homodimer formation is promoted and stability of the antibody is improved.

In one embodiment, the third binding site of a CH3(KSS)-engineered multispecific antibody according to the invention is disulfide stabilized by introduction of cysteine residues at the following positions to form a disulfide bond between the VH₃ and VL₃ domains (numbering according to Kabat):
- VH₃ at position 44, and VL₃ at position 100;
- VH₃ at position 105, and VL₃ at position 43; or
- VH₃ at position 101, and VL₃ at position 100.

In one preferred embodiment of the invention, the third binding site of a CH3(KSS)-engineered multispecific antibody according to the invention is disulfide stabilized by introduction of cysteine residues in the VH₃ domain at position 44, and in the VL₃ domain at position 100.

In another preferred embodiment of the invention, the third binding site of a CH3(KSS)-engineered multispecific antibody according to the invention is disulfide stabilized by introduction of cysteine residues in the VH₃ domain at position 44, and in the VL₃ domain at position 100; and the amino acid residue having a larger side chain volume than the original amino acid residue is selected from R, F, Y and W; and the amino acid residue having a smaller side chain volume than the original amino acid residue is selected from A, S, T and V, and the CH3 domains are disulfide stabilized by a E356C or a S354C mutation in one of the CH3 domains (in one embodiment a S354C mutation) and a Y349C mutation in the other CH3 domain (numberings according to EU index of Kabat).

In another preferred embodiment of the invention, the third binding site of a CH3(KSS)-engineered multispecific antibody according to the invention is disulfide stabilized by introduction of cysteine residues in the VH₃ domain at position 44, and in the VL₃ domain at position 100; and the CH3 domain of one polypeptide (the polypeptide comprising the "knob") comprises a T366W mutation, and the CH3 domain of the other polypeptide (the polypeptide comprising the "hole") comprises T366S, L368A and Y407V mutations (numberings according to EU index of Kabat), and the CH3 domains are disulfide stabilized by a E356C or a S354C mutation in one of the CH3 domains (in one embodiment a S354C mutation) and a Y349C mutation in the other CH3 domain (numberings according to EU index of Kabat).

In another preferred embodiment of the invention, the third binding site of a CH3(KSS)-engineered multispecific antibody according to the invention is disulfide stabilized by introduction of cysteine residues in the VH₃ domain at position 44, and in the VL₃ domain at position 100; and the CH3 domain of the one polypeptide (the polypeptide comprising the "knob") comprises T366W and G407Y mutations, and the CH3 domain of the other polypeptide (the polypeptide comprising the "hole") comprises T366S, L368A and Y407V mutations (numberings according to EU index of Kabat), and the CH3 domains are disulfide stabilized by a E356C or a S354C mutation in one of the CH3 domains (in one embodiment a S354C mutation) and a Y349C mutation in the other CH3 domain (numberings according to EU index of Kabat).

In another preferred embodiment of the invention, the third binding site of a CH3(KSS)-engineered multispecific antibody according to the invention is disulfide stabilized by introduction of cysteine residues in the VH₃ domain at position 44, and in the VL₃ domain at position 100; and the CH3 domain of one polypeptide (the polypeptide comprising the "knob") comprises T366W, R409D and K370E mutations, and the CH3 domain of the other polypeptide (the polypeptide comprising the "hole") comprises T366S, L368A, Y407V, D399K and E357K mutations (numberings according to EU index of Kabat), the CH3 domains are disulfide stabilized by a E356C or a S354C mutation in one of the CH3 domains (in one embodiment a S354C mutation) and a Y349C mutation in the other CH3 domain (numberings according to EU index of Kabat).

In another preferred embodiment of the invention, the heterodimerization is supported by the introduction of charged amino acids with opposite charges at specific amino acid positions in the CH3/CH3-domain-interface and, in addition, the third binding site of the multispecific antibody and the CH3 domains are disulfide stabilized, respectively. In a multispecific antibody according to this embodiment, the heterodimerization of the modified polypeptides is further supported by an artificial interchain disulfide bond, which is not located within the CH3 domain, but in a different domain (i.e. between the VH₃ and VL₃ domains). In one embodiment, the third binding site of a CH3(+/-/SS)-engineered multispecific antibody according to the invention is disulfide stabilized by introduction of cysteine residues at the following positions to form a disulfide bond between the VH₃ and VL₃ domains (numbering according to Kabat):
- VH₃ at position 44, and VL₃ at position 100;
- VH₃ at position 105, and VL₃ at position 43; or
- VH₃ at position 101, and VL₃ at position 100.

In one preferred embodiment of the invention, the third binding site of a CH3(+/-/SS)-engineered multispecific antibody according to the invention is disulfide stabilized by introduction of cysteine residues in the VH₃ domain at position 44, and in the VL₃ domain at position 100.

Further techniques for modifying the CH3 domains of the heavy chains of a multispecific antibody (apart from the "knobs-into-holes" technology) to enforce heterodimerization are known in the art. These technologies, especially the ones described in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954 and WO 2013/096291 are contemplated herein as alternatives to the "knob-into-hole technology" in combination with a multispecific antibody according to the invention. The multispecific antibody including one of these modification in order to support heterodimerization is further referred to herein as "CH3-engineered" multispecific antibody.

According to the approach described in EP 1870459 heterodimerization of CH3 domains is based on the introduction of charged amino acids with opposite charges at specific amino acid positions in the CH3/CH3-domain-interface between both, the first and the second heavy chain (herein further referred to as a "CH3(+/-)-engineered multispecific antibody").

In one embodiment of a multispecific antibody according to the invention the approach described in WO 2013/157953 is used to support heterodimerization of the first polypeptide and the second polypeptide of the multispecific antibody. In one embodiment of said CH3-engineered multispecific antibody according to the invention, in the CH3 domain of one polypeptide the amino acid T at position 366 (numbering according to EU index of Kabat) is substituted by K; and in the CH3 domain of the other polypeptide the amino acid L at position 351 (numbering according to EU index of Kabat) is substituted by D. In another embodiment of said CH3-engineered multispecific antibody according to the invention, in the CH3 domain of one polypeptide the amino acid T at position 366 (numbering according to EU index of Kabat) is substituted by K and the amino acid L at position 351 (numbering according to EU index of Kabat) is substituted by K; and in the CH3 domain of the other polypeptide the amino acid L at position 351 (numbering according to EU index of Kabat) is substituted by D.

In another embodiment of said CH3-engineered multispecific antibody according to the invention, in the CH3 domain of one polypeptide the amino acid T at position 366 (numbering according to EU index of Kabat) is substituted by K and the amino acid L at position 351 (numbering according to EU index of Kabat) is substituted by K; and in the CH3 domain of the other polypeptide the amino acid L at position 351 (numbering according to EU index of Kabat) is substituted by D. Additionally at least one of the following substitutions is comprised in the CH3 domain of the other polypeptide: the amino acid Y at position 349 (numbering according to EU index of Kabat) is substituted by E, the amino acid Y at position 349 (numbering according to EU index of Kabat) is substituted by D and the amino acid L at position 368 (numbering according to EU index of Kabat) is substituted by E. In one embodiment the amino acid L at position 368 (numbering according to EU index of Kabat) is substituted by E.

In one embodiment of a multispecific antibody according to the invention the approach described in WO 2012/058768 is used to support heterodimerization of the first polypeptide and the second polypeptide of the multispecific antibody. In one embodiment of said CH3-engineered multispecific antibody according to the invention, in the CH3 domain of one polypeptide the amino acid L at position 351 (numbering according to EU index of Kabat) is substituted by Y and the amino acid Y at position 407 (numbering according to EU index of Kabat) is substituted by A; and in the CH3 domain of the other polypeptide the amino acid T at position 366 (numbering according to EU index of Kabat) is substituted by A and the amino acid K at position 409 (numbering according to EU index of Kabat) is substituted by F. In another embodiment, in addition to the aforementioned substitutions, in the CH3 domain of the other polypeptide at least one of the amino acids at positions 411 (originally T), 399 (originally D), 400 (originally S), 405 (originally F), 390 (originally N) and 392 (originally K) is substituted. Preferred substitutions are:
- substituting the amino acid T at position 411 (numbering according to EU index of Kabat) by an amino acid selected from N, R, Q, K, D, E and W;
- substituting the amino acid D at position 399 (numbering according to EU index of Kabat) by an amino acid selected from R, W, Y, and K;
- substituting the amino acid S at position 400 (numbering according to EU index of Kabat) by an amino acid selected from E, D, R and K;
- substituting the amino acid F at position 405 (numbering according to EU index of Kabat) by an amino acid selected from I, M, T, S, V and W;
- substituting the amino acid N at position 390 (numbering according to EU index of Kabat) by an amino acid selected from R, K and D; and
- substituting the amino acid K at position 392 (numbering according to EU index of Kabat) by an amino acid selected from V, M, R, L, F and E.

In another embodiment of said CH3-engineered multispecific antibody according to the invention (engineered according to WO 2012/058768), in the CH3 domain of one polypeptide the amino acid L at position 351 (numbering according to EU index of Kabat) is substituted by Y and the amino acid Y at position 407 (numbering according to EU index of Kabat) is substituted by A; and in the CH3 domain of the other polypeptide the amino acid T at position 366 (numbering according to EU index of Kabat) is substituted by V and the amino acid K at position 409 (numbering according to EU index of Kabat) is substituted by F. In another embodiment of said CH3-engineered multispecific antibody according to the invention, in the CH3 domain of one polypeptide the amino acid Y at position 407 (numbering according to EU index of Kabat) is substituted by A; and in the CH3 domain of the other polypeptide the amino acid T at position 366 (numbering according to EU index of Kabat) is substituted by A and the amino acid K at position 409 (numbering according to EU index of Kabat) is substituted by F. In said last aforementioned embodiment, in the CH3 domain of said other polypeptide the amino acid K at position 392 (numbering according to EU index of Kabat) is substituted by E, the amino acid T at position 411 (numbering according to EU index of Kabat) is substituted by E, the amino acid D at position 399 (numbering according to EU index of Kabat) is substituted by R and the amino acid S at position 400 (numbering according to EU index of Kabat) is substituted by R.

In one embodiment of a multispecific antibody according to the invention the approach described in WO 2011/143545 is used to support heterodimerization of the first polypeptide and the second polypeptide of the multispecific antibody. In one embodiment of said CH3-engineered multispecific antibody according to the invention, amino acid modifications in the CH3 domains of both polypeptides are introduced at positions 368 and/or 409.

In one embodiment of a multispecific antibody according to the invention the approach described in WO 2011/090762 is used to support heterodimerization of the first polypeptide and the second polypeptide of the multispecific antibody. WO 2011/090762 relates to amino acid modifications according to the "knob-into-hole" technology. In one embodiment of said CH3(KiH)-engineered multispecific antibody according to the invention, in the CH3 domain of one polypeptide the amino acid T at position 366 (numbering according to EU index of Kabat) is substituted by W; and in the CH3 domain of the other polypeptide the amino acid Y at position 407 (numbering according to EU index of Kabat) is substituted by A. In another embodiment of said CH3(KiH)-engineered multispecific antibody according to the invention, in the CH3 domain of one polypeptide the amino acid T at position 366 (numbering according to EU index of Kabat) is substituted by Y; and in the CH3 domain of the other polypeptide the amino acid Y at position 407 (numbering according to EU index of Kabat) is substituted by T.

In one embodiment of a multispecific antibody according to the invention, which is of IgG2 isotype, the approach described in WO 2011/090762 is used to support heterodimerization of the first polypeptide and the second polypeptide of the multispecific antibody.

In one embodiment of a multispecific antibody according to the invention, the approach described in WO 2009/089004 is used to support heterodimerization of the first polypeptide and the second polypeptide of the multispecific antibody. In one embodiment of said CH3-engineered multispecific antibody according to the invention, in the CH3 domain of one polypeptide the amino acid K or N at position 392 (numbering according to EU index of Kabat) is substituted by a negatively charged amino acid (in one preferred embodiment by E or D, in one preferred embodiment by D); and in the CH3 domain of the other polypeptide the amino acid D at position 399 the amino acid E or D at position 356 or the amino acid E at position 357 (numberings according to EU index of Kabat) is substituted by a positively charged amino acid (in one preferred embodiment K or R, in one preferred embodiment by K, in one preferred embodiment the amino acids at positions 399 or 356 are substituted by K). In one further embodiment, in addition to the aforementioned substitutions, in the CH3 domain of the one polypeptide the amino acid K or R at position 409 (numbering according to EU index of Kabat) is substituted by a negatively charged amino acid (in one preferred embodiment by E or D, in one preferred embodiment by D). In one even further embodiment, in addition to or alternatively to the aforementioned substitutions, in the CH3 domain of the one polypeptide the amino acid K at position 439 and/or the amino acid K at position 370 (numbering according to EU index of Kabat) is substituted independently from each other by a negatively charged amino acid (in one preferred embodiment by E or D, in one preferred embodiment by D).

In one embodiment of a multispecific antibody according to the invention, the approach described in WO 2007/147901 is used to support heterodimerization of the first polypeptide and the second polypeptide of the multispecific antibody. In one embodiment of said CH3-engineered multispecific antibody according to the invention, in the CH3 domain of one polypeptide the amino acid K at position 253 (numbering according to EU index of Kabat) is substituted by E, the amino acid D at position 282 (numbering according to EU index of Kabat) is substituted by K and the amino acid K at position 322 (numbering according to EU index of Kabat) is substituted by D; and in the CH3 domain of the other polypeptide the amino acid D at position 239 (numbering according to EU index of Kabat) is substituted by K, the amino acid E at position 240 (numbering according to EU index of Kabat) is substituted by K and the amino acid K at position 292 (numbering according to EU index of Kabat) is substituted by D.

In one embodiment of a multispecific antibody according to the invention, the approach described in WO 2007/110205 is used to support heterodimerization of the first polypeptide and the second polypeptide of the multispecific antibody.

In addition or alternatively to engineering the CH3 domains by above identified heterodimerization strategies, the introduction of an additional interchain disulfide bridge stabilizes the heterodimers (Atwell, S., et al., J. Mol. Biol. 270 (1997) 26-35; Merchant, A.M., et al., Nature Biotech. 16 (1998) 677-681). This is also referred to herein as "disulfide stabilization of the CH3 domains".

### V. Recombinant Methods and Compositions

The multispecific antibody according to the current invention may be produced using recombinant methods and compositions, e.g., as described in US 4,816,567. In one embodiment, one or more isolated nucleic acids encoding the multispecific antibody described herein is/are provided. Such nucleic acid may encode an amino acid sequence comprising one circular fusion polypeptide and optionally also an amino acid sequence comprising a second circular fusion polypeptide (e.g., a first circular fusion polypeptide and a second circular fusion polypeptide of a dicircular fusion polypeptide). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with) in case of a multispecific antibody according to the current invention a first vector comprising a nucleic acid that encodes an amino acid sequence comprising the first circular fusion polypeptide and a second vector comprising a nucleic acid that encodes an amino acid sequence comprising the second circular fusion polypeptide or a single vector comprising these two nucleic acids. In one embodiment, the host cell is eukaryotic, e.g. a Chinese Hamster Ovary (CHO) cell or lymphoid cell (e.g., Y0, NS0, Sp2/0 cell). In one embodiment, a method of making a multispecific antibody according to the current invention is provided, wherein the method comprises culturing a host cell comprising a nucleic acid encoding the multispecific antibody, as provided above, under conditions suitable for expression of the multispecific antibody, and optionally recovering the multispecific antibody from the host cell (or host cell culture medium).

For recombinant production of a multispecific antibody according to the invention, nucleic acid encoding the circular fusion polypeptides, e.g., as described above, are isolated and inserted into one or more vectors for further cloning and/or expression in a host cell. Such nucleic acid may be readily produced using conventional procedures.

Suitable host cells for cloning or expression of multispecific antibody-encoding vectors include prokaryotic or eukaryotic cells described herein. For example, multispecific antibodies according to the invention may be produced in bacteria, in particular when glycosylation and Fc effector function are not needed. For expression of antibody fragments and polypeptides in bacteria, see, e.g., US 5,648,237, US 5,789,199, and US 5,840,523 (see also Charlton, K.A., In: Methods in Molecular Biology, Vol. 248, Lo, B.K.C. (ed.), Humana Press, Totowa, NJ (2003), pp. 245-254, describing expression of antibody fragments in *E. coli.).* After expression, multispecific antibody may be isolated from the bacterial cell paste in a soluble fraction and can be further purified.

In addition to prokaryotes, eukaryotic microbes such as filamentous fungi or yeast are suitable cloning or expression hosts for circular fusion polypeptide-encoding vectors, including fungi and yeast strains whose glycosylation pathways have been "humanized", resulting in the production of a multispecific antibody according to the invention with a partially or fully human glycosylation pattern (see Gerngross, T.U., Nat. Biotech. 22 (2004) 1409-1414; Li, H. et al., Nat. Biotech. 24 (2006) 210-215).

Suitable host cells for the expression of glycosylated multispecific antibodies according to the invention are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts (see, e.g., US 5,959,177, US 6,040,498, US 6,420,548, US 7,125,978, and US 6,417,429 (describing PLANTIBODIES^{™} technology for producing antibodies in transgenic plants)).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### VI. Assays

Multispecific antibody according to the invention may be identified, screened for, or characterized for their physical/chemical properties and/or biological activities by various assays known in the art for determining binding of a polypeptide to its target.

### VII. Immunoconjugates

The invention also provides immunoconjugates comprising a multispecific antibody according to the invention conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is a multispecific antibody according to the invention-drug conjugate in which a multispecific antibody according to the invention is conjugated to one or more drugs, including but not limited to a maytansinoid (see US 5,208,020, US 5,416,064 and EP 0 425 235 B1); an auristatin such as monomethyl auristatin drug moieties DE and DF (MMAE and MMAF) (see US 5,635,483, US 5,780,588, and US 7,498,298); a dolastatin; a calicheamicin or derivative thereof (see US 5,712,374, US 5,714,586, US 5,739,116, US 5,767,285, US 5,770,701, US 5,770,710, US 5,773,001, and US 5,877,296; Hinman, L.M. et al., Cancer Res. 53 (1993) 3336-3342; and Lode, H.N. et al., Cancer Res. 58 (1998) 2925-2928); an anthracycline such as daunomycin or doxorubicin (see Kratz, F. et al., Curr. Med. Chem. 13 (2006) 477-523; Jeffrey, S.C. et al., Bioorg. Med. Chem. Lett. 16 (2006) 358-362; Torgov, M.Y. et al., Bioconjug. Chem. 16 (2005) 717-721; Nagy, A. et al., Proc. Natl. Acad. Sci. USA 97 (2000) 829-834; Dubowchik, G.M. et al., Bioorg. & Med. Chem. Letters 12 (2002) 1529-1532; King, H.D. et al., J. Med. Chem. 45 (20029 4336-4343; and US 6,630,579); methotrexate; vindesine; a taxane such as docetaxel, paclitaxel, larotaxel, tesetaxel, and ortataxel; a trichothecene; and CC1065.

In another embodiment, an immunoconjugate comprises a multispecific antibody according to the invention conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAPS), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises a multispecific antibody according to the invention conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example TC^{99m} or I¹²³, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of a multispecific antibody according to the invention and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta, E.S. et al., Science 238 (1987) 1098-1104. Carbon-14-labeled 1-isothiocyanatobenzyl-3-methyldiethylene triamine pentaacetic acid (MX-DTPA) is an exemplary chelating agent for conjugation of radionucleotide to the circular fusion polypeptide (see WO 94/11026). The linker may be a "cleavable linker" facilitating release of a cytotoxic drug in the cell. For example, an acid-labile linker, peptidase-sensitive linker, photolabile linker, dimethyl linker or disulfide-containing linker (Chari, R.V. et al., Cancer Res. 52 (1992) 127-131; US 5,208,020) may be used.

The immunoconjugates herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### VIII. Methods and Compositions for Diagnostics and Detection

In certain embodiments, any of the multispecific antibody according to the invention is useful for detecting the presence of its target(s) in a biological sample. The term "detecting" as used herein encompasses quantitative or qualitative detection. In certain embodiments, a biological sample comprises a blood, serum, plasma, cell or tissue.

In one embodiment, a multispecific antibody according to the invention for use in a method of diagnosis or detection is provided. In a further aspect, a method of detecting the presence of the target(s) of the multispecific antibody according to the invention in a biological sample is provided. In certain embodiments, the method comprises contacting the biological sample with the multispecific antibody according to the invention under conditions permissive for binding of the multispecific antibody to its target, and detecting whether a complex is formed between the multispecific antibody and its target. Such method may be an *in vitro* or *in vivo* method. In one embodiment, a multispecific antibody according to the invention is used to select subjects eligible for therapy with said multispecific antibody.

In certain embodiments, labeled multispecific antibodies according to the invention are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (US 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### IX. Pharmaceutical Formulations

Pharmaceutical formulations of a multispecific antibody according to the invention are prepared by mixing such multispecific antibody having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX^{®}, Baxter International, Inc.). Certain exemplary sHASEGPs and methods of use, including rhuPH20, are described in US 2005/0260186 and US 2006/0104968. In one aspect, a sHASEGP is combined with one or more additional glycosaminoglycanases such as chondroitinases.

Exemplary lyophilized antibody formulations are described in US 6,267,958. Aqueous antibody formulations include those described in US 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the circular fusion polypeptide, which matrices are in the form of shaped articles, *e.g*. films, or microcapsules.

The formulations to be used for *in vivo* administration are generally sterile. Sterility may be readily accomplished, e.g., by filtration through sterile filtration membranes.

### X. Therapeutic Methods and Compositions

Any of the multispecific antibodies according to the invention may be used in therapeutic methods.

In one aspect, a multispecific antibody according to the invention for use as a medicament is provided. In further aspects, a multispecific antibody according to the invention for use in treating a disease is provided. In certain embodiments, a multispecific antibody according to the invention for use in a method of treatment is provided. In certain embodiments, the invention provides a multispecific antibody for use in a method of treating an individual having a disease comprising administering to the individual an effective amount of the multispecific antibody according to the invention. In one such embodiment, the method further comprises administering to the individual an effective amount of at least one additional therapeutic agent. An "individual" according to any of the above embodiments is preferably a human.

In a further aspect, the invention provides for the use of a multispecific antibody according to the invention in the manufacture or preparation of a medicament. In one embodiment, the medicament is for treatment of a disease. In a further embodiment, the medicament is for use in a method of treating a disease comprising administering to an individual having said disease an effective amount of the medicament. In one such embodiment, the method further comprises administering to the individual an effective amount of at least one additional therapeutic agent. An "individual" according to any of the above embodiments may be a human.

In a further aspect, the invention provides a method for treating a disease. In one embodiment, the method comprises administering to an individual having such disease an effective amount of a multispecific antibody according to the invention. In one such embodiment, the method further comprises administering to the individual an effective amount of at least one additional therapeutic agent. An "individual" according to any of the above embodiments may be a human.

In a further aspect, the invention provides pharmaceutical formulations comprising any of the multispecific antibodies according to the invention, e.g., for use in any of the above therapeutic methods. In one embodiment, a pharmaceutical formulation comprises any of the multispecific antibodies according to the invention and a pharmaceutically acceptable carrier. In another embodiment, a pharmaceutical formulation comprises any of the multispecific antibodies according to the invention and at least one additional therapeutic agent.

A multispecific antibody according to the invention can be used either alone or in combination with other agents in a therapy. For instance, a multispecific antibody according to the invention may be co-administered with at least one additional therapeutic agent.

Such combination therapies noted above encompass combined administration (where two or more therapeutic agents are included in the same or separate formulations), and separate administration, in which case, administration of the multispecific antibody according to the invention can occur prior to, simultaneously, and/or following, administration of the additional therapeutic agent or agents. In one embodiment, administration of the multispecific antibody and administration of an additional therapeutic agent occur within about one month, or within about one, two or three weeks, or within about one, two, three, four, five, or six days, of each other. Suited multispecific antibody according to the invention can also be used in combination with radiation therapy.

A multispecific antibody according to the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Multispecific antibodies according to the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The multispecific antibody need not be, but is optionally formulated with one or more agents currently used to prevent or treat the disorder in question. The effective amount of such other agents depends on the amount of multispecific antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a multispecific antibody according to the invention (when used alone or in combination with one or more other additional therapeutic agents) will depend on the type of disease to be treated, the type of multispecific antibody, the severity and course of the disease, whether the multispecific antibody is administered for preventive or therapeutic purposes, previous therapy, the patient's clinical history and response to the multispecific antibody, and the discretion of the attending physician. The multispecific antibody is suitably administered to the patient at one time or over a series of treatments. Depending on the type and severity of the disease, about 1 µg/kg to 15 mg/kg (e.g. 0.5 mg/kg - 10 mg/kg) of multispecific antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the multispecific antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or *e.g.* about six doses of the multispecific antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to a multispecific antibody according to the invention.

### XI. Articles of Manufacture

In another aspect of the invention, an article of manufacture containing materials useful for the treatment, prevention and/or diagnosis of a disorder is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a multispecific antibody according to the invention. The label or package insert indicates that the composition is used for treating the condition of choice.

Moreover, the article of manufacture may comprise (a) a first container with a composition contained therein, wherein the composition comprises a multispecific antibody according to the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The following examples and figures are provided to aid the understanding of the present invention, the true scope of which is set forth in the appended claims.

### Description of the Figures

- **Figure 1**: Scheme of the general structure of the circular fusion polypeptide as reported herein.
- **Figure 2**: Schemes of the general structure of dicircular fusion polypeptides as reported herein.
- **Figure 3**: Orientation and spatial distance between the binding sites of a normal antibody of the IgG type and an exemplary dicircular fusion polypeptides as reported herein.
- **Figure 4**: UV extinction chromatogram (280 nm) of the different Contorsbody forms.
- **Figure 5**: Product quality analysis of the circular fusion polypeptide as reported herein by mass spectrometry.
- **Figures 6+7**: Scheme of the relative positions and orientations of the VH domain in the "VH-in" orientation (close to the Fc-region) and the "VH-out" orientation (more apart from the Fc-region).
- **Figure 8**: Exemplary chains of anti-cMET circular fusion polypeptides with the respective orientation used for the production of bispecific bicircular fusion polypeptides as reported herein.
- **Figure 9**: The multispecific antibody according to the current invention comprising two circular fusion polypeptides and a third VH/VL-pair; upper Figure: sketch, lower Figure: three-dimensional model.
- **Figure 10**: SEC chromatogram (upper part) and SDS-page (lower part) of the KappaSelect purified cultivation supernatant of the LeY/biotin TriFab Contorsbody.
- **Figure 11**: m/z spectra of TriFab-Contorsbody LeY/biotin.
- **Figure 12**: FACS analysis of LeY/biotin-TriFab-Contorsbody.
- **Figure 13**: Scheme of bispecific anti-LeY/CD3 TriFab Contorsbody.
- **Figure 14**: Coomassie-stained SDS PAGE gel of anti-LeY/CD3 TriFab Contorsbody; kDa = kilodalton (molecular weight marker); nr = non-reduced; r = reduced.
- **Figure 15**: Scheme of bispecific anti-LeY/CD3 TriFab Contorsbody and bispecific TriFabs used for the incubation with MCF7 cells.
- **Figure 16**: Dose-dependent cell killing of MCF7 cells of an anti-LeY TriFab (positive control), ii) anti-LeY/X TriFab (X being not present on MCF7; negative control), iii) anti-LeY/CD3 TriFab Contorsbody according to the current invention.

### XII. EXAMPLES

The following are examples of methods and compositions of the invention. It is understood that various other embodiments may be practiced, given the general description provided above.

### Materials and Methods

### Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions.

### Gene and oligonucleotide synthesis

Desired gene segments were prepared by chemical synthesis at Geneart GmbH (Regensburg, Germany). The synthesized gene fragments were cloned into an E. coli plasmid for propagation/amplification. The DNA sequences of subcloned gene fragments were verified by DNA sequencing. Alternatively, short synthetic DNA fragments were assembled by annealing chemically synthesized oligonucleotides or via PCR. The respective oligonucleotides were prepared by metabion GmbH (Planegg-Martinsried, Germany).

### Reagents

All commercial chemicals, antibodies and kits were used as provided according to the manufacturer's protocol if not stated otherwise.

### Example 1

### Construction of the expression plasmids for the circular fusion polypeptides

For the expression of a circular fusion polypeptide as reported herein a transcription unit comprising the following functional elements was used:
- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- a nucleic acid encoding the respective circular fusion polypeptide, and
- the bovine growth hormone polyadenylation sequence (BGH pA).

Beside the expression unit/cassette including the desired gene to be expressed the basic/standard mammalian expression plasmid contains
- an origin of replication from the vector pUC18 which allows replication of this plasmid in E. coli, and
- a beta-lactamase gene which confers ampicillin resistance in E. coli.

### Example 2

### Expression of the circular fusion polypeptides

Transient expression of circular fusion polypeptides was performed in suspension-adapted HEK293F (FreeStyle 293-F cells; Invitrogen) cells with Transfection Reagent 293-free (Novagen).

Cells have been passaged, by dilution, at least four times (volume 30 ml) after thawing in a 125 ml shake flask (Incubate/Shake at 37 °C, 7 % CO₂, 85 % humidity, 135 rpm).

The cells were expanded to 3×10⁵ cells/ml in 250 ml volume. Three days later, cells have been split and new seeded with a density of 7^{∗}10⁵ cells/ml in a 250 ml volume in a 1-liter shake flask. Transfection will be 24 hours later at a cell density around 1.4 - 2.0 × 10⁶ cells/ml.

Before transfection 250 µg plasmid-DNA were diluted in a final volume of 10 ml with pre-heated (water bath; 37 °C) Opti-MEM (Gibco). The solution was gently mixed and incubated at room temperature for not longer than 5 min. Then 333.3 µl 293-free transfection reagent were added to the DNA-OptiMEM-solution. Thereafter the solution was gently mixed and incubated at room temperature for 15-20 minutes. The whole volume of mixture was added to 1 L shake flask with 250 ml HEK-cell-culture-volume.

Incubate/Shake at 37 °C, 7 % CO₂, 85 % humidity, 135 rpm for 6 or 7 days.

The supernatant was harvested by a first centrifugation-step at 2,000 rpm, 4 °C, for 10 minutes. Then the supernatant was transferred into a new centrifugation-flask for a second centrifuge at 4,000 rpm, 4 °C, for 20 minutes. Thereafter the cell-free-supernatant was filtered through a 0.22 µm bottle-top-filter and stored in a freezer (-20 °C).

### Example 3

### Purification of the circular fusion polypeptides

The antibody-containing culture supernatants were filtered and purified by two chromatographic steps. The antibodies were captured by affinity chromatography using HiTrap MabSelectSuRe (GE Healthcare) equilibrated with PBS (1 mM KH₂PO₄, 10 mM Na₂HPO₄, 137 mM NaCl, 2.7 mM KCl), pH 7.4. Unbound proteins were removed by washing with equilibration buffer, and the antibody was recovered with 50 mM citrate buffer, pH 2.8, and immediately after elution neutralized to pH 6.0 with 1 M Tris-base, pH 9.0. Size exclusion chromatography on Superdex 200^{™} (GE Healthcare) was used as second purification step. The size exclusion chromatography was performed in 20 mM histidine buffer, 0.14 M NaCl, pH 6.0. The antibody containing solutions were concentrated with an Ultrafree-CL centrifugal filter unit equipped with a Biomax-SK membrane (Millipore, Billerica, MA) and stored at -80 °C.

### Example 4

### Binding of the anti-Her2 circular fusion polypeptide

### Surface plasmon resonance Her2 receptor binding

| | |
|---|---|
| **Chip Surface:** | CM5-Chip |
| **T:** | 37 °C and 25 °C, respectively for assay setting 1 and 2 |
| **running buffer:** | PBS + 0.05% (v/v) Tween 20 |
| **dilution buffer:** | running buffer + 0.1 % BSA |
| **analytes:** | c(HER2 ECD) = 0.41-900 nM for assay setting 1; c(dimeric anti-Her2 Contorsbodies) = 3.7-300 nM, c(trastuzumab) = 3.7-300 nM for assay setting 2 |
| **Ligand:** | dimeric anti-Her2 Contorsbody, trastuzumab bound via antihuman Fc-region antibody for assay setting 1; Her2-ECD bound via pertuzumab for assay setting 2. |

The response units are directly proportional to molecular weight. The theoretical maximum of analyte binding is based on the known binding level of Her2 ECD. 100 % = 1 molecule dimeric anti-Her2 Contorsbodies or trastuzumab binds to 2 molecules HER2 ECD, respectively.

### Example 5

### ADCC assay-ACEA

BT-474 cells were "solubilized" with Accutase, counted in the medium and brought to a cell density of 2x10E5 cells/ml. 50 µl medium were pipetted in each well on a 96-wells plates, the background effect was measured on the ACEA, and, finally, 50 µl cells suspension/well (=10,000 cells/well) were added. The plates were placed in the ACEA to measure the cell index after 15 minutes. The medium was then removed by pipetting, a washing step was made with AIM-V medium, and 50 µl antibody in three different concentrations was added. Natural killer cells were counted and placed in AIM-V to a cell density of 6×10E5. 50 µl (30,000 cells/well ad E/T 3:1 were added in ACEA and the cell index was measured every 5 minutes. After 24 hours, the experiment was stopped and ADCC after 2 and 4 hours was calculated.

### Example 6

### Mass spectrometric analysis

PNGase F was obtained from Roche Diagnostics GmbH (14.3 U / µl; solution in sodium phosphate, EDTA and glycerol). A protease specifically cleaving in the hinge region of an IgG antibody was freshly reconstituted from a lyophilisate prior to digestion.

### Enzymatic deglycosylation of with PNGase F

50 µg Contorsbody was diluted to a final concentration of 0.6 mg/ml with 10 mM sodium phosphate buffer, pH 7.1, and deglycosylated with 1 µl PNGase F at 37°C for 16 hours.

### Enzymatic cleavage

The deglycosylated sample was diluted to a final concentration of 0.5 mg / ml with 200 mM Tris buffer, pH 8.0, and subsequently digested with the IgG specific protease at 37°C for 1 hour.

### ESI-QTOF mass spectrometry

The sample was desalted by HPLC on a Sephadex G25 column (Kronlab, 5x250mm, TAC05/250G0-SR) using 40% acetonitrile with 2% formic acid (v/v). The total mass was determined via ESI-QTOF MS on a maXis 4G UHR-QTOF MS system (Bruker Daltonik) equipped with a TriVersa NanoMate source (Advion). Calibration was performed with sodium iodide (Waters ToF G2-Sample Kit 2 Part: 700008892-1). For the digested Contorsbody, data acquisition was done at 1000-4000 m/z (ISCID: 30 eV). The raw mass spectra were evaluated and transformed into individual relative molar masses. For visualization of the results, a proprietary software was used to generate deconvoluted mass spectra.

### Example 7

### Purification of the TriFab-Contorsbody

The antibody-containing culture supernatants were filtered and purified by two chromatographic steps. Due to absence of a functional Fc-region as the molecules lack CH2 domains, TriFab-Contorsbodies were purified by standard protein L (KappaSelect) affinity chromatography. The antibodies were captured by affinity chromatography using KappaSelect (GE Healthcare) equilibrated with PBS (1 mM KH₂PO₄, 10 mM Na₂HPO₄, 137 mM NaCl, 2.7 mM KCl), pH 7.4. Unbound proteins were removed by washing with equilibration buffer, and the antibody was recovered with 50 mM citrate buffer, pH 2.8, and immediately after elution neutralized to pH 6.0 with 1 M Tris-base, pH 9.0. Size exclusion chromatography on Superdex 200^{™} (GE Healthcare) was used as second purification step. The size exclusion chromatography was performed in 20 mM histidine buffer, 0.14 M NaCl, pH 6.0. The antibody containing solutions were concentrated with an Ultrafree-CL centrifugal filter unit equipped with a Biomax-SK membrane (Millipore, Billerica, MA) and stored at -80 °C.

### Example 8

### FACS analysis of TriFab-Contorsbody

FACS analyses were applied to assess the binding functionality of the generated TriFab-Contorsbody. Therefore, LeY-antigen expressing MCF7 cells were exposed to either to the biotin-Cy5 conjugate (neg. control), a non-binding TriFab-Contorsbody as negative controls followed after washing of the cells by biotin-Cy5 conjugate (neg. control II), or to the TriFab-Contorsbody with biotin-Cy5 subsequently being added and fluorescence of the cells was assessed. Figure 12 shows the results.

### Example 9

### TriFab Contorsbody efficiently mediates T cell-induced tumor cell killing

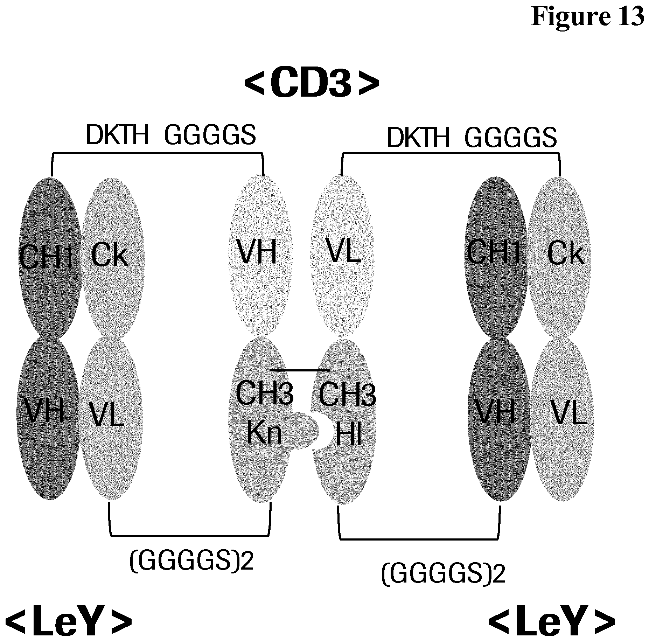

To assess the suitability of the TriFab Contorsbody format in T-cell-induced tumor cell killing, as third binding entity an anti-CD3 binding entity was used (Figure 13). The VH and VL sequences are exemplary sequences of the CD3-binder as described in US 2015/0166661 A1. Any anti-CD3 Fv can be substituted herein. This has simply been chosen as an example. This anti-LeY/CD3 TriFab Contorsbody comprises the polypeptides of SEQ ID NO: 36 and SEQ ID NO: 37.

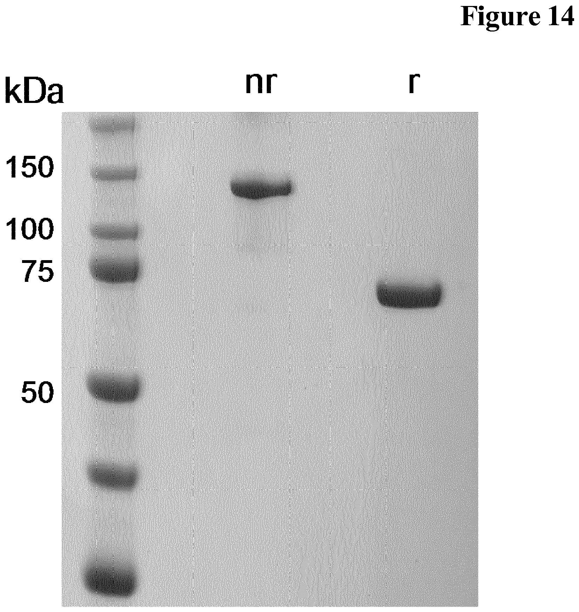

Expression and purification (performed as outlined in the Examples above) of the anti-LeY/CD3 TriFab Contorsbody was achieved in a yield of 8 mg/ L expression volume. In Coomassie-stained SDS PAGE analysis clear bands are present at expected weight (Figure 14).

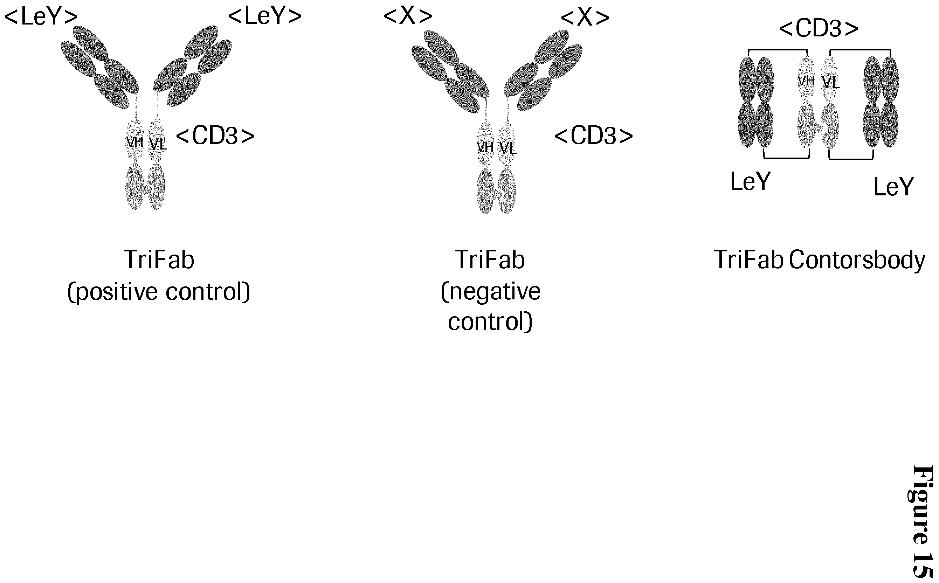

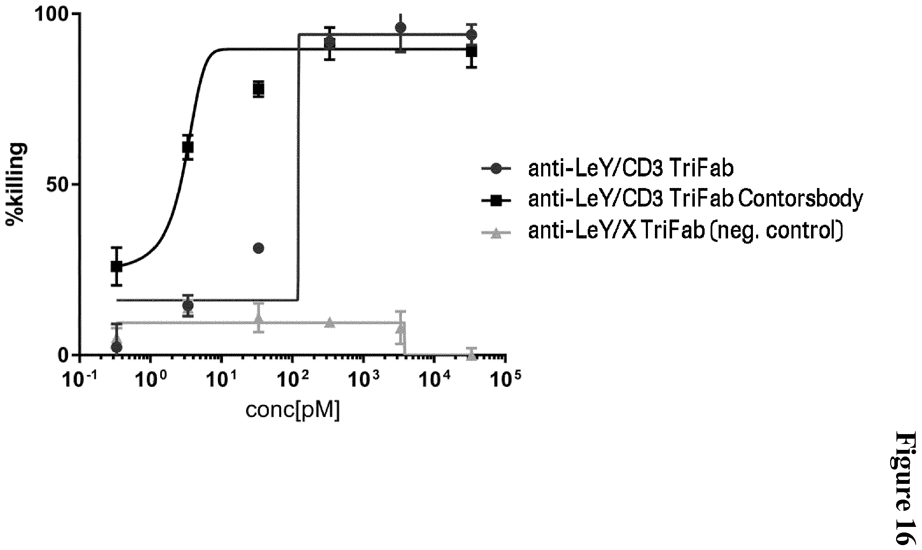

LeY positive MCF7 cells were seeded out in 96 well plates and incubated overnight, followed by exposure to different concentrations of i) an anti-LeY TriFab as positive control, ii) an anti-LeY/X TriFab with X being not present on MCF7 cells as negative control, and iii) the anti-LeY/CD3 TriFab Contorsbody (see Figure 15). To assess T cell-mediated killing, PBMCs from whole blood of healthy donors (isolated via Ficoll^{®} Paque Plus purification according to manufacturer's instructions (GE Healthcare) were added in a 5:1 ratio. Cultures were thereafter maintained at 37°C and 5% CO₂ for 48 hours, followed by assessment of the degree of tumor cell lysis (applying LDH release assays according to manufacturer's instructions (Cytotoxicity Detection Kit (LDH), Roche). It was confirmed that the TriFab Contorsbody induces dose-dependent killing even at low picomolar range. Compared to the positive control anti-LeY/CD3 TriFab (~120 pM) the TriFab Contorsbody showed significantly lower IC₅₀ value (~2.4 pM) (Figure 16).

Expression yields of different LeY/CD3 TriFab Contorsbodies:

| chain 1 | chain 2 | yield |
|---|---|---|
| | | less than 1 mg/L |
| | | less than 1 mg/L |
| | | less than 1 mg/L |
| | | about 8 mg/L |

## Claims

1. A multispecific antibody comprising three antigen binding sites and consisting of two circular fusion polypeptides, wherein
a) the first circular fusion polypeptide comprises a first part of a first binding domain, a second part of a first binding domain, and a first part of a third binding domain, wherein
- the first part of the first binding domain is fused either directly or via a first peptidic linker to the N-terminus of the first part of the third binding domain,
- the second part of the first binding domain is fused either directly or via a second peptidic linker to the C-terminus of the first part of the third binding domain,
- the first part of the first binding domain is a heavy chain Fab fragment (VH1-CH1) or a light chain Fab fragment (VL1-CL1), whereby the second part of the first binding domain is a light chain Fab fragment if the first part of the first binding domain is a heavy chain Fab fragment, or vice versa, and
- the first part of the first binding domain and the second part of the first binding domain are associated with each other and are together the first antigen binding site,
b) the second circular fusion polypeptide comprises a first part of a second binding domain, a second part of a second binding domain, and the second part of the third binding domain, wherein
- the first part of the second binding domain is fused either directly or via a third peptidic linker to the N-terminus of the second part of the third binding domain,
- the second part of the second binding domain is fused either directly or via a fourth peptidic linker to the C-terminus of the second part of the third binding domain,
- the first part of the second binding domain is a heavy chain Fab fragment (VH2-CH1) or a light chain Fab fragment (VL2-CL1), whereby the second part of the second binding domain is a light chain Fab fragment if the first part of the second binding domain is a heavy chain Fab fragment, or vice versa,
- the first part of the second binding domain and the second part of the second binding domain are associated with each other and are together the second antigen binding site,
c) the first part of the third binding domain and the second part of the third binding domain are together the third antigen binding site, wherein
- the first part of the third binding domain is either a variable heavy chain-CH3 domain fusion polypeptide (VH3-CH3) or a variable light chain-CH3 domain fusion polypeptide (VL3-CH3),
- the second part of the third binding domain is a variable heavy chain-CH3 domain fusion polypeptide if the first part of the second binding domain is a variable light chain-CH3 domain fusion polypeptide, or vice versa,
- the first part of the third binding domain and the second part of the third binding domain are associated with each other and form the third antigen binding site,
wherein the two constant heavy chain domains 3 (CH3) are altered to promote heterodimerization by
i) generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains, or
substituting at least one original amino acid residue in one of the CH3 domains by a positively charged amino acid, and substituting at least one original amino acid residue in the other one of the CH3 domains by a negatively charged amino acid, or
ii) introduction of at least one cysteine residue in each CH3 domain such that a disulfide bond is formed between the CH3 domains, or
iv) both modifications of i) and ii),
wherein the multispecific antibody is devoid of constant heavy chain domains 2 (CH2) and of a hinge region.

2. The multispecific antibody according to claim 1, wherein the first and/or the second and/or the third antigen binding site is independently of each other disulfide stabilized by introduction of cysteine residues at the following positions to form a disulfide bond between the VH and VL domains (numbering according to Kabat):
- VH at position 44, and VL at position 100,
- VH at position 105, and VL at position 43, or
- VH at position 101, and VL at position 100.

3. The multispecific antibody according to any one claims 1 to 2, wherein the first, second, third and fourth peptidic linker are peptides of at least 5 amino acids.

4. The multispecific antibody according to any one of claims 1 to 3, wherein the first and third peptidic linker have the amino acid sequence of SEQ ID NO: 38; and the second and fourth peptidic linker have the amino acid sequence of SEQ ID NO: 16.

5. The multispecific antibody according to any one of claims 1 to 4, wherein the C-terminus of the VH3 domain is directly connected to the N-terminus of one of the CH3 domains, and the C-terminus of the VL3 domain is directly connected to the N-terminus of the other one of the CH3 domains.

6. The multispecific antibody according to any one of claims 1 to 5, wherein the antibody is trivalent.

7. The multispecific antibody according to any one of claims 1 to 6, wherein the antibody is bispecific (the first binding site binds to a first antigen, the second binding site binds to a second antigen and the third binding site binds to a third antigen, whereby two of the first, the second and the third antigen are the same antigen and the other is a different antigen); or trispecific (the first binding site binds to a first antigen, the second binding site binds to a second antigen and the third binding site binds to a third antigen, whereby the first, the second and the third antigen are different antigens).

8. The multispecific antibody according to any one of claims 1 to 7, wherein the first part of the first binding domain and the second part of the first binding domain are associated covalently by a disulfide bond with each other, and/or wherein the first part of the second binding domain and the second part of the second binding domain are associated covalently by a disulfide bond with each other.

9. The multispecific antibody according to any one of claims 1 to 8, wherein the third binding site specifically binds to human CD3.

10. A method for the preparation of the multispecific antibody according to any one of claims 1 to 9, comprising the steps of
- transforming a host cell with expression vectors comprising nucleic acids encoding the multispecific antibody,
- culturing said host cell under conditions that allow synthesis of said multispecific antibody, and
- recovering said multispecific antibody from said host cell culture.

11. A multispecific antibody produced by the method according to claim 10.

12. A set of nucleic acid encoding the polypeptides of the multispecific antibody according to any one of claims 1 to 9.

13. An expression vector comprising a nucleic acid according to claim 12 or a set of expression vectors each comprising one of the nucleic acids according to claim 12.

14. A host cell comprising the nucleic acids according to claim 12.

15. A pharmaceutical composition comprising the multispecific antibody according to any one of claims 1 to 9, optionally in combination with at least one pharmaceutically acceptable carrier.

## Patentansprüche

1. Multispezifischer Antikörper, der drei Antigenbindungsstellen umfasst und aus zwei ringförmigen Fusionspolypeptiden besteht, wobei
a) das erste ringförmige Fusionspolypeptid einen ersten Teil einer ersten Bindungsdomäne, einen zweiten Teil einer ersten Bindungsdomäne und einen ersten Teil einer dritten Bindungsdomäne umfasst, wobei
- der erste Teil der ersten Bindungsdomäne entweder direkt oder über einen ersten Peptidlinker an den N-Terminus des ersten Teils der dritten Bindungsdomäne fusioniert ist,
- der zweite Teil der ersten Bindungsdomäne entweder direkt oder über einen zweiten Peptidlinker an den C-Terminus des ersten Teils der dritten Bindungsdomäne fusioniert ist,
- der erste Teil der ersten Bindungsdomäne ein Fab-Fragment der schweren Kette (VH1-CH1) oder ein Fab-Fragment der leichten Kette (VL1-CL1) ist, wobei der zweite Teil der ersten Bindungsdomäne ein Fab-Fragment der leichten Kette ist, wenn der erste Teil der ersten Bindungsdomäne ein Fab-Fragment der schweren Kette ist, oder umgekehrt und
- der erste Teil der ersten Bindungsdomäne und der zweite Teil der ersten Bindungsdomäne miteinander verbunden sind und zusammen die erste Antigenbindungsstelle sind,
b) das zweite ringförmige Fusionspolypeptid einen ersten Teil einer zweiten Bindungsdomäne, einen zweiten Teil einer zweiten Bindungsdomäne und den zweiten Teil der dritten Bindungsdomäne umfasst, wobei
- der erste Teil der zweiten Bindungsdomäne entweder direkt oder über einen dritten Peptidlinker an den N-Terminus des zweiten Teils der dritten Bindungsdomäne fusioniert ist,
- der zweite Teil der zweiten Bindungsdomäne entweder direkt oder über einen vierten Peptidlinker an den C-Terminus des zweiten Teils der dritten Bindungsdomäne fusioniert ist,
- der erste Teil der zweiten Bindungsdomäne ein Fab-Fragment der schweren Kette (VH2-CH1) oder ein Fab-Fragment der leichten Kette (VL2-CL1) ist, wobei der zweite Teil der zweiten Bindungsdomäne ein Fab-Fragment der leichten Kette ist, wenn der erste Teil der zweiten Bindungsdomäne ein Fab-Fragment der schweren Kette ist, oder umgekehrt,
- der erste Teil der zweiten Bindungsdomäne und der zweite Teil der zweiten Bindungsdomäne miteinander verbunden sind und zusammen die zweite Antigenbindungsstelle sind,
c) der erste Teil der dritten Bindungsdomäne und der zweite Teil der dritten Bindungsdomäne zusammen die dritte Antigenbindungsstelle sind, wobei
- der erste Teil der dritten Bindungsdomäne entweder ein Fusionspolypeptid der variablen Domäne der schweren Kette und der CH3-Domäne (VH3-CH3) oder ein Fusionspolypeptid der variablen Domäne der leichten Kette und der CH3-Domäne (VL3-CH3) ist,
- der zweite Teil der dritten Bindungsdomäne ein Fusionspolypeptid der variablen Domäne der schweren Kette und der CH3-Domäne ist, wenn der erste Teil der zweiten Bindungsdomäne ein Fusionspolypeptid der variablen Domäne der leichten Kette und der CH3-Domäne ist, oder umgekehrt,
- der erste Teil der dritten Bindungsdomäne und der zweite Teil der dritten Bindungsdomäne miteinander verbunden sind und die dritte Antigenbindungsstelle bilden,
wobei die zwei konstanten Domänen der schweren Kette 3 (CH3) so verändert sind, dass sie eine Heterodimerisation durch Folgendes unterstützen
i) Erzeugung einer Protuberanz in einer der CH3-Domänen durch Ersetzen mindestens eines ursprünglichen Aminosäurerestes durch einen Aminosäurerest mit einem größeren Seitenkettenvolumen als der ursprüngliche Aminosäurerest und Erzeugung einer Vertiefung in der anderen der CH3-Domänen durch Ersetzen mindestens eines ursprünglichen Aminosäurerestes durch einen Aminosäurerest mit einem kleineren Seitenkettenvolumen als der ursprüngliche Aminosäurerest, so dass die in einer der CH3-Domänen erzeugte Protuberanz in der in der anderen der CH3-Domänen erzeugten Vertiefung positioniert werden kann, oder
Ersetzen mindestens eines ursprünglichen Aminosäurerestes in einer der CH3-Domänen durch eine positiv geladene Aminosäure und Ersetzen mindestens eines ursprünglichen Aminosäurerestes in der anderen der CH3-Domänen durch eine negativ geladene Aminosäure, oder
ii) Einbringung mindestens eines Cystein-Restes in jede CH3-Domäne, so dass eine Disulfidbindung zwischen den CH3-Domänen gebildet wird, oder
iv) beide Modifikationen von i) und ii),
wobei der multispezifische Antikörper keine konstanten Domänen der schweren Kette 2 (CH2) und keine Gelenkregion aufweist.

2. Multispezifischer Antikörper nach Anspruch 1, wobei die erste und/oder die zweite und/oder die dritte Antigenbindungsstelle unabhängig voneinander durch Einbringung von Cystein-Resten an den folgenden Positionen disulfidstabilisiert wird/werden, um eine Disulfidbindung zwischen den VH- und VL-Domänen zu bilden (Nummerierung gemäß Kabat):
- VH an Position 44 und VL an Position 100,
- VH an Position 105 und VL an Position 43 oder
- VH an Position 101 und VL an Position 100.

3. Multispezifischer Antikörper nach einem der Ansprüche 1 bis 2, wobei der erste, zweite, dritte und vierte Peptidlinker Peptide aus mindestens 5 Aminosäuren sind.

4. Multispezifischer Antikörper nach einem der Ansprüche 1 bis 3, wobei der erste und dritte Peptidlinker die Aminosäuresequenz von SEQ ID NO:38 aufweisen und der zweite und vierte Peptidlinker die Aminosäuresequenz von SEQ ID NO:16 aufweisen.

5. Multispezifischer Antikörper nach einem der Ansprüche 1 bis 4, wobei der C-Terminus der VH3-Domäne direkt mit dem N-Terminus einer der CH3-Domänen verbunden ist und der C-Terminus der VL3-Domäne direkt mit dem N-Terminus der anderen der CH3-Domänen verbunden ist.

6. Multispezifischer Antikörper nach einem der Ansprüche 1 bis 5, wobei der Antikörper trivalent ist.

7. Multispezifischer Antikörper nach einem der Ansprüche 1 bis 6, wobei der Antikörper bispezifisch ist (die erste Bindungsstelle bindet an ein erstes Antigen, die zweite Bindungsstelle bindet an ein zweites Antigen und die dritte Bindungsstelle bindet an ein drittes Antigen, wobei zwei von dem ersten, dem zweiten und dem dritten Antigen dasselbe Antigen sind und das andere ein anderes Antigen ist) oder trispezifisch ist (die erste Bindungsstelle bindet an ein erstes Antigen, die zweite Bindungsstelle bindet an ein zweites Antigen und die dritte Bindungsstelle bindet an ein drittes Antigen, wobei das erste, das zweite und das dritte Antigen verschiedene Antigene sind).

8. Multispezifischer Antikörper nach einem der Ansprüche 1 bis 7, wobei der erste Teil der ersten Bindungsdomäne und der zweite Teil der ersten Bindungsdomäne durch eine Disulfidbindung kovalent miteinander verbunden sind und/oder wobei der erste Teil der zweiten Bindungsdomäne und der zweite Teil der zweiten Bindungsdomäne durch eine Disulfidbindung kovalent miteinander verbunden sind.

9. Multispezifischer Antikörper nach einem der Ansprüche 1 bis 8, wobei die dritte Bindungsstelle spezifisch an humanes CD3 bindet.

10. Verfahren zur Herstellung des multispezifischen Antikörpers nach einem der Ansprüche 1 bis 9, umfassend die folgenden Schritte
- Transformieren einer Wirtszelle mit Expressionsvektoren, die Nukleinsäuren umfassen, die für den multispezifischen Antikörper kodieren,
- Kultivieren der Wirtszelle unter Bedingungen, die die Synthese des multispezifischen Antikörpers ermöglichen, und
- Gewinnen des multispezifischen Antikörpers aus der Wirtszellenkultur.

11. Multispezifischer Antikörper, produziert durch das Verfahren nach Anspruch 10.

12. Satz von Nukleinsäuren, die für die Polypeptide des multispezifischen Antikörpers nach einem der Ansprüche 1 bis 9 kodieren.

13. Expressionsvektor, umfassend eine Nukleinsäure nach Anspruch 12, oder Satz von Expressionsvektoren, die jeweils eine der Nukleinsäuren nach Anspruch 12 umfassen.

14. Wirtszelle, umfassend die Nukleinsäuren nach Anspruch 12.

15. Pharmazeutische Zusammensetzung, umfassend den multispezifischen Antikörper nach einem der Ansprüche 1 bis 9, gegebenenfalls in Kombination mit mindestens einem pharmazeutisch unbedenklichen Träger.

## Revendications

1. Anticorps multispécifique comprenant trois sites de liaison à l'antigène et constitué de deux polypeptides de fusion circulaires, dans lequel
a) le premier polypeptide de fusion circulaire comprend une première partie d'un premier domaine de liaison, une seconde partie d'un premier domaine de liaison et une première partie d'un troisième domaine de liaison, dans lequel
- la première partie du premier domaine de liaison est fusionnée directement ou par l'intermédiaire d'un premier lieur peptidique à l'extrémité N-terminale de la première partie du troisième domaine de liaison,
- la seconde partie du premier domaine de liaison est fusionnée directement ou par l'intermédiaire d'un deuxième lieur peptidique à l'extrémité C-terminale de la première partie du troisième domaine de liaison,
- la première partie du premier domaine de liaison est un fragment Fab de chaîne lourde (VH1-CH1) ou un fragment Fab de chaîne légère (VL1-CL1), moyennant quoi la seconde partie du premier domaine de liaison est un fragment Fab de chaîne légère si la première partie du premier domaine de liaison est un fragment Fab de chaîne lourde, ou vice versa, et
- la première partie du premier domaine de liaison et la seconde partie du premier domaine de liaison sont associées l'une à l'autre et constituent ensemble le premier site de liaison à l'antigène,
b) le second polypeptide de fusion circulaire comprend une première partie d'un deuxième domaine de liaison, une seconde partie d'un deuxième domaine de liaison et la seconde partie du troisième domaine de liaison, dans lequel
- la première partie du deuxième domaine de liaison est fusionnée directement ou par l'intermédiaire d'un troisième lieur peptidique à l'extrémité N-terminale de la seconde partie du troisième domaine de liaison,
- la seconde partie du deuxième domaine de liaison est fusionnée directement ou par l'intermédiaire d'un quatrième lieur peptidique à l'extrémité C-terminale de la seconde partie du troisième domaine de liaison,
- la première partie du deuxième domaine de liaison est un fragment Fab de chaîne lourde (VH2-CH1) ou un fragment Fab de chaîne légère (VL2-CL1), moyennant quoi la seconde partie du deuxième domaine de liaison est un fragment Fab de chaîne légère si la première partie du deuxième domaine de liaison est un fragment Fab de chaîne lourde, ou vice versa,
- la première partie du deuxième domaine de liaison et la seconde partie du deuxième domaine de liaison sont associées l'une à l'autre et constituent ensemble le deuxième site de liaison à l'antigène,
c) la première partie du troisième domaine de liaison et la seconde partie du troisième domaine de liaison constituent ensemble le troisième site de liaison à l'antigène, dans lequel
- la première partie du troisième domaine de liaison est un polypeptide de fusion chaîne lourde variable-domaine CH3 (VH3-CH3) ou un polypeptide de fusion chaîne légère variable-domaine CH3 (VL3-CH3),
- la seconde partie du troisième domaine de liaison est un polypeptide de fusion chaîne lourde variable-domaine CH3 si la première partie du deuxième domaine de liaison est un polypeptide de fusion chaîne légère variable-domaine CH3, ou vice versa,
- la première partie du troisième domaine de liaison et la seconde partie du troisième domaine de liaison sont associées l'une à l'autre et forment le troisième site de liaison à l'antigène,
dans lequel les deux domaines constants de chaîne lourde 3 (CH3) sont modifiés pour favoriser l'hétérodimérisation par
i) génération d'une protubérance dans l'un des domaines CH3 par substitution d'au moins un résidu d'acide aminé d'origine par un résidu d'acide aminé ayant un volume de chaîne latérale plus grand que le résidu d'acide aminé d'origine, et génération d'une cavité dans l'autre des domaines CH3 par substitution d'au moins un résidu d'acide aminé d'origine par un résidu d'acide aminé ayant un volume de chaîne latérale plus petit que le résidu d'acide aminé d'origine, de sorte que la protubérance générée dans l'un des domaines CH3 est positionnable dans la cavité générée dans l'autre des domaines CH3, ou
substitution d'au moins un résidu d'acide aminé d'origine dans un des domaines CH3 par un acide aminé chargé positivement, et substitution d'au moins un résidu d'acide aminé d'origine dans l'autre des domaines CH3 par un acide aminé chargé négativement, ou
ii) introduction d'au moins un résidu cystéine dans chaque domaine CH3 de sorte qu'une liaison disulfure est formée entre les domaines CH3, ou
iv) les deux modifications de i) et ii),
dans lequel l'anticorps multispécifique est dépourvu de domaines constants de chaîne lourde 2 (CH2) et d'une région charnière.

2. Anticorps multispécifique selon la revendication 1, dans lequel le premier et/ou le deuxième et/ou le troisième site de liaison à l'antigène est indépendamment des autres un disulfure stabilisé par l'introduction de résidus cystéine au niveau des positions suivantes pour former une liaison disulfure entre les domaines VH et VL (numérotation selon Kabat) :
- VH au niveau de la position 44 et VL au niveau de la position 100,
- VH au niveau de la position 105 et VL au niveau de la position 43, ou
- VH au niveau de la position 101 et VL au niveau de la position 100.

3. Anticorps multispécifique selon l'une quelconque des revendications 1 à 2, dans lequel les premier, deuxième, troisième et quatrième lieurs peptidiques sont des peptides d'au moins 5 acides aminés.

4. Anticorps multispécifique selon l'une quelconque des revendications 1 à 3, dans lequel le premier et le troisième lieur peptidique ont la séquence d'acides aminés de SEQ ID NO : 38 ; et le deuxième et le quatrième lieur peptidique ont la séquence d'acides aminés de SEQ ID NO : 16.

5. Anticorps multispécifique selon l'une quelconque des revendications 1 à 4, dans lequel l'extrémité C-terminale du domaine VH3 est reliée directement à l'extrémité N-terminale de l'un des domaines CH3 et l'extrémité C-terminale du domaine VL3 est reliée directement à l'extrémité N-terminale de l'autre des domaines CH3.

6. Anticorps multispécifique selon l'une quelconque des revendications 1 à 5, dans lequel l'anticorps est trivalent.

7. Anticorps multispécifique selon l'une quelconque des revendications 1 à 6, dans lequel l'anticorps est bispécifique (le premier site de liaison se lie à un premier antigène, le deuxième site de liaison se lie à un deuxième antigène et le troisième site de liaison se lie à un troisième antigène, moyennant quoi deux du premier, du deuxième et du troisième antigène sont le même antigène et l'autre est un antigène différent) ; ou trispécifique (le premier site de liaison se lie à un premier antigène, le deuxième site de liaison se lie à un deuxième antigène et le troisième site de liaison se lie à un troisième antigène, moyennant quoi le premier, le deuxième et le troisième antigène sont des antigènes différents).

8. Anticorps multispécifique selon l'une quelconque des revendications 1 à 7, dans lequel la première partie du premier domaine de liaison et la seconde partie du premier domaine de liaison sont associées l'une à l'autre de manière covalente par une liaison disulfure, et/ou dans lequel la première partie du deuxième domaine de liaison et la seconde partie du deuxième domaine de liaison sont associées l'une à l'autre de manière covalente par une liaison disulfure.

9. Anticorps multispécifique selon l'une quelconque des revendications 1 à 8, dans lequel le troisième site de liaison se lie spécifiquement au CD3 humain.

10. Procédé de préparation de l'anticorps multispécifique selon l'une quelconque des revendications 1 à 9, comprenant les étapes de
- transformation d'une cellule hôte avec des vecteurs d'expression comprenant des acides nucléiques codant pour l'anticorps multispécifique,
- culture de ladite cellule hôte dans des conditions qui permettent la synthèse dudit anticorps multispécifique, et
- récupération dudit anticorps multispécifique de ladite culture de cellule hôte.

11. Anticorps multispécifique produit par le procédé selon la revendication 10.

12. Ensemble d'acides nucléiques codant pour les polypeptides de l'anticorps multispécifique selon l'une quelconque des revendications 1 à 9.

13. Vecteur d'expression comprenant un acide nucléique selon la revendication 12 ou un ensemble de vecteurs d'expression comprenant chacun l'un des acides nucléiques selon la revendication 12.

14. Cellule hôte comprenant les acides nucléiques selon la revendication 12.

15. Composition pharmaceutique comprenant l'anticorps multispécifique selon l'une quelconque des revendications 1 à 9, éventuellement en combinaison avec au moins un véhicule pharmaceutiquement acceptable.


### SEQUENCE LISTING

<110> F. Hoffmann-La Roche AG
<120> TriFab-Contorsbody
<130> P34509-WO
<150> EP17199608.5
   <151> 2017-11-01
<160> 38
<170> PatentIn version 3.5
<210> 1
   <211> 98
   <212> PRT
   <213> Homo sapiens
<400> 1
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val
<210> 2
   <211> 107
   <212> PRT
   <213> Homo sapiens
<400> 2
   Ala Pro Glu Leu Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Trp Asp Val Ser His Glu Asp Pro Glu Val Lys Phe Asn Trp Tyr Val
           35                  40                  45
   Asp Gly Val Glu Val His Asn Ala Lys Thr Lys Pro Arg Glu Glu Gln
       50                  55                  60
   Glu Ser Thr Tyr Arg Trp Ser Val Leu Thr Val Leu His Gln Asp Trp
   65                  70                  75                  80
   Leu Asn Gly Lys Glu Tyr Lys Cys Lys Val Ser Asn Lys Ala Leu Pro
                   85                  90                  95
   Ala Pro Ile Glu Lys Thr Ile Ser Lys Ala Lys
               100                 105
<210> 3
   <211> 105
   <212> PRT
   <213> Homo sapiens
<400> 3
   Gly Gln Pro Arg Glu Pro Gln Val Tyr Thr Leu Pro Pro Ser Arg Asp
   1               5                   10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro
               100                 105
<210> 4
   <211> 107
   <212> PRT
   <213> Homo sapiens
<400> 4
<210> 5
   <211> 105
   <212> PRT
   <213> Homo sapiens
<400> 5
   Gln Pro Lys Ala Ala Pro Ser Val Thr Leu Phe Pro Pro Ser Ser Glu
   1               5                   10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Ser Ser Pro Val
           35                  40                  45
   Lys Ala Gly Val Glu Thr Thr Thr Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Cys Ser
               100                 105
<210> 6
   <211> 4
   <212> PRT
   <213> Artificial Sequence
<220>
   <223> peptidic linker
<400> 6
   Gly Gly Gly Ser
   1
<210> 7
   <211> 5
   <212> PRT
   <213> Artificial Sequence
<220>
   <223> peptidic linker
<400> 7
   Gly Gly Gly Gly Ser
   1               5
<210> 8
   <211> 4
   <212> PRT
   <213> Artificial Sequence
<220>
   <223> peptidic linker
<400> 8
   Gln Gln Gln Ser
   1
<210> 9
   <211> 5
   <212> PRT
   <213> Artificial Sequence
<220>
   <223> peptidic linker
<400> 9
   Gln Gln Gln Gln Ser
   1               5
<210> 10
   <211> 4
   <212> PRT
   <213> Artificial Sequence
<220>
   <223> peptidic linker
<400> 10
   Ser Ser Ser Gly
   1
<210> 11
   <211> 5
   <212> PRT
   <213> Artificial Sequence
<220>
   <223> peptidic linker
<400> 11
   Ser Ser Ser Ser Gly
   1 5
<210> 12
   <211> 8
   <212> PRT
   <213> Artificial Sequence
<220>
   <223> peptidic linker
<400> 12
   Gly Gly Gly Ser Gly Gly Gly Ser
   1 5
<210> 13
   <211> 12
   <212> PRT
   <213> Artificial Sequence
<220>
   <223> peptidic linker
<400> 13
   Gly Gly Gly Ser Gly Gly Gly Ser Gly Gly Gly Ser
   1 5 10
<210> 14
   <211> 16
   <212> PRT
   <213> Artificial Sequence
<220>
   <223> peptidic linker
<400> 14
   Gly Gly Gly Ser Gly Gly Gly Ser Gly Gly Gly Ser Gly Gly Gly Ser
   1 5 10 15
<210> 15
   <211> 20
   <212> PRT
   <213> Artificial Sequence
<220>
   <223> peptidic linker
<400> 15
   Gly Gly Gly Ser Gly Gly Gly Ser Gly Gly Gly Ser Gly Gly Gly Ser
   1               5                   10                  15
   Gly Gly Gly Ser
               20
<210> 16
   <211> 10
   <212> PRT
   <213> Artificial Sequence
<220>
   <223> peptidic linker
<400> 16
   Gly Gly Gly Gly Ser Gly Gly Gly Gly Ser
   1               5                   10
<210> 17
   <211> 15
   <212> PRT
   <213> Artificial Sequence
<220>
   <223> peptidic linker
<400> 17
   Gly Gly Gly Gly Ser Gly Gly Gly Gly Ser Gly Gly Gly Gly Ser
   1               5                   10                  15
<210> 18
   <211> 20
   <212> PRT
   <213> Artificial Sequence
<220>
   <223> peptidic linker
<400> 18
   Gly Gly Gly Gly Ser Gly Gly Gly Gly Ser Gly Gly Gly Gly Ser Gly
   1               5                   10                  15
   Gly Gly Gly Ser
               20
<210> 19
   <211> 22
   <212> PRT
   <213> Artificial Sequence
<220>
   <223> peptidic linker
<400> 19
   Gly Gly Gly Gly Ser Gly Gly Gly Gly Ser Gly Gly Gly Gly Ser Gly
   1               5                   10                  15
   Gly Gly Gly Ser Gly Gly
               20
<210> 20
   <211> 18
   <212> PRT
   <213> Artificial Sequence
<220>
   <223> peptidic linker
<400> 20
   Gly Gly Ser Gly Gly Gly Gly Ser Gly Gly Gly Gly Ser Gly Gly Gly
   1               5                   10                  15
   Gly Ser
<210> 21
   <211> 30
   <212> PRT
   <213> Artificial Sequence
<220>
   <223> peptidic linker
<400> 21
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser
               20                  25                  30
<210> 22
   <211> 17
   <212> PRT
   <213> Artificial Sequence
<220>
   <223> peptidic linker
<400> 22
   Gly Ser Ser Ser Ser Ser Ser Ser Ser Ser Ser Ser Ser Ser Ser Ser
   1               5                   10                  15
   Gly
<210> 23
   <211> 693
   <212> PRT
   <213> Artificial Sequence
<220>
   <223> anti-Her2 circular fusion polypeptide
<400> 23
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
           35                  40                  45
   Ala Arg Ile Tyr Pro Thr Asn Gly Tyr Thr Arg Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ser Arg Trp Gly Gly Asp Gly Phe Tyr Ala Met Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Gly
       210                 215                 220
   Gly Gly Gly Ser Gly Gly Gly Gly Ser Asp Lys Thr His Thr Cys Pro
   225                 230                 235                 240
   Pro Cys Pro Ala Pro Glu Leu Leu Gly Gly Pro Ser Val Phe Leu Phe
                   245                 250                 255
   Pro Pro Lys Pro Lys Asp Thr Leu Met Ile Ser Arg Thr Pro Glu Val
               260                 265                 270
   Thr Cys Val Val Val Asp Val Ser His Glu Asp Pro Glu Val Lys Phe
           275                 280                 285
   Asn Trp Tyr Val Asp Gly Val Glu Val His Asn Ala Lys Thr Lys Pro
       290                 295                 300
   Arg Glu Glu Gln Tyr Asn Ser Thr Tyr Arg Val Val Ser Val Leu Thr
   305                 310                 315                 320
   Val Leu His Gln Asp Trp Leu Asn Gly Lys Glu Tyr Lys Cys Lys Val
                   325                 330                 335
   Ser Asn Lys Ala Leu Pro Ala Pro Ile Glu Lys Thr Ile Ser Lys Ala
               340                 345                 350
   Lys Gly Gln Pro Arg Glu Pro Gln Val Tyr Thr Leu Pro Pro Ser Arg
           355                 360                 365
   Asp Glu Leu Thr Lys Asn Gln Val Ser Leu Thr Cys Leu Val Lys Gly
       370                 375                 380
   Phe Tyr Pro Ser Asp Ile Ala Val Glu Trp Glu Ser Asn Gly Gln Pro
   385                 390                 395                 400
   Glu Asn Asn Tyr Lys Thr Thr Pro Pro Val Leu Asp Ser Asp Gly Ser
                   405                 410                 415
   Phe Phe Leu Tyr Ser Lys Leu Thr Val Asp Lys Ser Arg Trp Gln Gln
   420 425 430
   Gly Asn Val Phe Ser Cys Ser Val Met His Glu Ala Leu His Asn His
   435 440 445
   Tyr Thr Gln Lys Ser Leu Ser Leu Ser Pro Gly Lys Gly Gly Gly Gly
   450 455 460
   Ser Gly Gly Gly Gly Ser Asp Ile Gln Met Thr Gln Ser Pro Ser Ser
   465 470 475 480
   Leu Ser Ala Ser Val Gly Asp Arg Val Thr Ile Thr Cys Arg Ala Ser
   485 490 495
   Gln Asp Val Asn Thr Ala Val Ala Trp Tyr Gln Gln Lys Pro Gly Lys
   500 505 510
   Ala Pro Lys Leu Leu Ile Tyr Ser Ala Ser Phe Leu Tyr Ser Gly Val
   515 520 525
   Pro Ser Arg Phe Ser Gly Ser Arg Ser Gly Thr Asp Phe Thr Leu Thr
   530 535 540
   Ile Ser Ser Leu Gln Pro Glu Asp Phe Ala Thr Tyr Tyr Cys Gln Gln
   545 550 555 560
   His Tyr Thr Thr Pro Pro Thr Phe Gly Gln Gly Thr Lys Val Glu Ile
   565 570 575
   Lys Arg Thr Val Ala Ala Pro Ser Val Phe Ile Phe Pro Pro Ser Asp
   580 585 590
   Glu Gln Leu Lys Ser Gly Thr Ala Ser Val Val Cys Leu Leu Asn Asn
   595 600 605
   Phe Tyr Pro Arg Glu Ala Lys Val Gln Trp Lys Val Asp Asn Ala Leu
   610 615 620
   Gln Ser Gly Asn Ser Gln Glu Ser Val Thr Glu Gln Asp Ser Lys Asp
   625 630 635 640
   Ser Thr Tyr Ser Leu Ser Ser Thr Leu Thr Leu Ser Lys Ala Asp Tyr
   645 650 655
   Glu Lys His Lys Val Tyr Ala Cys Glu Val Thr His Gln Gly Leu Ser
   660 665 670
   Ser Pro Val Thr Lys Ser Phe Asn Arg Gly Glu Cys Gly Ser Gly His
   675 680 685
   His His His His His
   690
<210> 24
   <211> 6
   <212> PRT
   <213> Artificial Sequence
<220>
   <223> His-tag
<400> 24
   His His His His His His
   1 5
<210> 25
   <211> 692
   <212> PRT
   <213> Artificial Sequence
<220>
   <223> anti-cMet circular fusion polypeptide
<400> 25
   Asp Ile Gln Met Thr Gln Ser Pro Ser Ser Leu Ser Ala Ser Val Gly
   1               5                   10                  15
   Asp Arg Val Thr Ile Thr Cys Arg Thr Ser Glu Asn Ile Tyr Ser Tyr
               20                  25                  30
   Leu Ala Trp Tyr Gln Gln Lys Pro Gly Lys Ala Pro Lys Leu Leu Val
           35                  40                  45
   Tyr Asn Ala Lys Thr Leu Ala Glu Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln His His Tyr Gly Thr Pro Phe
                   85                  90                  95
   Thr Phe Gly Gln Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Gly Gly Gly Gly Ser Gly Gly Gly Gly Ser
   210 215 220
   Gly Gly Gly Gly Ser Asp Lys Thr His Thr Cys Pro Pro Cys Pro Ala
   225 230 235 240
   Pro Glu Leu Leu Gly Gly Pro Ser Val Phe Leu Phe Pro Pro Lys Pro
   245 250 255
   Lys Asp Thr Leu Met Ile Ser Arg Thr Pro Glu Val Thr Cys Val Val
   260 265 270
   Val Asp Val Ser His Glu Asp Pro Glu Val Lys Phe Asn Trp Tyr Val
   275 280 285
   Asp Gly Val Glu Val His Asn Ala Lys Thr Lys Pro Arg Glu Glu Gln
   290 295 300
   Tyr Asn Ser Thr Tyr Arg Val Val Ser Val Leu Thr Val Leu His Gln
   305 310 315 320
   Asp Trp Leu Asn Gly Lys Glu Tyr Lys Cys Lys Val Ser Asn Lys Ala
   325 330 335
   Leu Pro Ala Pro Ile Glu Lys Thr Ile Ser Lys Ala Lys Gly Gln Pro
   340 345 350
   Arg Glu Pro Gln Val Tyr Thr Leu Pro Pro Ser Arg Asp Glu Leu Thr
   355 360 365
   Lys Asn Gln Val Ser Leu Thr Cys Leu Val Lys Gly Phe Tyr Pro Ser
   370 375 380
   Asp Ile Ala Val Glu Trp Glu Ser Asn Gly Gln Pro Glu Asn Asn Tyr
   385 390 395 400
   Lys Thr Thr Pro Pro Val Leu Asp Ser Asp Gly Ser Phe Phe Leu Tyr
   405 410 415
   Ser Lys Leu Thr Val Asp Lys Ser Arg Trp Gln Gln Gly Asn Val Phe
   420 425 430
   Ser Cys Ser Val Met His Glu Ala Leu His Asn His Tyr Thr Gln Lys
   435 440 445
   Ser Leu Ser Leu Ser Pro Gly Lys Gly Gly Gly Gly Ser Gly Gly Gly
   450 455 460
   Gly Ser Gln Val Gln Leu Gln Glu Ser Gly Pro Gly Leu Val Lys Pro
   465 470 475 480
   Ser Glu Thr Leu Ser Leu Thr Cys Thr Val Ser Gly Tyr Ser Ile Thr
   485 490 495
   Ser Asp Tyr Ala Trp Asn Trp Ile Arg Gln Phe Pro Gly Lys Gly Leu
   500 505 510
   Glu Trp Ile Gly Tyr Ile Ser Tyr Ser Gly Ser Thr Ser Tyr Leu Pro
   515 520 525
   Ser Leu Lys Ser Arg Val Thr Ile Ser Arg Asp Thr Ser Lys Asn Gln
   530 535 540
   Phe Ser Leu Lys Leu Ser Ser Val Thr Ala Ala Asp Thr Ala Val Tyr
   545 550 555 560
   Tyr Cys Ala Pro Ser Tyr Tyr Tyr Gly Gly Lys His Val Ala Leu Phe
   565 570 575
   Ala Tyr Trp Gly Gln Gly Thr Leu Val Thr Val Ser Ser Ala Ser Thr
   580 585 590
   Lys Gly Pro Ser Val Phe Pro Leu Ala Pro Ser Ser Lys Ser Thr Ser
   595 600 605
   Gly Gly Thr Ala Ala Leu Gly Cys Leu Val Lys Asp Tyr Phe Pro Glu
   610 615 620
   Pro Val Thr Val Ser Trp Asn Ser Gly Ala Leu Thr Ser Gly Val His
   625                 630                 635                 640
   Thr Phe Pro Ala Val Leu Gln Ser Ser Gly Leu Tyr Ser Leu Ser Ser
                   645                 650                 655
   Val Val Thr Val Pro Ser Ser Ser Leu Gly Thr Gln Thr Tyr Ile Cys
               660                 665                 670
   Asn Val Asn His Lys Pro Ser Asn Thr Lys Val Asp Lys Lys Val Glu
           675                 680                 685
   Pro Lys Ser Cys
       690
<210> 26
   <211> 693
   <212> PRT
   <213> Artificial Sequence
<220>
   <223> anti-CD20 circular fusion polypeptide (1)
<400> 26
   Gln Val Gln Leu Gln Gln Pro Gly Ala Glu Leu Val Lys Pro Gly Ala
   1               5                   10                  15
   Ser Val Lys Met Ser Cys Lys Ala Ser Gly Tyr Thr Phe Thr Ser Tyr
               20                  25                  30
   Asn Met His Trp Val Lys Gln Thr Pro Gly Arg Gly Leu Glu Trp Ile
           35                  40                  45
   Gly Ala Ile Tyr Pro Gly Asn Gly Asp Thr Ser Tyr Asn Gln Lys Phe
       50                  55                  60
   Lys Gly Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Ser Thr Tyr Tyr Gly Gly Asp Trp Tyr Phe Asn Val Trp Gly
               100                 105                 110
   Ala Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Gly Gly Gly Gly Ser Gly Gly Gly Gly Ser Asp Lys Thr His Thr Cys
   225 230 235 240
   Pro Pro Cys Pro Ala Pro Glu Leu Leu Gly Gly Pro Ser Val Phe Leu
   245 250 255
   Phe Pro Pro Lys Pro Lys Asp Thr Leu Met Ile Ser Arg Thr Pro Glu
   260 265 270
   Val Thr Cys Val Val Val Asp Val Ser His Glu Asp Pro Glu Val Lys
   275 280 285
   Phe Asn Trp Tyr Val Asp Gly Val Glu Val His Asn Ala Lys Thr Lys
   290 295 300
   Pro Arg Glu Glu Gln Tyr Asn Ser Thr Tyr Arg Val Val Ser Val Leu
   305 310 315 320
   Thr Val Leu His Gln Asp Trp Leu Asn Gly Lys Glu Tyr Lys Cys Lys
   325 330 335
   Val Ser Asn Lys Ala Leu Pro Ala Pro Ile Glu Lys Thr Ile Ser Lys
   340 345 350
   Ala Lys Gly Gln Pro Arg Glu Pro Gln Val Tyr Thr Leu Pro Pro Ser
   355 360 365
   Arg Asp Glu Leu Thr Lys Asn Gln Val Ser Leu Thr Cys Leu Val Lys
   370 375 380
   Gly Phe Tyr Pro Ser Asp Ile Ala Val Glu Trp Glu Ser Asn Gly Gln
   385 390 395 400
   Pro Glu Asn Asn Tyr Lys Thr Thr Pro Pro Val Leu Asp Ser Asp Gly
   405 410 415
   Ser Phe Phe Leu Tyr Ser Lys Leu Thr Val Asp Lys Ser Arg Trp Gln
   420 425 430
   Gln Gly Asn Val Phe Ser Cys Ser Val Met His Glu Ala Leu His Asn
   435 440 445
   His Tyr Thr Gln Lys Ser Leu Ser Leu Ser Pro Gly Lys Gly Gly Gly
   450 455 460
   Gly Ser Gly Gly Gly Gly Ser Gln Ile Val Leu Ser Gln Ser Pro Ala
   465 470 475 480
   Ile Leu Ser Ala Ser Pro Gly Glu Lys Val Thr Met Thr Cys Arg Ala
   485 490 495
   Ser Ser Ser Val Ser Tyr Ile His Trp Phe Gln Gln Lys Pro Gly Ser
   500 505 510
   Ser Pro Lys Pro Trp Ile Tyr Ala Thr Ser Asn Leu Ala Ser Gly Val
   515 520 525
   Pro Val Arg Phe Ser Gly Ser Gly Ser Gly Thr Ser Tyr Ser Leu Thr
   530 535 540
   Ile Ser Arg Val Glu Ala Glu Asp Ala Ala Thr Tyr Tyr Cys Gln Gln
   545 550 555 560
   Trp Thr Ser Asn Pro Pro Thr Phe Gly Gly Gly Thr Lys Leu Glu Ile
   565 570 575
   Lys Arg Thr Val Ala Ala Pro Ser Val Phe Ile Phe Pro Pro Ser Asp
   580 585 590
   Glu Gln Leu Lys Ser Gly Thr Ala Ser Val Val Cys Leu Leu Asn Asn
   595 600 605
   Phe Tyr Pro Arg Glu Ala Lys Val Gln Trp Lys Val Asp Asn Ala Leu
   610 615 620
   Gln Ser Gly Asn Ser Gln Glu Ser Val Thr Glu Gln Asp Ser Lys Asp
   625 630 635 640
   Ser Thr Tyr Ser Leu Ser Ser Thr Leu Thr Leu Ser Lys Ala Asp Tyr
   645 650 655
   Glu Lys His Lys Val Tyr Ala Cys Glu Val Thr His Gln Gly Leu Ser
   660 665 670
   Ser Pro Val Thr Lys Ser Phe Asn Arg Gly Glu Cys Gly Ser Gly His
   675 680 685
   His His His His His
   690
<210> 27
   <211> 697
   <212> PRT
   <213> Artificial Sequence
<220>
   <223> anti-CD20 circular fusion polypeptide (2)
<400> 27
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Ile Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Ile Cys Asn Val Asn His Lys Pro
   195 200 205
   Ser Asn Thr Lys Val Asp Lys Lys Val Glu Pro Lys Ser Cys Gly Gly
   210 215 220
   Gly Gly Ser Gly Gly Gly Gly Ser Asp Lys Thr His Thr Cys Pro Pro
   225 230 235 240
   Cys Pro Ala Pro Glu Leu Leu Gly Gly Pro Ser Val Phe Leu Phe Pro
   245 250 255
   Pro Lys Pro Lys Asp Thr Leu Met Ile Ser Arg Thr Pro Glu Val Thr
   260 265 270
   Cys Val Val Val Asp Val Ser His Glu Asp Pro Glu Val Lys Phe Asn
   275 280 285
   Trp Tyr Val Asp Gly Val Glu Val His Asn Ala Lys Thr Lys Pro Arg
   290 295 300
   Glu Glu Gln Tyr Asn Ser Thr Tyr Arg Val Val Ser Val Leu Thr Val
   305 310 315 320
   Leu His Gln Asp Trp Leu Asn Gly Lys Glu Tyr Lys Cys Lys Val Ser
   325 330 335
   Asn Lys Ala Leu Pro Ala Pro Ile Glu Lys Thr Ile Ser Lys Ala Lys
   340 345 350
   Gly Gln Pro Arg Glu Pro Gln Val Tyr Thr Leu Pro Pro Ser Arg Asp
   355 360 365
   Glu Leu Thr Lys Asn Gln Val Ser Leu Thr Cys Leu Val Lys Gly Phe
   370 375 380
   Tyr Pro Ser Asp Ile Ala Val Glu Trp Glu Ser Asn Gly Gln Pro Glu
   385 390 395 400
   Asn Asn Tyr Lys Thr Thr Pro Pro Val Leu Asp Ser Asp Gly Ser Phe
                   405                 410                 415
   Phe Leu Tyr Ser Lys Leu Thr Val Asp Lys Ser Arg Trp Gln Gln Gly
               420                 425                 430
   Asn Val Phe Ser Cys Ser Val Met His Glu Ala Leu His Asn His Tyr
           435                 440                 445
   Thr Gln Lys Ser Leu Ser Leu Ser Pro Gly Lys Gly Gly Gly Gly Ser
       450                 455                 460
   Gly Gly Gly Gly Ser Asp Ile Val Met Thr Gln Thr Pro Leu Ser Leu
   465                 470                 475                 480
   Pro Val Thr Pro Gly Glu Pro Ala Ser Ile Ser Cys Arg Ser Ser Lys
                   485                 490                 495
   Ser Leu Leu His Ser Asn Gly Ile Thr Tyr Leu Tyr Trp Tyr Leu Gln
               500                 505                 510
   Lys Pro Gly Gln Ser Pro Gln Leu Leu Ile Tyr Gln Met Ser Asn Leu
           515                 520                 525
   Val Ser Gly Val Pro Asp Arg Phe Ser Gly Ser Gly Ser Gly Thr Asp
       530                 535                 540
   Phe Thr Leu Lys Ile Ser Arg Val Glu Ala Glu Asp Val Gly Val Tyr
   545                 550                 555                 560
   Tyr Cys Ala Gln Asn Leu Glu Leu Pro Tyr Thr Phe Gly Gly Gly Thr
                   565                 570                 575
   Lys Val Glu Ile Lys Arg Thr Val Ala Ala Pro Ser Val Phe Ile Phe
               580                 585                 590
   Pro Pro Ser Asp Glu Gln Leu Lys Ser Gly Thr Ala Ser Val Val Cys
           595                 600                 605
   Leu Leu Asn Asn Phe Tyr Pro Arg Glu Ala Lys Val Gln Trp Lys Val
       610                 615                 620
   Asp Asn Ala Leu Gln Ser Gly Asn Ser Gln Glu Ser Val Thr Glu Gln
   625                 630                 635                 640
   Asp Ser Lys Asp Ser Thr Tyr Ser Leu Ser Ser Thr Leu Thr Leu Ser
                   645                 650                 655
   Lys Ala Asp Tyr Glu Lys His Lys Val Tyr Ala Cys Glu Val Thr His
   660 665 670
   Gln Gly Leu Ser Ser Pro Val Thr Lys Ser Phe Asn Arg Gly Glu Cys
   675 680 685
   Gly Ser Gly His His His His His His
   690 695
<210> 28
   <211> 689
   <212> PRT
   <213> Artificial Sequence
<220>
   <223> anti-cMet circular fusion polypeptide VH-out-knob
<400> 28
   Gln Val Gln Leu Gln Glu Ser Gly Pro Gly Leu Val Lys Pro Ser Glu
   1 5 10 15
   Thr Leu Ser Leu Thr Cys Thr Val Ser Gly Tyr Ser Ile Thr Ser Asp
   20 25 30
   Tyr Ala Trp Asn Trp Ile Arg Gln Phe Pro Gly Lys Gly Leu Glu Trp
   35 40 45
   Ile Gly Tyr Ile Ser Tyr Ser Gly Ser Thr Ser Tyr Leu Pro Ser Leu
   50 55 60
   Lys Ser Arg Val Thr Ile Ser Arg Asp Thr Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Pro Ser Tyr Tyr Tyr Gly Gly Lys His Val Ala Leu Phe Ala Tyr
   100 105 110
   Trp Gly Gln Gly Thr Leu Val Thr Val Ser Ser Ala Ser Thr Lys Gly
   115 120 125
   Pro Ser Val Phe Pro Leu Ala Pro Ser Ser Lys Ser Thr Ser Gly Gly
   130 135 140
   Thr Ala Ala Leu Gly Cys Leu Val Lys Asp Tyr Phe Pro Glu Pro Val
   145 150 155 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Gly Ser Gly Gly Gly Gly Ser Gly Gly Gly Gly Ser Asp Lys
   225                 230                 235                 240
   Thr His Thr Cys Pro Pro Cys Pro Ala Pro Glu Leu Leu Gly Gly Pro
                   245                 250                 255
   Ser Val Phe Leu Phe Pro Pro Lys Pro Lys Asp Thr Leu Met Ile Ser
               260                 265                 270
   Arg Thr Pro Glu Val Thr Cys Val Val Val Asp Val Ser His Glu Asp
           275                 280                 285
   Pro Glu Val Lys Phe Asn Trp Tyr Val Asp Gly Val Glu Val His Asn
       290                 295                 300
   Ala Lys Thr Lys Pro Arg Glu Glu Gln Tyr Asn Ser Thr Tyr Arg Val
   305                 310                 315                 320
   Val Ser Val Leu Thr Val Leu His Gln Asp Trp Leu Asn Gly Lys Glu
                   325                 330                 335
   Tyr Lys Cys Lys Val Ser Asn Lys Ala Leu Pro Ala Pro Ile Glu Lys
               340                 345                 350
   Thr Ile Ser Lys Ala Lys Gly Gln Pro Arg Glu Pro Gln Val Tyr Thr
           355                 360                 365
   Leu Pro Pro Cys Arg Asp Glu Leu Thr Lys Asn Gln Val Ser Leu Trp
       370                 375                 380
   Cys Leu Val Lys Gly Phe Tyr Pro Ser Asp Ile Ala Val Glu Trp Glu
   385                 390                 395                 400
   Ser Asn Gly Gln Pro Glu Asn Asn Tyr Lys Thr Thr Pro Pro Val Leu
                   405                 410                 415
   Asp Ser Asp Gly Ser Phe Phe Leu Tyr Ser Lys Leu Thr Val Asp Lys
   420 425 430
   Ser Arg Trp Gln Gln Gly Asn Val Phe Ser Cys Ser Val Met His Glu
   435 440 445
   Ala Leu His Asn His Tyr Thr Gln Lys Ser Leu Ser Leu Ser Pro Gly
   450 455 460
   Lys Gly Gly Gly Gly Ser Gly Gly Gly Gly Ser Asp Ile Gln Met Thr
   465 470 475 480
   Gln Ser Pro Ser Ser Leu Ser Ala Ser Val Gly Asp Arg Val Thr Ile
   485 490 495
   Thr Cys Arg Thr Ser Glu Asn Ile Tyr Ser Tyr Leu Ala Trp Tyr Gln
   500 505 510
   Gln Lys Pro Gly Lys Ala Pro Lys Leu Leu Val Tyr Asn Ala Lys Thr
   515 520 525
   Leu Ala Glu Gly Val Pro Ser Arg Phe Ser Gly Ser Gly Ser Gly Thr
   530 535 540
   Asp Phe Thr Leu Thr Ile Ser Ser Leu Gln Pro Glu Asp Phe Ala Thr
   545 550 555 560
   Tyr Tyr Cys Gln His His Tyr Gly Thr Pro Phe Thr Phe Gly Gln Gly
   565 570 575
   Thr Lys Leu Glu Ile Lys Arg Thr Val Ala Ala Pro Ser Val Phe Ile
   580 585 590
   Phe Pro Pro Ser Asp Glu Gln Leu Lys Ser Gly Thr Ala Ser Val Val
   595 600 605
   Cys Leu Leu Asn Asn Phe Tyr Pro Arg Glu Ala Lys Val Gln Trp Lys
   610 615 620
   Val Asp Asn Ala Leu Gln Ser Gly Asn Ser Gln Glu Ser Val Thr Glu
   625 630 635 640
   Gln Asp Ser Lys Asp Ser Thr Tyr Ser Leu Ser Ser Thr Leu Thr Leu
   645 650 655
   Ser Lys Ala Asp Tyr Glu Lys His Lys Val Tyr Ala Cys Glu Val Thr
   660 665 670
   His Gln Gly Leu Ser Ser Pro Val Thr Lys Ser Phe Asn Arg Gly Glu
   675 680 685
   Cys
<210> 30
   <211> 685
   <212> PRT
   <213> Artificial Sequence
<220>
   <223> anti-cMet circular fusion polypeptide VH-out-hole
<400> 30
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ala Tyr
               20                  25                  30
   Phe Ile Asn Trp Val Arg Gln Met Pro Gly Lys Gly Leu Glu Trp Met
           35                  40                  45
   Gly Arg Ile Tyr Pro Tyr Asn Gly Asn Thr Phe Tyr Asp Gln Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Asp Tyr Asn Tyr Asp Val Trp Gly Gln Gly Thr Thr Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
   195 200 205
   Lys Val Asp Lys Lys Val Glu Pro Lys Ser Cys Gly Gly Gly Gly Ser
   210 215 220
   Gly Gly Gly Gly Ser Gly Gly Gly Gly Ser Asp Lys Thr His Thr Cys
   225 230 235 240
   Pro Pro Cys Pro Ala Pro Glu Leu Leu Gly Gly Pro Ser Val Phe Leu
   245 250 255
   Phe Pro Pro Lys Pro Lys Asp Thr Leu Met Ile Ser Arg Thr Pro Glu
   260 265 270
   Val Thr Cys Val Val Val Asp Val Ser His Glu Asp Pro Glu Val Lys
   275 280 285
   Phe Asn Trp Tyr Val Asp Gly Val Glu Val His Asn Ala Lys Thr Lys
   290 295 300
   Pro Arg Glu Glu Gln Tyr Asn Ser Thr Tyr Arg Val Val Ser Val Leu
   305 310 315 320
   Thr Val Leu His Gln Asp Trp Leu Asn Gly Lys Glu Tyr Lys Cys Lys
   325 330 335
   Val Ser Asn Lys Ala Leu Pro Ala Pro Ile Glu Lys Thr Ile Ser Lys
   340 345 350
   Ala Lys Gly Gln Pro Arg Glu Pro Gln Val Cys Thr Leu Pro Pro Ser
   355 360 365
   Arg Asp Glu Leu Thr Lys Asn Gln Val Ser Leu Ser Cys Ala Val Lys
   370 375 380
   Gly Phe Tyr Pro Ser Asp Ile Ala Val Glu Trp Glu Ser Asn Gly Gln
   385 390 395 400
   Pro Glu Asn Asn Tyr Lys Thr Thr Pro Pro Val Leu Asp Ser Asp Gly
   405 410 415
   Ser Phe Phe Leu Val Ser Lys Leu Thr Val Asp Lys Ser Arg Trp Gln
   420 425 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys Gly Gly Gly
       450                 455                 460
   Gly Ser Gly Gly Gly Gly Ser Glu Ile Val Leu Thr Gln Ser Pro Ala
   465                 470                 475                 480
   Thr Leu Ser Leu Ser Pro Gly Glu Arg Ala Thr Leu Ser Cys Arg Ala
                   485                 490                 495
   Ser Ser Ser Val Ser His Leu Tyr Trp Tyr Gln Gln Lys Pro Gly Gln
               500                 505                 510
   Ala Pro Arg Leu Trp Ile Tyr Asp Thr Ser Asn Leu Ala Ser Gly Val
           515                 520                 525
   Pro Ala Arg Phe Ser Gly Ser Arg Ser Gly Thr Asp Phe Thr Leu Thr
       530                 535                 540
   Ile Ser Ser Leu Glu Pro Glu Asp Phe Ala Val Tyr Phe Cys His Gln
   545                 550                 555                 560
   Arg Thr Asn Tyr Pro Trp Thr Phe Gly Gln Gly Thr Lys Leu Glu Ile
                   565                 570                 575
   Lys Arg Thr Val Ala Ala Pro Ser Val Phe Ile Phe Pro Pro Ser Asp
               580                 585                 590
   Glu Gln Leu Lys Ser Gly Thr Ala Ser Val Val Cys Leu Leu Asn Asn
           595                 600                 605
   Phe Tyr Pro Arg Glu Ala Lys Val Gln Trp Lys Val Asp Asn Ala Leu
       610                 615                 620
   Gln Ser Gly Asn Ser Gln Glu Ser Val Thr Glu Gln Asp Ser Lys Asp
   625                 630                 635                 640
   Ser Thr Tyr Ser Leu Ser Ser Thr Leu Thr Ser Leu Ser Lys Ala Asp
                   645                 650                 655
   Tyr Glu Lys His Lys Val Tyr Ala Cys Glu Val Thr His Gln Gly Leu
               660                 665                 670
   Ser Ser Pro Val Thr Lys Ser Phe Asn Arg Gly Glu Cys
           675                 680                 685
<210> 31
   <211> 686
   <212> PRT
   <213> Artificial Sequence
<220>
   <223> anti-cMet circular fusion polypeptide VH-out-hole-CH-CL-crossed
<400> 31
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ala Tyr
               20                  25                  30
   Phe Ile Asn Trp Val Arg Gln Met Pro Gly Lys Gly Leu Glu Trp Met
           35                  40                  45
   Gly Arg Ile Tyr Pro Tyr Asn Gly Asn Thr Phe Tyr Asp Gln Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Asp Tyr Asn Tyr Asp Val Trp Gly Gln Gly Thr Thr Val
               100                 105                 110
   Thr Val Ser Ser Ser Ala Val Ala Ala Pro Ser Val Phe Ile Phe Pro
           115                 120                 125
   Pro Ser Asp Glu Gln Leu Lys Ser Gly Thr Ala Ser Val Val Cys Leu
       130                 135                 140
   Leu Asn Asn Phe Tyr Pro Arg Glu Ala Lys Val Gln Trp Lys Val Asp
   145                 150                 155                 160
   Asn Ala Leu Gln Ser Gly Asn Ser Gln Glu Ser Val Thr Glu Gln Asp
                   165                 170                 175
   Ser Lys Asp Ser Thr Tyr Ser Leu Ser Ser Thr Leu Thr Leu Ser Lys
               180                 185                 190
   Ala Asp Tyr Glu Lys His Lys Val Tyr Ala Cys Glu Val Thr His Gln
           195                 200                 205
   Gly Leu Ser Ser Pro Val Thr Lys Ser Phe Asn Arg Gly Glu Cys Gly
   210 215 220
   Gly Gly Gly Ser Gly Gly Gly Gly Ser Gly Gly Gly Gly Ser Asp Lys
   225 230 235 240
   Thr His Thr Cys Pro Pro Cys Pro Ala Pro Glu Leu Leu Gly Gly Pro
   245 250 255
   Ser Val Phe Leu Phe Pro Pro Lys Pro Lys Asp Thr Leu Met Ile Ser
   260 265 270
   Arg Thr Pro Glu Val Thr Cys Val Val Val Asp Val Ser His Glu Asp
   275 280 285
   Pro Glu Val Lys Phe Asn Trp Tyr Val Asp Gly Val Glu Val His Asn
   290 295 300
   Ala Lys Thr Lys Pro Arg Glu Glu Gln Tyr Asn Ser Thr Tyr Arg Val
   305 310 315 320
   Val Ser Val Leu Thr Val Leu His Gln Asp Trp Leu Asn Gly Lys Glu
   325 330 335
   Tyr Lys Cys Lys Val Ser Asn Lys Ala Leu Pro Ala Pro Ile Glu Lys
   340 345 350
   Thr Ile Ser Lys Ala Lys Gly Gln Pro Arg Glu Pro Gln Val Cys Thr
   355 360 365
   Leu Pro Pro Ser Arg Asp Glu Leu Thr Lys Asn Gln Val Ser Leu Ser
   370 375 380
   Cys Ala Val Lys Gly Phe Tyr Pro Ser Asp Ile Ala Val Glu Trp Glu
   385 390 395 400
   Ser Asn Gly Gln Pro Glu Asn Asn Tyr Lys Thr Thr Pro Pro Val Leu
   405 410 415
   Asp Ser Asp Gly Ser Phe Phe Leu Val Ser Lys Leu Thr Val Asp Lys
   420 425 430
   Ser Arg Trp Gln Gln Gly Asn Val Phe Ser Cys Ser Val Met His Glu
   435 440 445
   Ala Leu His Asn His Tyr Thr Gln Lys Ser Leu Ser Leu Ser Pro Gly
   450 455 460
   Lys Gly Gly Gly Gly Ser Gly Gly Gly Gly Ser Glu Ile Val Leu Thr
   465                 470                 475                 480
   Gln Ser Pro Ala Thr Leu Ser Leu Ser Pro Gly Glu Arg Ala Thr Leu
                   485                 490                 495
   Ser Cys Arg Ala Ser Ser Ser Val Ser His Leu Tyr Trp Tyr Gln Gln
               500                 505                 510
   Lys Pro Gly Gln Ala Pro Arg Leu Trp Ile Tyr Asp Thr Ser Asn Leu
           515                 520                 525
   Ala Ser Gly Val Pro Ala Arg Phe Ser Gly Ser Arg Ser Gly Thr Asp
       530                 535                 540
   Phe Thr Leu Thr Ile Ser Ser Leu Glu Pro Glu Asp Phe Ala Val Tyr
   545                 550                 555                 560
   Phe Cys His Gln Arg Thr Asn Tyr Pro Trp Thr Phe Gly Gln Gly Thr
                   565                 570                 575
   Lys Leu Glu Ile Lys Ser Ser Ala Ser Thr Lys Gly Pro Ser Val Phe
               580                 585                 590
   Pro Leu Ala Pro Ser Ser Lys Ser Thr Ser Gly Gly Thr Ala Ala Leu
           595                 600                 605
   Gly Cys Leu Val Lys Asp Tyr Phe Pro Glu Pro Val Thr Val Ser Trp
       610                 615                 620
   Asn Ser Gly Ala Leu Thr Ser Gly Val His Thr Phe Pro Ala Val Leu
   625                 630                 635                 640
   Gln Ser Ser Gly Leu Tyr Ser Leu Ser Ser Val Val Thr Val Pro Ser
                   645                 650                 655
   Ser Ser Leu Gly Thr Gln Thr Tyr Ile Cys Asn Val Asn His Lys Pro
               660                 665                 670
   Ser Asn Thr Lys Val Asp Lys Lys Val Glu Pro Lys Ser Cys
           675                 680                 685
<210> 32
   <211> 683
   <212> PRT
   <213> Artificial Sequence
<220>
   <223> anti-cMet circular fusion polypeptide VH-out-hole-VH-VL-crossed
<400> 32
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Ser Ser Val Ser His Leu
               20                  25                  30
   Tyr Trp Tyr Gln Gln Lys Pro Gly Gln Ala Pro Arg Leu Trp Ile Tyr
           35                  40                  45
   Asp Thr Ser Asn Leu Ala Ser Gly Val Pro Ala Arg Phe Ser Gly Ser
       50                  55                  60
   Arg Ser Gly Thr Asp Phe Thr Leu Thr Ile Ser Ser Leu Glu Pro Glu
   65                  70                  75                  80
   Asp Phe Ala Val Tyr Phe Cys His Gln Arg Thr Asn Tyr Pro Trp Thr
                   85                  90                  95
   Phe Gly Gln Gly Thr Lys Leu Glu Ile Lys Ser Ser Ala Ser Thr Lys
               100                 105                 110
   Gly Pro Ser Val Phe Pro Leu Ala Pro Ser Ser Lys Ser Thr Ser Gly
           115                 120                 125
   Gly Thr Ala Ala Leu Gly Cys Leu Val Lys Asp Tyr Phe Pro Glu Pro
       130                 135                 140
   Val Thr Val Ser Trp Asn Ser Gly Ala Leu Thr Ser Gly Val His Thr
   145                 150                 155                 160
   Phe Pro Ala Val Leu Gln Ser Ser Gly Leu Tyr Ser Leu Ser Ser Val
                   165                 170                 175
   Val Thr Val Pro Ser Ser Ser Leu Gly Thr Gln Thr Tyr Ile Cys Asn
               180                 185                 190
   Val Asn His Lys Pro Ser Asn Thr Lys Val Asp Lys Lys Val Glu Pro
           195                 200                 205
   Lys Ser Cys Gly Ser Gly Gly Gly Gly Ser Gly Gly Gly Gly Ser Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Cys
   340 345 350
   Thr Leu Pro Pro Ser Arg Asp Glu Leu Thr Lys Asn Gln Val Ser Leu
   355 360 365
   Ser Cys Ala Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Val Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys Gly Gly Gly Gly Ser Gly Gly Gly Gly Ser Glu Val Gln Leu
   450 455 460
   Val Gln Ser Gly Ala Glu Val Lys Lys Pro Gly Glu Ser Leu Lys Ile
   465 470 475 480
   Ser Cys Lys Gly Ser Gly Tyr Ser Phe Thr Ala Tyr Phe Ile Asn Trp
   485 490 495
   Val Arg Gln Met Pro Gly Lys Gly Leu Glu Trp Met Gly Arg Ile Tyr
   500 505 510
   Pro Tyr Asn Gly Asn Thr Phe Tyr Asp Gln Ser Phe Gln Gly Gln Val
   515 520 525
   Thr Ile Ser Ala Asp Lys Ser Ile Ser Thr Ala Tyr Leu Gln Trp Ser
   530 535 540
   Ser Leu Lys Ala Ser Asp Thr Ala Met Tyr Tyr Cys Ala Arg Trp Asp
   545 550 555 560
   Tyr Asn Tyr Asp Val Trp Gly Gln Gly Thr Thr Val Thr Val Ser Ser
   565 570 575
   Ser Ala Val Ala Ala Pro Ser Val Phe Ile Phe Pro Pro Ser Asp Glu
   580 585 590
   Gln Leu Lys Ser Gly Thr Ala Ser Val Val Cys Leu Leu Asn Asn Phe
   595 600 605
   Tyr Pro Arg Glu Ala Lys Val Gln Trp Lys Val Asp Asn Ala Leu Gln
   610 615 620
   Ser Gly Asn Ser Gln Glu Ser Val Thr Glu Gln Asp Ser Lys Asp Ser
   625 630 635 640
   Thr Tyr Ser Leu Ser Ser Thr Leu Thr Leu Ser Lys Ala Asp Tyr Glu
   645 650 655
   Lys His Lys Val Tyr Ala Cys Glu Val Thr His Gln Gly Leu Ser Ser
   660 665 670
   Pro Val Thr Lys Ser Phe Asn Arg Gly Glu Cys
   675 680
<210> 33
   <211> 3
   <212> PRT
   <213> Artificial Sequence
<220>
   <223> linker
<400> 33
   Lys Ala Lys
   1
<210> 34
   <211> 681
   <212> PRT
   <213> Artificial Sequence
<220>
   <223> LeY-biotin-knob
<400> 34
   Asp Val Lys Leu Val Glu Ser Gly Gly Gly Leu Val Gln Pro Gly Gly
   1               5                   10                  15
   Ser Leu Lys Leu Ser Cys Ala Thr Ser Gly Phe Thr Phe Ser Asp Tyr
               20                  25                  30
   Tyr Met Tyr Trp Val Arg Gln Thr Pro Glu Lys Arg Leu Glu Trp Val
           35                  40                  45
   Ala Tyr Ile Ser Asn Asp Asp Ser Ser Ala Ala Tyr Ser Asp Thr Val
       50                  55                  60
   Lys Gly Arg Phe Thr Ile Ser Arg Asp Asn Ala Arg Asn Thr Leu Tyr
   65                  70                  75                  80
   Leu Gln Met Ser Arg Leu Lys Ser Glu Asp Thr Ala Ile Tyr Tyr Cys
                   85                  90                  95
   Ala Arg Gly Leu Ala Trp Gly Ala Trp Phe Ala Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val Glu Pro Lys Ser Cys Asp Lys
   210 215 220
   Thr Gly Gly Gly Gly Ser Gly Gly Gly Gly Ser Asp Ile Gln Met Thr
   225 230 235 240
   Gln Ser Pro Ser Ser Leu Ser Ala Ser Val Gly Asp Arg Val Thr Ile
   245 250 255
   Thr Cys Arg Ala Ser Gly Asn Ile His Asn Tyr Leu Ser Trp Tyr Gln
   260 265 270
   Gln Lys Pro Gly Lys Val Pro Lys Leu Leu Ile Tyr Ser Ala Lys Thr
   275 280 285
   Leu Ala Asp Gly Val Pro Ser Arg Phe Ser Gly Ser Gly Ser Gly Thr
   290 295 300
   Asp Phe Thr Leu Thr Ile Ser Ser Leu Gln Pro Glu Asp Val Ala Thr
   305 310 315 320
   Tyr Tyr Cys Gln His Phe Trp Ser Ser Ile Tyr Thr Phe Gly Cys Gly
   325 330 335
   Thr Lys Leu Glu Ile Lys Lys Ala Lys Gly Gln Pro Arg Glu Pro Gln
   340 345 350
   Val Tyr Thr Leu Pro Pro Cys Arg Asp Glu Leu Thr Lys Asn Gln Val
   355 360 365
   Ser Leu Trp Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Val Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys Gly Gly Gly Gly Ser Gly Gly Gly Gly Ser Asp Val
   450 455 460
   Leu Met Thr Gln Ser Pro Leu Ser Leu Pro Val Ser Leu Gly Asp Gln
   465                 470                 475                 480
   Ala Ser Ile Ser Cys Arg Ser Ser Gln Ile Ile Val His Ser Asn Gly
                   485                 490                 495
   Asn Thr Tyr Leu Glu Trp Tyr Leu Gln Lys Pro Gly Gln Ser Pro Lys
               500                 505                 510
   Leu Leu Ile Tyr Lys Val Ser Asn Arg Phe Ser Gly Val Pro Asp Arg
           515                 520                 525
   Phe Ser Gly Ser Gly Ser Gly Thr Asp Phe Thr Leu Lys Ile Ser Arg
       530                 535                 540
   Val Glu Ala Glu Asp Leu Gly Val Tyr Tyr Cys Phe Gln Gly Ser His
   545                 550                 555                 560
   Val Pro Phe Thr Phe Gly Ser Gly Thr Lys Leu Glu Ile Lys Arg Thr
                   565                 570                 575
   Val Ala Ala Pro Ser Val Phe Ile Phe Pro Pro Ser Asp Glu Gln Leu
               580                 585                 590
   Lys Ser Gly Thr Ala Ser Val Val Cys Leu Leu Asn Asn Phe Tyr Pro
           595                 600                 605
   Arg Glu Ala Lys Val Gln Trp Lys Val Asp Asn Ala Leu Gln Ser Gly
       610                 615                 620
   Asn Ser Gln Glu Ser Val Thr Glu Gln Asp Ser Lys Asp Ser Thr Tyr
   625                 630                 635                 640
   Ser Leu Ser Ser Thr Leu Thr Leu Ser Lys Ala Asp Tyr Glu Lys His
                   645                 650                 655
   Lys Val Tyr Ala Cys Glu Val Thr His Gln Gly Leu Ser Ser Pro Val
               660                 665                 670
   Thr Lys Ser Phe Asn Arg Gly Glu Cys
           675                 680
<210> 35
   <211> 694
   <212> PRT
   <213> Artificial Sequence
<220>
   <223> LeY-biotin-hole
<400> 35
   Asp Val Lys Leu Val Glu Ser Gly Gly Gly Leu Val Gln Pro Gly Gly
   1               5                   10                  15
   Ser Leu Lys Leu Ser Cys Ala Thr Ser Gly Phe Thr Phe Ser Asp Tyr
               20                  25                  30
   Tyr Met Tyr Trp Val Arg Gln Thr Pro Glu Lys Arg Leu Glu Trp Val
           35                  40                  45
   Ala Tyr Ile Ser Asn Asp Asp Ser Ser Ala Ala Tyr Ser Asp Thr Val
       50                  55                  60
   Lys Gly Arg Phe Thr Ile Ser Arg Asp Asn Ala Arg Asn Thr Leu Tyr
   65                  70                  75                  80
   Leu Gln Met Ser Arg Leu Lys Ser Glu Asp Thr Ala Ile Tyr Tyr Cys
                   85                  90                  95
   Ala Arg Gly Leu Ala Trp Gly Ala Trp Phe Ala Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val Glu Pro Lys Ser Cys Asp Lys
       210                 215                 220
   Thr Gly Gly Gly Gly Ser Gly Gly Gly Gly Ser Gln Val Gln Leu Val
   225                 230                 235                 240
   Gln Ser Gly Ala Glu Val Lys Lys Pro Gly Ser Ser Val Lys Val Ser
   245 250 255
   Cys Lys Ser Ser Gly Phe Asn Asn Lys Asp Thr Phe Phe Gln Trp Val
   260 265 270
   Arg Gln Ala Pro Gly Gln Cys Leu Glu Trp Met Gly Arg Ile Asp Pro
   275 280 285
   Ala Asn Gly Phe Thr Lys Tyr Ala Gln Lys Phe Gln Gly Arg Val Thr
   290 295 300
   Ile Thr Ala Asp Thr Ser Thr Ser Thr Ala Tyr Met Glu Leu Ser Ser
   305 310 315 320
   Leu Arg Ser Glu Asp Thr Ala Val Tyr Tyr Cys Ala Arg Trp Asp Thr
   325 330 335
   Tyr Gly Ala Ala Trp Phe Ala Tyr Trp Gly Gln Gly Thr Leu Val Thr
   340 345 350
   Val Ser Ser Lys Ala Lys Gly Gln Pro Arg Glu Pro Gln Val Cys Thr
   355 360 365
   Leu Pro Pro Ser Arg Asp Glu Leu Thr Lys Asn Gln Val Ser Leu Ser
   370 375 380
   Cys Ala Val Lys Gly Phe Tyr Pro Ser Asp Ile Ala Val Glu Trp Glu
   385 390 395 400
   Ser Asn Gly Gln Pro Glu Asn Asn Tyr Lys Thr Thr Pro Pro Val Leu
   405 410 415
   Asp Ser Asp Gly Ser Phe Phe Leu Val Ser Lys Leu Thr Val Asp Lys
   420 425 430
   Ser Arg Trp Gln Gln Gly Asn Val Phe Ser Cys Ser Val Met His Glu
   435 440 445
   Ala Leu His Asn His Tyr Thr Gln Lys Ser Leu Ser Leu Ser Pro Gly
   450 455 460
   Lys Gly Gly Gly Gly Ser Gly Gly Gly Gly Ser Asp Val Leu Met Thr
   465 470 475 480
   Gln Ser Pro Leu Ser Leu Pro Val Ser Leu Gly Asp Gln Ala Ser Ile
   485 490 495
   Ser Cys Arg Ser Ser Gln Ile Ile Val His Ser Asn Gly Asn Thr Tyr
   500 505 510
   Leu Glu Trp Tyr Leu Gln Lys Pro Gly Gln Ser Pro Lys Leu Leu Ile
   515 520 525
   Tyr Lys Val Ser Asn Arg Phe Ser Gly Val Pro Asp Arg Phe Ser Gly
   530 535 540
   Ser Gly Ser Gly Thr Asp Phe Thr Leu Lys Ile Ser Arg Val Glu Ala
   545 550 555 560
   Glu Asp Leu Gly Val Tyr Tyr Cys Phe Gln Gly Ser His Val Pro Phe
   565 570 575
   Thr Phe Gly Ser Gly Thr Lys Leu Glu Ile Lys Arg Thr Val Ala Ala
   580 585 590
   Pro Ser Val Phe Ile Phe Pro Pro Ser Asp Glu Gln Leu Lys Ser Gly
   595 600 605
   Thr Ala Ser Val Val Cys Leu Leu Asn Asn Phe Tyr Pro Arg Glu Ala
   610 615 620
   Lys Val Gln Trp Lys Val Asp Asn Ala Leu Gln Ser Gly Asn Ser Gln
   625 630 635 640
   Glu Ser Val Thr Glu Gln Asp Ser Lys Asp Ser Thr Tyr Ser Leu Ser
   645 650 655
   Ser Thr Leu Thr Leu Ser Lys Ala Asp Tyr Glu Lys His Lys Val Tyr
   660 665 670
   Ala Cys Glu Val Thr His Gln Gly Leu Ser Ser Pro Val Thr Lys Ser
   675 680 685
   Phe Asn Arg Gly Glu Cys
   690
<210> 36
   <211> 686
   <212> PRT
   <213> Artificial Sequence
<220>
   <223> LeY-CD3-knob chain
<400> 36
   Asp Val Lys Leu Val Glu Ser Gly Gly Gly Leu Val Gln Pro Gly Gly
   1               5                   10                  15
   Ser Leu Lys Leu Ser Cys Ala Thr Ser Gly Phe Thr Phe Ser Asp Tyr
               20                  25                  30
   Tyr Met Tyr Trp Val Arg Gln Thr Pro Glu Lys Arg Leu Glu Trp Val
           35                  40                  45
   Ala Tyr Ile Ser Asn Asp Asp Ser Ser Ala Ala Tyr Ser Asp Thr Val
       50                  55                  60
   Lys Gly Arg Phe Thr Ile Ser Arg Asp Asn Ala Arg Asn Thr Leu Tyr
   65                  70                  75                  80
   Leu Gln Met Ser Arg Leu Lys Ser Glu Asp Thr Ala Ile Tyr Tyr Cys
                   85                  90                  95
   Ala Arg Gly Leu Ala Trp Gly Ala Trp Phe Ala Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val Glu Pro Lys Ser Cys Asp Lys
       210                 215                 220
   Thr His Gly Gly Gly Gly Ser Glu Val Gln Leu Val Gln Ser Gly Ala
   225                 230                 235                 240
   Glu Val Lys Lys Pro Gly Ala Ser Val Lys Val Ser Cys Lys Ala Ser
                   245                 250                 255
   Gly Tyr Thr Phe Thr Asn Tyr Tyr Ile His Trp Val Arg Gln Ala Pro
   260 265 270
   Gly Gln Gly Leu Glu Trp Ile Gly Trp Ile Tyr Pro Gly Asp Gly Asn
   275 280 285
   Thr Lys Tyr Asn Glu Lys Phe Lys Gly Arg Ala Thr Leu Thr Ala Asp
   290 295 300
   Thr Ser Thr Ser Thr Ala Tyr Leu Glu Leu Ser Ser Leu Arg Ser Glu
   305 310 315 320
   Asp Thr Ala Val Tyr Tyr Cys Ala Arg Asp Ser Tyr Ser Asn Tyr Tyr
   325 330 335
   Phe Asp Tyr Trp Gly Gln Gly Thr Leu Val Thr Val Ser Ser Gly Gln
   340 345 350
   Pro Arg Glu Pro Gln Val Tyr Thr Leu Pro Pro Cys Arg Asp Glu Leu
   355 360 365
   Thr Lys Asn Gln Val Ser Leu Trp Cys Leu Val Lys Gly Phe Tyr Pro
   370 375 380
   Ser Asp Ile Ala Val Glu Trp Glu Ser Asn Gly Gln Pro Glu Asn Asn
   385 390 395 400
   Tyr Lys Thr Thr Pro Pro Val Leu Asp Ser Asp Gly Ser Phe Phe Leu
   405 410 415
   Tyr Ser Lys Leu Thr Val Asp Lys Ser Arg Trp Gln Gln Gly Asn Val
   420 425 430
   Phe Ser Cys Ser Val Met His Glu Ala Leu His Asn His Tyr Thr Gln
   435 440 445
   Lys Ser Leu Ser Leu Ser Pro Gly Lys Gly Gly Gly Gly Ser Gly Gly
   450 455 460
   Gly Gly Ser Asp Val Leu Met Thr Gln Ser Pro Leu Ser Leu Pro Val
   465 470 475 480
   Ser Leu Gly Asp Gln Ala Ser Ile Ser Cys Arg Ser Ser Gln Ile Ile
   485 490 495
   Val His Ser Asn Gly Asn Thr Tyr Leu Glu Trp Tyr Leu Gln Lys Pro
   500 505 510
   Gly Gln Ser Pro Lys Leu Leu Ile Tyr Lys Val Ser Asn Arg Phe Ser
   515 520 525
   Gly Val Pro Asp Arg Phe Ser Gly Ser Gly Ser Gly Thr Asp Phe Thr
   530 535 540
   Leu Lys Ile Ser Arg Val Glu Ala Glu Asp Leu Gly Val Tyr Tyr Cys
   545 550 555 560
   Phe Gln Gly Ser His Val Pro Phe Thr Phe Gly Ser Gly Thr Lys Leu
   565 570 575
   Glu Ile Lys Arg Thr Val Ala Ala Pro Ser Val Phe Ile Phe Pro Pro
   580 585 590
   Ser Asp Glu Gln Leu Lys Ser Gly Thr Ala Ser Val Val Cys Leu Leu
   595 600 605
   Asn Asn Phe Tyr Pro Arg Glu Ala Lys Val Gln Trp Lys Val Asp Asn
   610 615 620
   Ala Leu Gln Ser Gly Asn Ser Gln Glu Ser Val Thr Glu Gln Asp Ser
   625 630 635 640
   Lys Asp Ser Thr Tyr Ser Leu Ser Ser Thr Leu Thr Leu Ser Lys Ala
   645 650 655
   Asp Tyr Glu Lys His Lys Val Tyr Ala Cys Glu Val Thr His Gln Gly
   660 665 670
   Leu Ser Ser Pro Val Thr Lys Ser Phe Asn Arg Gly Glu Cys
   675 680 685
<210> 37
   <211> 679
   <212> PRT
   <213> Artificial Sequence
<220>
   <223> LeY-CD3-hole chain
<400> 37
   Asp Val Leu Met Thr Gln Ser Pro Leu Ser Leu Pro Val Ser Leu Gly
   1 5 10 15
   Asp Gln Ala Ser Ile Ser Cys Arg Ser Ser Gln Ile Ile Val His Ser
   20 25 30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Phe Thr Phe Gly Ser Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys Asp Lys Thr His Gly
       210                 215                 220
   Gly Gly Gly Ser Asp Ile Val Met Thr Gln Ser Pro Asp Ser Leu Ala
   225                 230                 235                 240
   Val Ser Leu Gly Glu Arg Ala Thr Ile Asn Cys Lys Ser Ser Gln Ser
                   245                 250                 255
   Leu Leu Asn Ser Arg Thr Arg Lys Asn Tyr Leu Ala Trp Tyr Gln Gln
               260                 265                 270
   Lys Pro Gly Gln Pro Pro Lys Leu Leu Ile Tyr Trp Ala Ser Thr Arg
           275                 280                 285
   Glu Ser Gly Val Pro Asp Arg Phe Ser Gly Ser Gly Ser Gly Thr Asp
   290 295 300
   Phe Thr Leu Thr Ile Ser Ser Leu Gln Ala Glu Asp Val Ala Val Tyr
   305 310 315 320
   Tyr Cys Thr Gln Ser Phe Ile Leu Arg Thr Phe Gly Gln Gly Thr Lys
   325 330 335
   Val Glu Ile Lys Gly Gln Pro Arg Glu Pro Gln Val Cys Thr Leu Pro
   340 345 350
   Pro Ser Arg Asp Glu Leu Thr Lys Asn Gln Val Ser Leu Ser Cys Ala
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Val Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys Gly
   435 440 445
   Gly Gly Gly Ser Gly Gly Gly Gly Ser Asp Val Lys Leu Val Glu Ser
   450 455 460
   Gly Gly Gly Leu Val Gln Pro Gly Gly Ser Leu Lys Leu Ser Cys Ala
   465 470 475 480
   Thr Ser Gly Phe Thr Phe Ser Asp Tyr Tyr Met Tyr Trp Val Arg Gln
   485 490 495
   Thr Pro Glu Lys Arg Leu Glu Trp Val Ala Tyr Ile Ser Asn Asp Asp
   500 505 510
   Ser Ser Ala Ala Tyr Ser Asp Thr Val Lys Gly Arg Phe Thr Ile Ser
   515 520 525
   Arg Asp Asn Ala Arg Asn Thr Leu Tyr Leu Gln Met Ser Arg Leu Lys
   530 535 540
   Ser Glu Asp Thr Ala Ile Tyr Tyr Cys Ala Arg Gly Leu Ala Trp Gly
   545 550 555 560
   Ala Trp Phe Ala Tyr Trp Gly Gln Gly Thr Leu Val Thr Val Ser Ser
   565 570 575
   Ala Ser Thr Lys Gly Pro Ser Val Phe Pro Leu Ala Pro Ser Ser Lys
   580 585 590
   Ser Thr Ser Gly Gly Thr Ala Ala Leu Gly Cys Leu Val Lys Asp Tyr
   595 600 605
   Phe Pro Glu Pro Val Thr Val Ser Trp Asn Ser Gly Ala Leu Thr Ser
   610 615 620
   Gly Val His Thr Phe Pro Ala Val Leu Gln Ser Ser Gly Leu Tyr Ser
   625 630 635 640
   Leu Ser Ser Val Val Thr Val Pro Ser Ser Ser Leu Gly Thr Gln Thr
   645 650 655
   Tyr Ile Cys Asn Val Asn His Lys Pro Ser Asn Thr Lys Val Asp Lys
   660 665 670
   Lys Val Glu Pro Lys Ser Cys
   675
<210> 38
   <211> 9
   <212> PRT
   <213> Artificial Sequence
<220>
   <223> DKTHGGGGS - linker
<400> 38
   Asp Lys Thr His Gly Gly Gly Gly Ser
   1 5